# EUROPEAN PATENT SPECIFICATION

(11) **EP 2 362 218 B1**
(45) Date of publication and mention of the grant of the patent: **27.08.2014**
(21) Application number: 11158145.0
(22) Date of filing: 02.11.2005
(51) Int. Cl.: G01N 33/50, A61K 31/4709, A61P 7/00, G01N 33/68, C12Q 1/68

(54) **METHODS OF MONITORING THE EFFICACY OF FARNESYLTRANSFERASE INHIBITORS**
METHODEN ZUR ÜBERWACHUNG DER WIRKSAMKEIT VON FARNESYLTRANSFERASE-HEMMERN
METHODES DE CONTROLE DE L'EFFICACITE D'INHIBITEURS DE FARNESYLTRANSFERASE

(30) Priority: 05.11.2004 US 625204 P; 12.08.2005 US 708075 P
(43) Date of publication of application: 31.08.2011
(62) Divisional of application: 05851365.6
(73) Proprietor: Janssen Pharmaceutica N.V., 2340 Beerse (BE)
(72) Inventor: Fourie, Anne, San Diego, CA 92130 (US)
(74) Representative: Warner, James Alexander

(56) References cited:
- WO-A1-97/30992
- WO-A2-2004/044554
- RAPONI MITCH ET AL: "Microarray analysis reveals genetic pathways modulated by tipifarnib in acute myeloid leukemia", BMC CANCER, BIOMED CENTRAL, LONDON, GB, vol. 4, no. 1, 25 August 2004 (2004-08-25) , page 56, XP021004657, ISSN: 1471-2407, DOI: 10.1186/1471-2407-4-56 & Raponi M et al.: "Microarray analysis reveals genetic pathways modulated by tipifarnib in acute myeloid leukemia; Additional File 2", BMC Cancer, vol. 4, no. 56 25 August 2004 (2004-08-25), Retrieved from the Internet: URL:http://www.biomedcentral.com/1471-2407 /4/56/additional/ [retrieved on 2011-09-12]
- Y. TAKADA: "Protein Farnesyltransferase Inhibitor (SCH 66336) Abolishes NF- B Activation Induced by Various Carcinogens and Inflammatory Stimuli Leading to Suppression of NF- B-regulated Gene Expression and Up-regulation of Apoptosis", JOURNAL OF BIOLOGICAL CHEMISTRY, vol. 279, no. 25, 1 January 2004 (2004-01-01), pages 26287-26299, XP55006918, ISSN: 0021-9258, DOI: 10.1074/jbc.M400963200
- NA: "Inhibition of farnesyltransferase prevents collagen-induced arthritis by down-regulation of inflammatory gene expression through suppression of p21ras-dependent NF-[kappa]B activation", THE JOURNAL OF IMMUNOLOGY, vol. 173, no. 2, 1 January 2004 (2004-01-01), page 1276, XP55006924, ISSN: 0022-1767

### SEQUENCE LISTING

<110> JANSSEN PHARMACEUTICA, N.V.
<120> THERAPEUTIC USE OF FARNESYLTRANSFERASE INHIBITORS AND METHODS OF MONITORING THE EFFICACY THEREOF
<130> P056627EP
<140> EP ...
   <141> 2005-11-02
<150> US60/625,204
   <151> 2004-11-05
<150> US60/708,075
   <151> 2005-08-12
<160> 14
<170> PatentIn version 3.5
<210> 1
   <211> 23
   <212> DNA
   <213> Primer
<400> 1
   ggataacgag gcttatgtgc acg 23
<210> 2
   <211> 24
   <212> DNA
   <213> Primer
<400> 2
   ggacatggag aacaccactt gttg 24
<210> 3
   <211> 21
   <212> DNA
   <213> Primer
<400> 3
   agccagatgc aatcaatgcc c 21
<210> 4
   <211> 22
   <212> DNA
   <213> Primer
<400> 4
   ccttggccac aatggtcttg aa 22
<210> 5
   <211> 20
   <212> DNA
   <213> Primer
<400> 5
   accgctatgg ttacactcgg 20
<210> 6
   <211> 20
   <212> DNA
   <213> Primer
<400> 6
   agggaccaca actcgtcatc 20
<210> 7
   <211> 24
   <212> DNA
   <213> Primer
<400> 7
   ctgatttctg cagctctgtg tgaa 24
<210> 8
   <211> 23
   <212> DNA
   <213> Primer
<400> 8
   tggcatcttc actgattctt gga 23
<210> 9
   <211> 20
   <212> DNA
   <213> Primer
<400> 9
   ttcagagctc gtttgtggtg 20
<210> 10
   <211> 20
   <212> DNA
   <213> Primer
<400> 10
   agaggtcgtc tcgaggtcaa 20
<210> 11
   <211> 24
   <212> DNA
   <213> Primer
<400> 11
   tgggacccag cattgaggag gatt 24
<210> 12
   <211> 22
   <212> DNA
   <213> Primer
<400> 12
   aaactggatg tcgctggggc aa 22
<210> 13
   <211> 22
   <212> DNA
   <213> Primer
<400> 13
   tggattcaat gaggagactt gc 22
<210> 14
   <211> 20
   <212> DNA
   <213> Primer
<400> 14
   caggaactgg atcaggactt 20

## Description

### FIELD OF THE INVENTION

The present disclosure relates to the use of farnesyltransferase inhibitors for the treatment of sepsis or septic shock. The invention relates to methods of determining or monitoring a patient's response to treatment with a farnesyltransferase inhibitor.

### BACKGROUND OF THE INVENTION

Statins inhibit the activity of 3-hydroxyl-3-methylglutaryl-coenzyme A (HMG-CoA) reductase, the rate-limiting enzyme in cholesterol biosynthesis (Brown 1990), and are widely prescribed to lower cholesterol in hyperlipidemic patients. Recent observations from clinical trials suggest that statins provide cardiovascular benefit beyond their lipid lowering effects. The recognition that atherosclerosis involves an inflammatory component suggests that some of the beneficial effects of statins may be related to their modulation of immunity and inflammation (Schonbeck U 2004).

Inflammation is normally an acute response by the immune system to microbial pathogens, chemicals or physical injury, and is characterized by redness, heat, swelling and pain. These symptoms are the result of a cascade of events including cytokine and chemokine production, cell migration and accumulation, coagulation, fibrinolysis, blood vessel dilation and increased permeability, extravasation of plasma and proteins. If the inflammatory response is not appropriately regulated, it can progress to a chronic state, and be the cause of inflammatory diseases, a major cause of morbidity and mortality. A functional relationship between inflammation and cancer has been recognized for a long time, but the molecular pathways underlying this link were unknown. Recent studies have shown that inflammation-induced tumor growth is mediated through activation of the transcription factor, NF-κB, and production of the inflammatory mediator, TNF-α (Luo, Maeda et al. 2004; Pikarsky, Porat et al. 2004).

Treatment of animals with lipopolysaccharide (LPS) from gram-negative bacteria is used to experimentally induce a systemic inflammatory response, including the classic inflammatory symptoms of redness, heat, swelling and pain. LPS administration to animals serves as a model for LPS-induced sepsis and inflammation-induced tumor growth, as well as a more general model for inflammatory responses induced by other agents in inflammatory diseases. LPS treatment of cells or animals has been widely used as an experimental model to assess the roles of mediators such as cytokines and chemokines in inflammation, and to study agents that can modulate the production of such mediators.

Potential therapeutic effects of statins in inflammatory diseases have been supported by several animal studies using LPS treatment as well as other animal models for inflammatory diseases. Cerivastatin was shown to reduce the serum levels of TNF-α, IL-1β, nitric oxide, and improve survival of mice in an LPS-induced sepsis model (Ando, Takamura et al. 2000).

Atorvastatin and lovastatin were reported to prevent or reverse experimental autoimmune encephalomyelitis (EAE) (Stanislaus, Pahan et al. 1999; Youssef, Stueve et al. 2002). Simvastatin has shown anti-inflammatory activity in collagen-induced arthritis (Leung, Sattar et al. 2003), and in an allergic asthma model (McKay A 2004) It was also found that pravastatin relieved cachexia, hematochezia and intestinal epithelial permeability in dextran sulfate-induced colitis (Sasaki M, et al. 2003. J Pharmacol Exp Ther 305(1), 78-85).

MCP-1, IL-1b, IL-6, MMP-9, MyD88 and TNF-α have been implicated in the pathology of atherosclerosis (Gu, Okada et al. 1998; Aiello, Bourassa et al. 1999; Dawson, Kuziel et al. 1999; Ito, Ikeda et al. 2002; Haddy, Sass et al. 2003; Kirii, Niwa et al. 2003; Luttun, Lutgens et al. 2004; Michelsen, Wong et al. 2004) and sepsis (Bossink, Paemen et al. 1995; Weighardt, Kaiser-Moore et al. 2002; Das 2003; Lalu, Gao et al. 2003; Mancuso, Midiri et al. 2004). Statins are used for treatment of atherosclerosis, where their beneficial effect are believed to be due both to lipid-lowering-dependent and -independent effects. Rezaie-Majd et al. (2002. Arterioscler Thromb Vasc Biol 22, 1194-9) reported that simvastatin reduced both protein and RNA levels of the cytokines, IL-6, IL-8 and monocyte chemoattractant protein-1 (MCP-1), in hypercholesterolemic patients after 6 weeks of treatment. In patients with coronary artery disease (CAD), gene expression of the chemokines, MIP-1α, MIP-1β, IL-8, and receptors, CCR1 and CCR2, was significantly down-regulated after treatment with atovastatin or simvastatin for six months (Waehre T 2003). The therapeutic potential of statins for inflammatory diseases has been highlighted by recent findings in the Trial of Atorvastatin in Rheumatoid Arthritis (TARA), which have shown that the disease activity score, erythrocyte sedimentation and C-reactive protein were significantly improved compared to existing therapy after 6 months treatment with atorvastatin (McCarey DW 2004).

Sepsis and septic shock are complex inflammatory syndromes. Recent data has shown that statin therapy is associated with a decreased rate of severe sepsis (Almog, Shefer et al. 2004) and improves survival in a murine model of sepsis (Ando, Takamura et al. 2000).

Many observations of anti-inflammatory effects for statins in animal models have been at higher than therapeutic doses. However, there is evidence for anti-inflammatory effects of statins in human clinical trials at therapeutic doses (Rezaie-Majd, Maca et al. 2002; Waehre T 2003; McCarey DW 2004). While animal models and human clinical observations demonstrate that statins have anti-inflammatory properties, the mechanism underlying these properties is currently unclear. *In vitro* studies on cells have shown that statins affect many different processes and it is not known which processes are required for anti-inflammatory effects in vivo. For example, it is known that the activation of transcription factors such as NF-κB, AP-1 and hypoxia-inducible factor-1α, which regulate the transcription of many inflammatory genes, is down-regulated *in vitro* by simvastatin, atorvastatin and lovastatin (Ortego M 1999; Dichtl, Dulak et al. 2003).

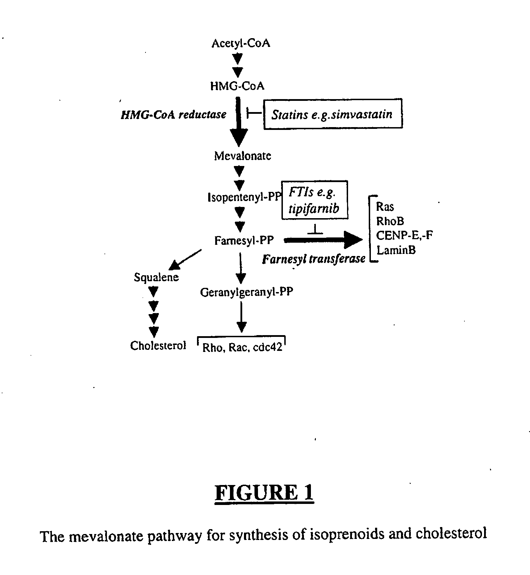

Statins inhibit mevalonate synthesis, the rate-limiting step in production of cholesterol (Brown 1990) and also decrease the synthesis of the isoprenoids, farnesyl pyrophosphate (FPP) and geranylgeranyl pyrophosphate (GGPP) (see **Figure 1**). GGPP and FPP are required for prenylation, activation and sub-cellular location of numerous intracellular proteins (Casey PJ. 1995; Zhang FL 1996).

Studies have shown that exogenous GGPP (or geraniol) and/or FPP (or farnesol) can reverse certain statin-mediated anti-inflammatory functions including: (1) leukocyte adhesion (Liu L 1999); (2) cell proliferation/apoptosis (Guijarro C 1998; Wen-Bin Zhong 2003); and (3) NF-κB activity (Ortego M 1999), and (4) LPS-induced MMP-9 production (Wong, Lumma et al. 2001). In contrast, squalestatin, a selective inhibitor of squalene synthase and cholesterol synthesis, is unable to mimic the inhibitory effect of a statin on LPS-induced leukocyte recruitment and chemokine production (Diomede L 2001).

It has been reported that statins suppress LPS-induced MMP-9 secretion by THP-1 cells, but that inhibition of farnesyltransferase (Ftase, also referred to as farnesyl protein transferase or FPTase) has no effect (Wong, B., et al. 2001. J Leukoc Biol 69, 959-62).

FTIs were originally developed to prevent post-translational farnesylation and activation of Ras oncoproteins (Prendergast and Rane 2001). Recent studies demonstrated inhibition by an FTI of NF-κB activation (Takada, Y., et al. 2004. J Biol Chem 279, 26287-99) and downregulation of inflammatory gene expression through suppression of Ras-dependent NF-κB activation (Na et al. 2004). Thus FTIs share some, but not all, properties with statins.

A variety of FTase inhibitors are known. For example, see U.S. Patent No. 6,451,812, which describes certain FTIs and their use in treating arthropathies such as rheumatoid arthritis, osteoarthritis, juvenile arthritis, and gout.

WO-97/21701 and U.S. Patent No. 6,037,350, describe the preparation, formulation and pharmaceutical properties of certain farnesyl transferase inhibiting (imidazoly-5-yl)methyl-2-quinolinone derivatives of formulas (I), (II) and (III), as well as intermediates of formula (II) and (III) that are metabolized *in vivo* to the compounds of formula (I). The compounds of formulas (I), (II) and (III) are represented by 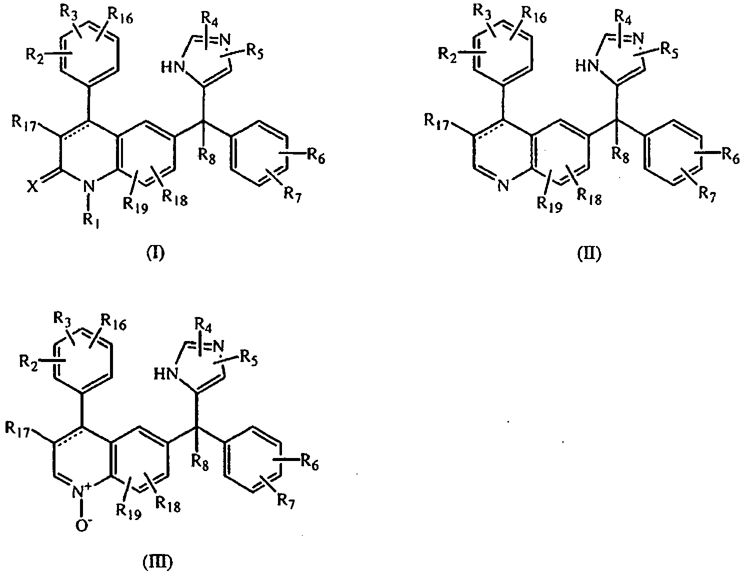 the pharmaceutically acceptable acid or base addition salts and the stereochemically isomeric forms thereof, wherein
the dotted line represents an optional bond;
- X: is oxygen or sulfur;
- R¹: is hydrogen, C₁₋₁₂alkyl, Ar¹, Ar²C₁₋₆alkyl,quinolinylC₁₋₆alkyl, pyridylC₁₋₆alkyl, hydroxyC₁₋₆alkyl, C₁₋₆alkyloxyC₁₋₆alkyl, mono- or di(C₁₋₆alkyl)aminoC₁₋₆alkyl, aminoC₁₋₆alkyl, or a radical of formula -Alk¹-C(=O)-R⁹, -Alk¹-S(O)-R⁹ or -Alk¹-S(O)₂-R⁹, wherein Alk¹ is C₁₋₆alkanediyl,
R⁹ is hydroxy, C₁₋₆alkyl, C₁₋₆alkyloxy, amino, C₁₋₈alkylamino or C₁₋₈alkylamino substituted with C₁₋₆alkyloxycarbonyl;
- R², R³ and R¹⁶: each independently are hydrogen, hydroxy, halo, cyano, C₁-₆alkyl, C₁₋₆alkyloxy, hydroxyC₁₋₆alkyloxy, C₁₋₆alkyloxyC₁₋₆alkyloxy, aminoC₁₋₆alkyloxy, mono- or di(C₁₋₆alkyl)aminoC₁₋₆alkyloxy, Ar¹, Ar²C₁₋₆alkyl, Ar²oxy, Ar²C₁₋₆alkyloxy, hydroxycarbonyl, C₁₋₆alkyloxycarbonyl, trihalomethyl, trihalomethoxy, C₂₋₆alkenyl, 4,4-dimethyloxazolyl; or when on adjacent positions R² and R³ taken together may form a bivalent radical of formula

-O-CH₂-O- (a-1),

-O-CH₂-CH₂-O- (a-2),

-O-CH=CH- (a-3),

-O-CH₂-CH₂- (a-4),

-O-CH₂-CH₂-CH₂- (a-5),

or

-CH=CH-CH=CH- (a-6);
- R⁴ and R⁵: each independently are hydrogen, halo, Ar¹, C₁₋₆alkyl, hydroxyC₁₋₆alkyl, C₁₋₆alkyloxyC₁₋₆alkyl, C₁₋₆alkyloxy, C₁₋₆alkylthio, amino, hydroxycarbonyl, C₁₋₆alkyloxycarbonyl, C₁₋₆alkylS(O)C₁₋₆alkyl or C₁₋₆alkylS(O)₂C₁₋₆alkyl;
- R⁶ and R⁷: each independently are hydrogen, halo, cyano, C₁₋₆alkyl, C₁₋₆alkyloxy, Ar²oxy, trihalomethyl, C₁₋₆alkylthio, di(C₁₋₆alkyl)amino, or when on adjacent positions R⁶ and R⁷ taken together may form a bivalent radical of formula

-O-CH₂-O- (c-1),

or

-CH=CH-CH=CH- (c-2);
- R⁸: is hydrogen, C₁₋₆alkyl, cyano, hydroxycarbonyl, C₁₋₆alkyloxycarbonyl, C₁₋₆alkylcarbonylC₁₋₆alkyl, cyanoC₁₋₆alkyl, C₁₋₆alkyloxycarbonylC₁₋₆alkyl, carboxyC₁₋₆alkyl, hydroxyC₁₋₆alkyl, aminoC₁₋₆alkyl, mono- or di(C₁₋₆alkyl)aminoC₁₋₆alkyl, imidazolyl, haloC₁₋₆alkyl, C₁₋₆alkyloxyC₁₋₆alkyl, aminocarbonylC₁₋₆alkyl, or a radical of formula

-O-R¹⁰ (b-1),

-S-R¹⁰ (b-2),

-N-R¹¹R¹² (b-3),

wherein R¹⁰ is hydrogen, C₁₋₆alkyl, C₁₋₆alkylcarbonyl, Ar¹, Ar²C₁₋₆alkyl, C₁₋₆alkyloxycarbonylC₁₋₆alkyl, or a radical of formula -Alk²-OR¹³ or -Alk²-NR¹⁴R¹⁵;
R¹¹ is hydrogen, C₁₋₁₂alkyl, Ar¹ or Ar²C₁₋₆alkyl;
R¹² is hydrogen, C₁₋₆alkyl, C₁₋₁₆alkylcarbonyl, C₁₋₆alkyloxycarbonyl, C₁₋₆alkylaminocarbonyl, Ar¹, Ar²C₁₋₆alkyl, C₁₋₆alkylcarbonylC₁₋₆alkyl, a natural amino acid, Ar¹carbonyl, Ar²C₁₋₆alkylcarbonyl, aminocarbonylcarbonyl, C₁₋₆alkyloxyC₁₋₆alkylcarbonyl, hydroxy, C₁₋₆alkyloxy, aminocarbonyl, di(C ₁₋₆alkyl)aminoC₁₋₆alkylcarbonyl, amino, C₁₋₆alkylamino, C₁₋₆alkylcarbonylamino, or a radical of formula -Alk²-OR¹³ or - Alk²-NR¹⁴R¹⁵;wherein Alk² is C₁₋₆alkanediyl;
R¹³ is hydrogen, C₁₋₆alkyl, C₁₋₆alkylcarbonyl, hydroxyC₁₋₆alkyl, Ar¹ or Ar²C₁₋₆alkyl;
R¹⁴ is hydrogen, C₁₋₆alkyl, Ar¹ or Ar²C₁₋₆alkyl;
- R¹⁷: is hydrogen, C₁₋₆alkyl, C₁₋₆alkylcarbonyl, Ar¹ or Ar²C₁₋₆alkyl; is hydrogen, halo, cyano, C₁₋₆alkyl, C₁₋₆alkyloxycarbonyl, Ar¹;
- R¹⁸: is hydrogen, C₁₋₆alkyl, C₁₋₆alkyloxy or halo;
- R¹⁹: is hydrogen or C₁₋₆alkyl;
- Ar¹: is phenyl or phenyl substituted with C₁₋₆alkyl, hydroxy, amino, C₁₋₆alkyloxy or halo; and
- Ar²: is phenyl or phenyl substituted with C₁₋₆alkyl, hydroxy, amino, C₁₋₆alkyloxy or halo.

WO-97/16443 and U.S, Patent No. 5,968,952 describe the preparation, formulation and pharmaceutical properties of farnesyltransferase inhibiting compounds of formula (IV), as well as intermediates of formula (V) and (VI) that are metabolized *in vivo* to the compounds of formula (IV). The compounds of formulas (IV), (V) and (VI) are represented by 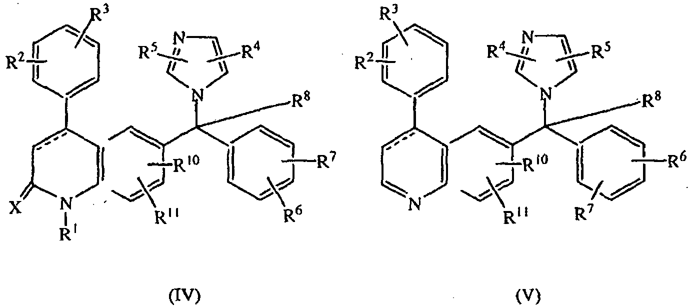 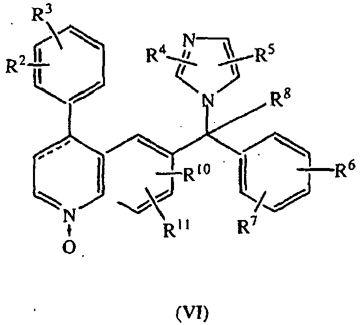 the pharmaceutically acceptable acid or base addition salts and the stereochemically isomeric forms thereof, wherein
the dotted line represents an optional bond;
- X: is oxygen or sulfur;
- R¹: is hydrogen, C₁₋₁₂alkyl, Ar¹, Ar²C₁₋₆alkyl, quinolinylC₁₋₆alkyl, pyridylC₁₋₆alkyl, hydroxyC₁₋₆alkyl, C₁₋₆alkyloxyC₁₋₆alkyl, mono- or di(C₁₋₆alkyl)aminoC₁₋₆alkyl, aminoC₁₋₆alkyl, or a radical of formula -Alk¹-C(=O)-R⁹, -Alk¹-S(O)-R⁹ or -Alk¹-S(O)₂-R⁹, wherein Alk¹ is C₁₋₆alkanediyl,
- R⁹: is hydroxy, C₁₋₆alkyl, C₁₋₆alkyloxy, amino, C₁₋₈alkylamino or C₁₋₈alkylamino substituted with C₁₋₆alkyloxycarbonyl;
- R² and R³: each independently are hydrogen, hydroxy, halo, cyano, C₁₋₆alkyl, C₁₋₆alkyloxy, hydroxyC₁₋₆alkyloxy, C₁₋₆alkyloxyC₁₋₆alkyloxy, aminoC₁₋₆alkyloxy, mono- or di(C₁₋₆alkyl)aminoC₁₋₆alkyloxy, Ar¹, Ar²C₁₋₆alkyl, Ar²oxy, Ar²C₁₋₆alkyloxy, hydroxycarbonyl, C₁₋₆alkyloxycarbonyl, trihalomethyl, trihalomethoxy, C₂₋₆alkenyl; or when on adjacent positions R² and R³ taken together may form a bivalent radical of formula

-O-CH₂-O- (a-1),

-O-CH₂-CH₂-O- (a-2),

-O-CH=CH- (a-3),

-O-CH₂-CH₂- (a-4),

-O-CH₂-CH₂-CH₂- (a-5),

or

-CH=CH-CH=CH- (a-6);
- R⁴ and R⁵: each independently are hydrogen, Ar¹, C₁₋₆alkyl, C₁₋₆alkyloxyC₁₋₆alkyl, C₁₋₆alkyloxy, C₁₋₆alkylthio, amino, hydroxycarbonyl, C₁₋₆alkyloxycarbonyl, C₁₋₆alkylS(O)C₁₋₆alkyl or C₁₋₆alkylS(O)₂C₁₋₆alkyl;
- R⁶ and R⁷: each independently are hydrogen, halo, cyano, C₁₋₆alkyl, C₁₋₆alkyloxy or Ar²oxy;
- R⁸: is hydrogen, C₁₋₆alkyl, cyano, hydroxycarbonyl, C₁₋₆alkyloxycarbonyl, C₁₋₆alkylcarbonylC₁₋₆alkyl, cyanoC₁₋₆alkyl, C₁₋₆alkyloxycarbonylC₁₋₆alkyl, hydroxycarbonylC₁₋₆alkyl, hydroxyC₁₋₆alkyl, aminoC₁₋₆alkyl, mono- or di(C₁₋₆alkyl)aminoC₁₋₆alkyl, haloC₁₋₆alkyl, C₁₋₆alkyloxyC₁₋₆alkyl, aminocarbonylC₁₋₆alkyl, Ar¹, Ar²C₁₋₆alkyloxyC₁₋₆alkyl, C₁₋₆alkylthioC₁₋₆alkyl;
- R¹⁰: is hydrogen, C₁₋₆alkyl, C₁₋₆alkyloxy or halo;
- R¹¹: is hydrogen or C₁₋₆alkyl;
- Ar¹: is phenyl or phenyl substituted with C₁₋₆alkyl,hydroxy,amino,C₁₋₆alkyloxy or halo;
- Ar²: is phenyl or phenyl substituted with C₁₋₆alkyl,hydroxy,amino,C₁₋₆alkyloxy or halo.
WO-98/40383 and U.S. Patent No. 6,187,786 disclose the preparation, formulation and pharmaceutical properties of farnesyltransferase inhibiting compounds of formula (VII) 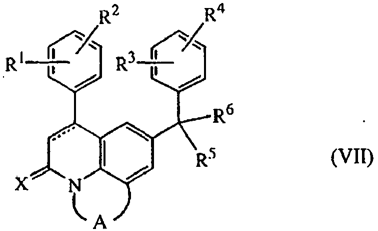 the pharmaceutically acceptable acid addition salts and the stereochemically isomeric forms thereof, wherein
the dotted line represents an optional bond;
- X: is oxygen or sulfur;
- -A-: is a bivalent radical of formula

-CH=CH- (a-1),

-CH₂-S- (a-6),

-CH₂-CH₂- (a-2),

-CH₂-CH₂-S- (a-7),

-CH₂-CH₂-CH₂- (a-3),

-CH=N- (a-8),

-CH₂-O- (a-4),

-N=N- (a-9),

or

-CH₂-CH₂-O- (a-5),

-CO-NH- (a-10);

wherein optionally one hydrogen atom may be replaced by C₁₋₄alkyl or Ar¹;
- R¹ and R²: each independently are hydrogen, hydroxy, halo, cyano, C₁₋₆alkyl, trihalomethyl, trihalomethoxy, C₂₋₆alkenyl, C₁₋₆alkyloxy, hydroxyC₁₋₆alkyloxy, C₁₋₆alkyloxyC₁₋₆alkyloxy, C₁₋₆alkyloxycarbonyl, aminoC₁₋₆alkyloxy, mono- or di(C₁₋₆alkyl)aminoC₁₋₆ₐlkyloxy, Ar², Ar²-C₁₋₆alkyl, Ar²-oxy,
Ar²-C₁₋₆alkyloxy; or when on adjacent positions R¹ and R² taken together may form a bivalent radical of formula

-O-CH₂-O- (b-1),

-O-CH₂-CH₂-O- (b-2),

-O-CH=CH- (b-3),

-O-CH₂-CH₂- (b-4),

-O-CH₂-CH₂-CH₂- (b-5),

or

-CH=CH-CH=CH- (b-6);
- R³ and R⁴: each independently are hydrogen, halo, cyano, C₁₋₆alkyl, C₁₋₆alkyloxy, Ar³-oxy, C₁₋₆alkylthio, di(C₁₋₆alkyl)amino, trihalomethyl, trihalomethoxy, or when on adjacent positions R³ and R⁴ taken together may form a bivalent radical of formula

-O-CH₂-O- (c-1),

-O-CH₂-CH₂-O- (c-2),

or

-CH=CH-CH=CH- (c-3);
- R⁵: is a radical of formula 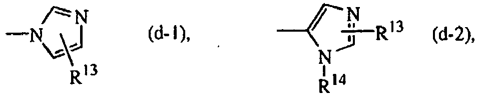 wherein R¹³ is hydrogen, halo, Ar⁴, C₁₋₆alkyl, hydroxyC₁₋₆alkyl,
C₁₋₆alkyloxyC₁₋₆alkyl, C₁₋₆alkyloxy, C₁₋₆alkylthio, amino,
C₁₋₆alkyloxycarbonyl, C₁₋₆alkylS(O)C₁₋₆alkyl or
C₁₋₆alkylS(O)₂C₁₋₆alkyl;
R¹⁴ is hydrogen, C₁₋₆alkyl or di(C₁₋₄alkyl)aminosulfonyl;
- R⁶: is hydrogen, hydroxy, halo, C₁₋₆alkyl, cyano, haloC₁₋₆alkyl, hydroxyC₁₋₆alkyl, cyanoC₁₋₆alkyl, aminoC₁₋₆alkyl, C₁₋₆alkyloxyC₁₋₆alkyl, C₁₋₆alkylthioC₁₋₆alkyl, aminocarbonylC₁₋₆alkyl, C₁₋₆alkyloxycarbonylC₁₋₆alkyl, C₁₋₆alkylcarbonyl-C₁₋₆alkyl, C₁₋₆alkyloxycarbonyl, mono- or di(C₁₋₆alkyl)aminoC₁₋₆alkyl, Ar⁵,
Ar⁵-C₁₋₆alkyloxyC₁₋₆alkyl; or a radical of formula

-O-R⁷ (e-1),

-S-R⁷ (e-2),

-N-R⁸R⁹ (e-3),
- wherein R⁷: is hydrogen, C₁₋₆alkyl, C₁₋₆alkylcarbonyl, Ar⁶, Ar⁶-C₁₋₆alkyl, C₁₋₆alkyloxycarbonylC₁₋₆alkyl, or a radical of formula -Alk-OR¹⁰ or -Alk-NR¹¹R¹²;
- R⁸: is hydrogen, C₁₋₆alkyl, Ar⁷ or Ar⁷-C₁₋₆alkyl;
- R⁹: is hydrogen, C₁₋₆alkyl, C₁₋₆alkylcarbonyl, C₁₋₆alkyloxycarbonyl, C₁₋₆alkylaminocarbonyl, Ar⁸, Ar⁸-C₁₋₆alkyl, C₁₋₆alkylcarbonyl-C₁₋₆alkyl, Ar⁸-carbonyl, Ar⁸-C₁₋₆alkylcarbonyl, aminocarbonylcarbonyl, C₁₋₆alkyloxyC₁₋₆alkylcarbonyl, hydroxy, C₁₋₆alkyloxy, aminocarbonyl, di(C₁₋₆alkyl)aminoC₁₋₆alkylcarbonyl, amino, C₁₋₆alkylamino, C₁₋₆alkylcarbonylamino,
or a radical of formula -Alk-OR¹⁰ or -Alk-NR¹¹R¹²;
- wherein: Alk is C₁₋₆alkanediyl;
- R¹⁰: is hydrogen, C₁₋₆alkyl, C₁₋₆alkylcarbonyl, hydroxyC₁₋₆alkyl, Ar⁹ or Ar⁹-C₁₋₆alkyl;
- R¹¹: is hydrogen, C₁₋₆alkyl, C₁₋₆alkylcarbonyl, Ar¹⁰ or Ar¹⁰-C₁₋₆alkyl;
- R¹²: is hydrogen, C₁₋₆alkyl, Ar¹¹ or Ar¹¹-C₁₋₆alkyl; and
- Ar¹ to Ar¹¹: are each independently selected from phenyl; or phenyl substituted with halo, C₁₋₆alkyl, C₁₋₆alkyloxy or trifluoromethyl.

WO-98/49157 and U.S. Patent No. 6,117,432 concern the preparation, formulation and pharmaceutical properties of farnesyltransferase inhibiting compounds of formula (VIII) 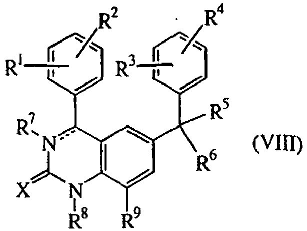 the pharmaceutically acceptable acid addition salts and the stereochemically isomeric forms thereof, wherein
the dotted line represents an optional bond;
- X: is oxygen or sulfur;
- R¹ and R²: each independently are hydrogen, hydroxy, halo, cyano, C₁₋₆alkyl, trihalomethyl, trihalomethoxy, C₂₋₆alkenyl, C₁₋₆alkyloxy, hydroxyC₁₋₆alkyloxy, C₁₋₆alkyloxyC₁₋₆alkyloxy, C₁₋₆alkyloxycarbonyl, aminoC₁₋₆alkyloxy, mono- or di(C₁₋₆alkyl)aminoC₁₋₆alkyloxy, Ar¹, Ar¹C₁₋₆alkyl, Ar¹oxy or Ar¹C₁₋₆alkyloxy;
- R³ and R⁴: each independently are hydrogen, halo, cyano, C₁₋₆alkyl, C₁₋₆alkyloxy, Ar¹oxy, C₁₋₆alkylthio, di(C₁₋₆alkyl)amino, trihalomethyl or trihalomethoxy;
- R⁵: is hydrogen, halo, C₁₋₆alkyl, cyano, haloC₁₋₆alkyl, hydroxyC₁₋₆alkyl, cyanoC₁₋₆alkyl, aminoC₁₋₆alkyl, C₁₋₆alkyloxyC₁₋₆alkyl, C₁₋₆alkylthioC₁₋₆alkyl, aminocarbonylC₁₋₆alkyl, C₁₋₆alkyloxycarbonylC₁₋₆alkyl, C₁₋₆alkylcarbonyl-C₁₋₆alkyl, C₁₋₆alkyloxycarbonyl, mono- or di(C₁₋₆alkyl)aminoC₁₋₆alkyl, Ar¹, Ar¹C₁₋₆alkyloxyC₁₋₆alkyl; or a radical of formula

-O-R¹⁰ (a-1),

-S-R¹⁰ (a-2),

-N-R¹¹R¹² (a-3),

wherein R¹⁰ is hydrogen, C₁₋₆alkyl, C₁₋₆alkylcarbonyl, Ar¹, Ar¹C₁₋₆alkyl, C₁₋₆alkyloxycarbonylC₁₋₆alkyl, or a radical of formula -Alk-OR¹³ or -Alk-NR¹⁴R¹⁵;
- R¹¹: is hydrogen, C₁₋₆alkyl, Ar¹ or Ar¹C₁₋₆alkyl;
- R¹²: is hydrogen, C₁₋₆alkyl, C₁₋₆alkylcarbonyl, C₁₋₆alkyloxycarbonyl, C₁₋₆alkylaminocarbonyl, Ar¹, Ar¹C₁₋₆alkyl, C₁₋₆alkylcarbonyl-C₁₋₆alkyl, Ar¹carbonyl, Ar¹C₁₋₆alkylcarbonyl, aminocarbonylcarbonyl, C₁₋₆alkyloxyC₁₋₆alkylcarbonyl, hydroxy, C₁₋₆alkyloxy, aminocarbonyl, di(C₁₋₆alkyl)aminoC₁₋₆alkylcarbonyl, amino, C₁₋₆alkylamino, C₁₋₆alkylcarbonylamino, or a radical of formula -Alk-OR¹³ or -Alk-NR¹⁴R¹⁵; wherein Alk is C₁₋₆alkanediyl ;
- R¹³: is hydrogen, C₁₋₆alkyl, C₁₋₆alkylcarbonyl, hydroxyC₁₋₆alkyl, Ar¹ or Ar¹C₁₋₆alkyl;
- R¹⁴: is hydrogen, C₁₋₆alkyl, Ar¹ or Ar¹C₁₋₆alkyl;
- R¹⁵: is hydrogen, C₁₋₆alkyl, C₁₋₆alkylcarbonyl, Ar¹ or Ar¹C₁₋₆alkyl;
- R⁶: is a radical of formula 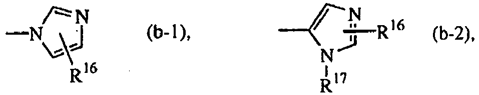
wherein R¹⁶ is hydrogen, halo, Ar¹, C₁₋₆alkyl, hydroxyC₁₋₆alkyl,
C₁₋₆alkyloxyC₁₋₆alkyl, C₁₋₆alkyloxy, C₁₋₆alkylthio, amino,
C₁₋₆alkyloxycarbonyl, C₁₋₆alkylthioC₁₋₆alkyl,
C₁₋₆alkylS(O)C₁₋₆alkyl or C₁₋₆alkylS(O)₂C₁₋₆alkyl;
R¹⁷ is hydrogen, C₁₋₆alkyl or di(C₁₋₄alkyl)aminosulfonyl;
- R⁷: is hydrogen or C₁₋₆alkyl provided that the dotted line does not represent a bond;
- R⁸: is hydrogen, C₁₋₆alkyl or Ar²CH₂ or Het¹CH₂;
- R⁹: is hydrogen, C₁₋₆alkyl, C₁₋₆alkyloxy or halo; or
- R⁸ and R⁹: taken together to form a bivalent radical of formula

-CH=CH- (c-1),

-CH₂-CH₂- (c-2),

-CH₂-CH₂-CH₂- (c-3),

-CH₂-O- (c-4),

or

-CH₂-CH₂-O- (c-5);
- Ar¹: is phenyl; or phenyl substituted with 1 or 2 substituents each independently selected from halo, C₁₋₆alkyl, C₁₋₆alkyloxy or trifluoromethyl;
- Ar²: is phenyl; or phenyl substituted with 1 or 2 substituents each independently selected from halo, C₁₋₆alkyl, C₁₋₆alkyloxy or trifluoromethyl; and
- Het¹: is pyridinyl; pyridinyl substituted with 1 or 2 substituents each independently selected from halo, C₁₋₆alkyl, C₁₋₆alkyloxy or trifluoromethyl.

WO-00/39082 and U.S. Patent No. 6,458,800 describe the preparation, formulation and pharmaceutical properties of farnesyltransferase inhibiting compounds of formula (IX) 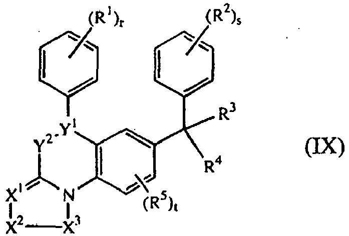 or the pharmaceutically acceptable acid addition salts and the stereochemically isomeric forms thereof, wherein
=X¹-X²-X³- is a trivalent radical of formula

=N-CR⁶=CR⁷- (x-1),

=CR⁶-CR⁷=CR⁸- (x-6),

=N-N=CR⁶- (x-2),

=CR⁶-N=CR⁷- (x-7),

=N-NH-C(=O)- (x-3),

=CR⁶-NH-C(=O)- (x-8),

or

=N-N=N- (x-4),

=CR⁶-N=N- (x-9);

=N-CR⁶=N- (x-5),

wherein each R⁶, R⁷ and R⁸ are independently hydrogen, C₁₋₄alkyl, hydroxy, C₁₋₄alkyloxy, aryloxy, C₁₋₄alkyloxycarbonyl, hydroxyC₁₋₄alkyl, C₁₋₄alkyloxyC₁₋₄alkyl, mono- or di(C₁₋₄alkyl)aminoC₁₋₄alkyl, cyano, amino, thio, C₁₋₄alkylthio, arylthio or aryl;
>Y¹-Y²- is a trivalent radical of formula

>CH-CHR⁹- (y-1),

>C=N- (y-2),

>CH-NR⁹- (y-3),

or

>C=CR⁹- (y-4);

wherein each R⁹ independently is hydrogen, halo, halocarbonyl, aminocarbonyl, hydroxyC₁₋₄alkyl, cyano, carboxyl, C₁₋₄alkyl, C₁₋₄alkyloxy, C₁₋₄alkyloxyC₁₋₄alkyl, C₁₋₄alkyloxycarbonyl, mono- or di(C₁₋₄alkyl)amino, mono- or di(C₁₋₄alkyl)aminoC₁₋₄alkyl, aryl;
r and s are each independently 0, 1, 2, 3, 4 or 5;
t is 0, 1, 2 or 3;
each R¹ and R² are independently hydroxy, halo, cyano, C₁₋₆alkyl, trihalomethyl, trihalomethoxy, C₂₋₆alkenyl, C₁₋₆alkyloxy, hydroxyC₁₋₆alkyloxy, C₁₋₆alkylthio, C₁₋₆alkyloxyC₁₋₆alkyloxy, C₁₋₆alkyloxycarbonyl, aminoC₁₋₆alkyloxy, mono- or di(C₁₋₆alkyl)amino, mono- or di(C₁₋₆alkyl)aminoC₁₋₆alkyloxy, aryl, arylC₁₋₆alkyl, aryloxy or arylC₁₋₆alkyloxy, hydroxycarbonyl, C₁₋₆alkyloxycarbonyl, aminocarbonyl, aminoC₁₋₆alkyl, mono- or di(C₁₋₆alkyl)aminocarbonyl, mono- or di(C₁₋₆alkyl)aminoC₁₋₆alkyl; or
two R¹ or R² substituents adjacent to one another on the phenyl ring may independently form together a bivalent radical of formula

-O-CH₂-O- (a-1),

-O-CH₂-CH₂-O- (a-2),

-O=CH=CH- (a-3),

-O-CH₂-CH₂- (a-4),

-O-CH₂-CH₂- CH2- (a-5),

or

-CH=CH-CH=CH- (a-6);

R³ is hydrogen, halo, C₁₋₆alkyl, cyano, haloC₁₋₆alkyl, hydroxyC₁₋₆alkyl, cyanoC₁₋₆alkyl, aminoC₁₋₆alkyl, C₁₋₆alkyloxyC₁₋₆alkyl, C₁₋₆alkylthioC₁₋₆alkyl, aminocarbonylC₁₋₆alkyl, hydroxycarbonyl, hydroxycarbonylC₁₋₆alkyl, C₁₋₆alkyloxycarbonylC₁₋₆alkyl, C₁₋₆alkylcarbonylC₁₋₆alkyl, C₁₋₆alkyloxycarbonyl, aryl, arylC₁₋₆alkyloxyC₁₋₆alkyl, mono- or di(C₁₋₆alkyl)aminoC₁₋₆alkyl;
or a radical of formula

-O-R¹⁰ (b-1),

-S-R¹⁰ (b-2),

-NR¹¹ R¹² (b-3),

wherein R¹⁰ is hydrogen, C₁₋₆alkyl, C₁₋₆alkylcarbonyl, aryl, arylC₁₋₆alkyl, C₁₋₆alkyloxycarbonylC₁₋₆alkyl, or a radical of formula -Alk-OR¹³ or -Alk-NR¹⁴R¹⁵;
R¹¹ is hydrogen, C₁₋₆alkyl, aryl or arylC₁₋₆alkyl;
R¹² is hydrogen, C₁₋₆alkyl, aryl, hydroxy, amino, C₁₋₆alkyloxy, C₁₋₆alkylcarbonylC₁₋₆alkyl, arylC₁₋₆alkyl, C₁₋₆alkylcarbonylamino, mono- or di(C₁₋₆alkyl)amino, C₁₋₆alkylcarbonyl, aminocarbonyl, arylcarbonyl, haloC₁₋₆alkylcarbonyl, arylC₁₋₆alkylcarbonyl, C₁₋₆alkyloxycarbonyl, C₁₋₆alkyloxyC₁₋₆alkylcarbonyl, mono- or di(C₁₋₆alkyl)aminocarbonyl wherein the alkyl moiety may optionally be substituted by one or more substituents independently selected from aryl or C₁₋₃alkyloxycarbonyl, aminocarbonylcarbonyl, mono- or di(C₁₋₆alkyl)aminoC₁₋₆alkylcarbonyl, or a radical of formula -Alk-OR¹³ or -Alk-NR¹⁴R¹⁵;
wherein Alk is C₁₋₆alkanediyl;
R¹³ is hydrogen, C₁₋₆alkyl, C₁₋₆alkylcarbonyl, hydroxyC₁₋₆alkyl, aryl or arylC₁₋₆alkyl;
R¹⁴ is hydrogen, C₁₋₆alkyl, aryl or arylC₁₋₆alkyl;
R¹⁵ is hydrogen, C₁₋₆alkyl, C₁₋₆alkylcarbonyl, aryl or arylC₁₋₆alkyl; R⁴ is a radical of formula 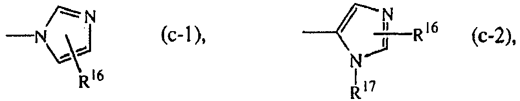 wherein R¹⁶ is hydrogen, halo, aryl, C₁₋₆alkyl, hydroxyC₁₋₆alkyl, C₁₋₆alkyloxyC₁₋₆alkyl, C₁₋₆alkyloxy, C₁₋₆alkylthio, amino, mono- or di(C₁₋₄alkyl)amino, hydroxycarbonyl, C₁₋₆alkyloxycarbonyl, C₁₋₆alkylthioC₁₋₆alkyl, C₁₋₆alkylS(O)C₁₋₆alkyl or C₁₋₆alkylS(O)₂C₁₋₆alkyl;
R¹⁶ may also be bound to one of the nitrogen atoms in the imidazole ring of formula (c-1) or (c-2), in which case the meaning of R¹⁶ when bound to the nitrogen is limited to hydrogen, aryl, C₁₋₆alkyl, hydroxyC₁₋₆alkyl, C₁₋₆alkyloxyC₁₋₆alkyl, C₁₋₆alkyloxycarbonyl, C₁₋₆alkylS(O)C₁₋₆alkyl or C₁₋₆alkylS(O)₂C₁₋₆alkyl;
R¹⁷ is hydrogen, C₁₋₆alkyl, C₁₋₆alkyloxyC₁₋₆alkyl, arylC₁₋₆alkyl, trifluoromethyl or di(C₁₋₄alkyl)aminosulfonyl;
R⁵ is C₁₋₆alkyl , C₁₋₆alkyloxy or halo;
aryl is phenyl, naphthalenyl or phenyl substituted with 1 or more substituents each independently selected from halo, C₁₋₆alkyl, C₁₋₆alkyloxy or trifluoromethyl.

Additionally, potent FTIs, including tipifarnib (also known as R115777 or ZARNESTRA^{®}), have been described in the literature. See, e.g., Kurzrock, R. et al. (2003), WO 98/43629, and WO 02/40015. Other known farnesyltransferase inhibitors include Arglabin (i.e.1(R)-10-epoxy-5(S),7(S)-guaia-3(4),11(13)-dien-6,12-olide described in WO-98/28303 (NuOncology Labs); perrilyl alcohol described in WO-99/45912 (Wisconsin Genetics); SCH-66336, i.e. (+)-(R)-4-[2-[4-(3,10-dibromo-8-chloro-5,6-dihydro-11H-benzo[5,6]cyclohepta[1,2-b]pyridin-11-yl)piperidin-1-yl]-2-oxoethyl]piperidine-1-carboxamide, described in U.S. Patent No. 5874442 (Schering); L778123, i.e. 1-(3-chlorophenyl)-4-[1-(4-cyanobenzyl)-5-imidazolylmethyl]-2-piperazinone, described in WO-00/01691 (Merck); compound 2(S)-[2(S)-[2(R)-amino-3-mercapto]propylamino-3(S)-methyl]-pentyloxy-3-phenylpropionyl-methionine sulfone described in WO-94/10138 (Merck); and BMS 214662, i.e. (R)-2,3,4,5-tetrahydro-1-(1H-imidazol-4-ylmethyl)-3-(phenylmethyl)-4-(2-thienylsulphonyl)-1H-1,4-benzodiazapine-7-carbonitrile, described in WO 97/30992 (Bristol Myers Squibb); and Pfizer compounds (A) and (B) described in WO-00/12498 and WO-00/12499:

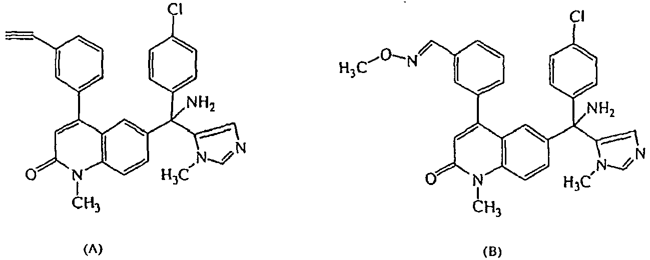

The use of certain of such FTIs has been studied in the area of anti-cancer therapy. More recently, the use of certain FTIs as therapeutic agents for treating inflammatory diseases, particularly those involving an immune component, and for treating pain associated with inflammatory conditions has been disclosed. See WO 98/43629, which describes the use of certain FTIs for treating conditions including ulcerative colitis, Crohn's disease, allergic rhinitis, graft-vs-host disease, conjunctivitis, asthma, rheumatoid arthritis, osteoarthritis, ARDS, Behcet's disease, transplant rejection, uticaria, allergic dermatitis, allopecia areata, scleroderma, exanthem, eczema, dermatomyositis, acne, diabetes, systemic lupus erythematosis, Kawasaki's disease, multiple sclerosis, emphysema, cystic fibrosis, chronic bronchitis, and psoriasis.

Raponi M et al ("Microarray analysis reveals genetic pathways modulated by tipifarnib in acute myeloid leukemia", BMC CANCER, vol. 4, no. 1, 2004, page 56) discloses genetic pathways modulated by tipifarnib in acute myeloid leukemia.

Although various FTIs are known, their biological effects in cells and animals, and therefore their therapeutic applications, have not been fully elucidated.

### SUMMARY OF THE INVENTION

In one general aspect, the disclosure relates to a method of treating a subject for sepsis or septic shock, comprising administering to a subject in need of such treatment a therapeutically effective amount of a farnesyltransferase inhibitor. Preferably, the farnesyltransferase inhibitor is selected from the farnesyltransferase inhibitor compounds disclosed herein. More preferably, the farnesyltransferase inhibitor is tipifarnib.

In a general aspect, the invention relates to methods for determining a subject's response to treatment with a farnesyltransferase inhibitor as defined in the claims. One general embodiment of such a method comprises: (a) measuring a level of at least one of IL-6, MCP-1, IL-1β, MMP-9, and TNF-α in an untreated blood sample which is a blood sampletaken from the subject before treatment with the farnesyltransferase inhibitor, or an extract thereof, measuring a level of said at least one of IL-6, MCP-1, IL-1β, MMP-9, and TNF-α in each treated blood sample which is a blood sampletaken from a subject at least one time after treatment with the farnesyltransferase inhibitor has commenced, or an extract thereof, and comparing the measured levels, where a decrease indicates a response to the treatment; wherein the method optionally comprises (b) isolating cells, plasma or serum from the untreated blood sample to obtain an untreated sample extract, and isolating cells, plasma or serum from each treated blood sample to obtain a treated sample extract and/or optionally measuring a level of IL-8 in the untreated blood sample or extract, measuring a level of IL-8 in each treated blood sample or extract, and comparing the levels of IL-8 to obtain a control measurement.

In one preferred embodiment, the extracts are plasma samples (isolated by, e.g., centrifuging the blood). In another preferred embodiment, the extracts are peripheral blood mononuclear cells isolated by, e.g., Ficoll-Paque gradient. Before measuring, the blood or cells may be stimulated with LPS.

Another general embodiment relates to a method for monitoring a subject's response to treatment with a farnesyltransferase inhibitor, comprising: (a) optionally isolating peripheral blood mononuclear cells (PBMC) from an untreated blood sample which is a blood sample taken from the subject before treatment with the farriesyltransferase inhibitor to obtain an untreated PBMC sample, and isolating peripheral blood mononuclear cells from each treated blood sample which is a blood sample taken from the subject at least one time after treatment with the farnesyltransferase inhibitor has commenced to obtain a treated PBMC sample; (c) stimulating the untreated blood or PBMC sample with lipopolysaccharide to obtain an LPS-stimulated untreated blood or PBMC sample, and stimulating each treated blood or PBMC sample with lipopolysaccharide to obtain an LPS-stimulated treated blood or PBMC sample; (d) isolating RNA of the untreated LPS-stimulated blood or PBMC sample, and isolating RNA of each treated LPS-stimulated blood or PBMC sample; (e) optionally measuring expression of IL-8 in the RNA of the untreated LPS-stimulated blood or PBMC sample, measuring expression of IL-8 in the RNA of each treated LPS-stimulated blood or PBMC sample, and comparing said measurements of RNA expression of IL-8 as a control; (f) measuring expression of at least one of IL-1β, MCP-1, MMP-9, MyD88, STAT1, and IL-6 in the RNA of the untreated LPS-stimulated blood or PBMC sample, measuring expression of at least one of IL- 1β, MCP-1, MMP-9, MyD88, STAT1, and IL-6 in the RNA of each treated LPS-stimulated blood or PBMC sample, and comparing the expression measurements, where a decrease indicates a response to the treatment.

In preferred embodiments, plural treated blood samples from the subject (e.g., a patient in a clinical trial) are taken over periodic intervals of time after treatment with the farnesyltransferase inhibitor has commenced.

Other embodiments, features, advantages, and aspects of the invention will become apparent from the detailed description below in reference to the drawing figures.

### BRIEF DESCRIPTION OF DRAWINGS

**Figure 1** shows the mevalonate pathway for synthesis of isoprenoids and cholesterol, indicating the sites for inhibition of HMG CoA reductase by simvastatin, and of farnesyltransferase activity by tipifarnib. Inhibition of HMG CoA reductase by statins leads to inhibition of cholesterol synthesis, and of isoprenoid production, required for both farnesylation and geranylgeranylation of proteins. Inhibition of farnesyltransferase by FTIs selectively inhibits farnesylation of proteins such as Ras.

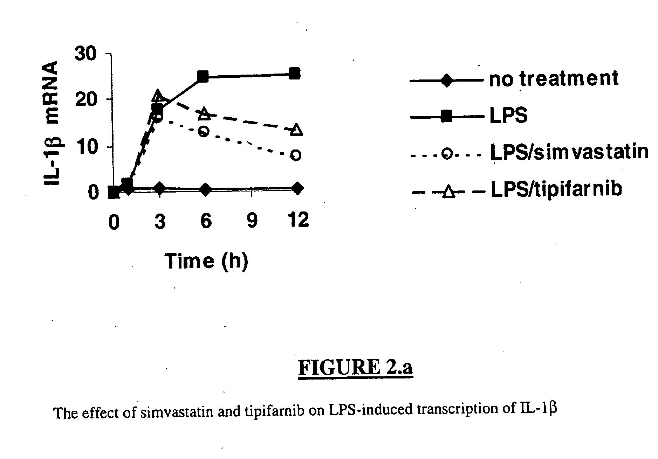

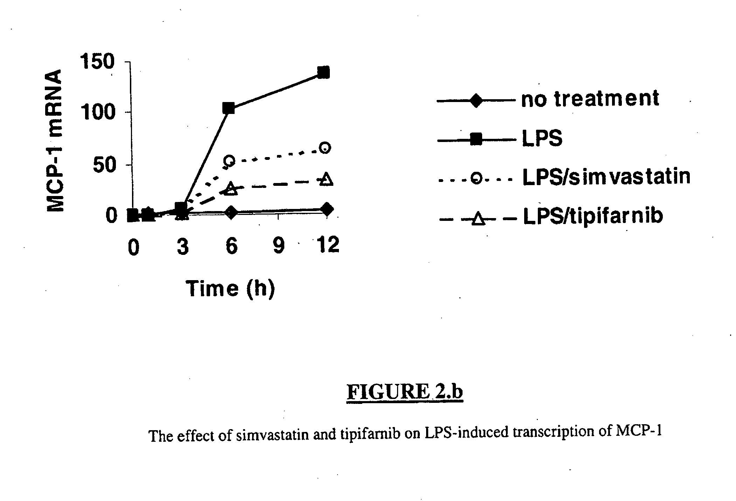

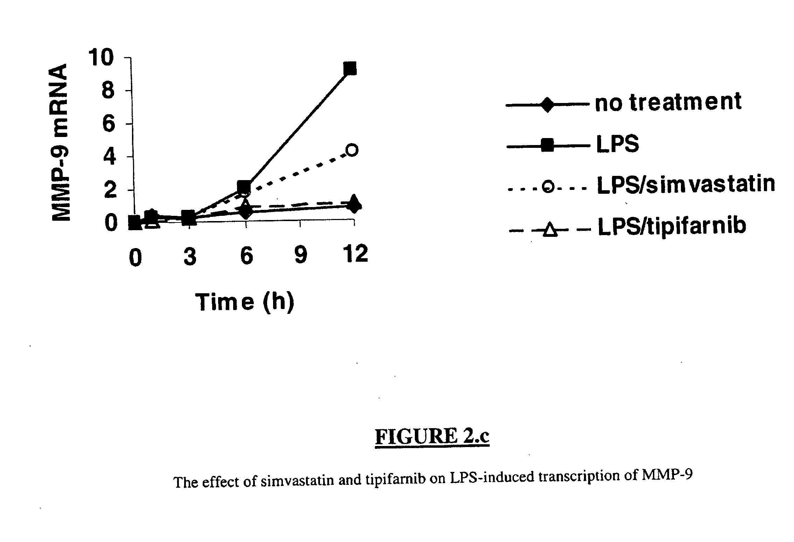

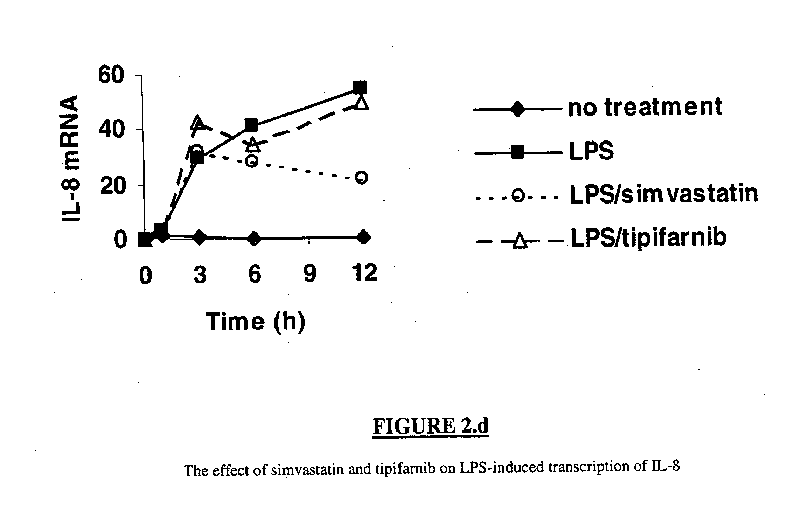

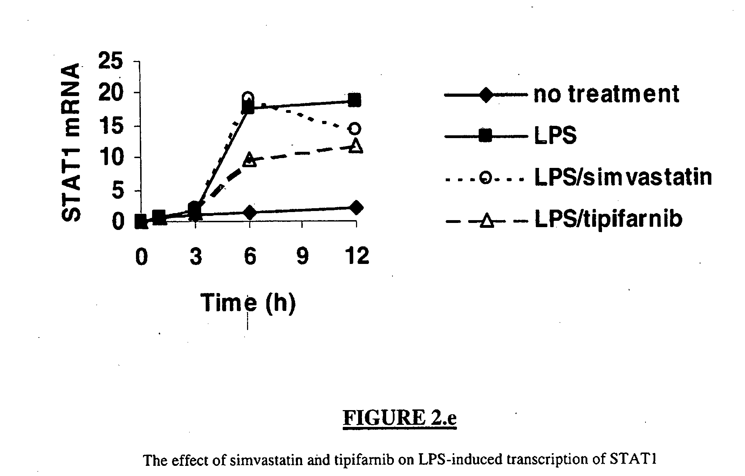

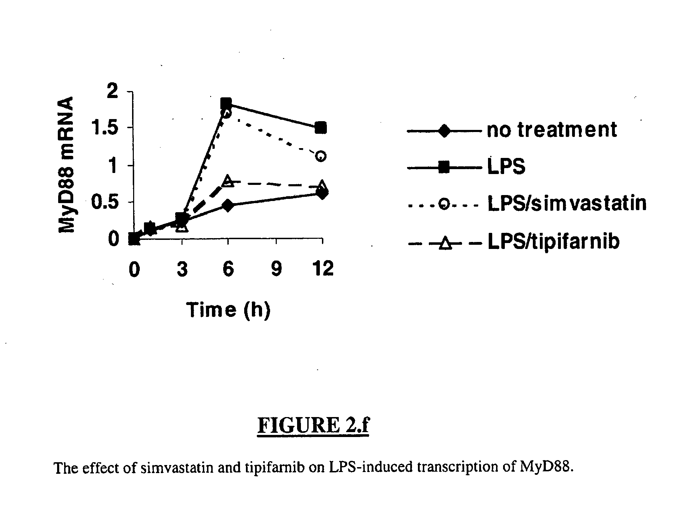

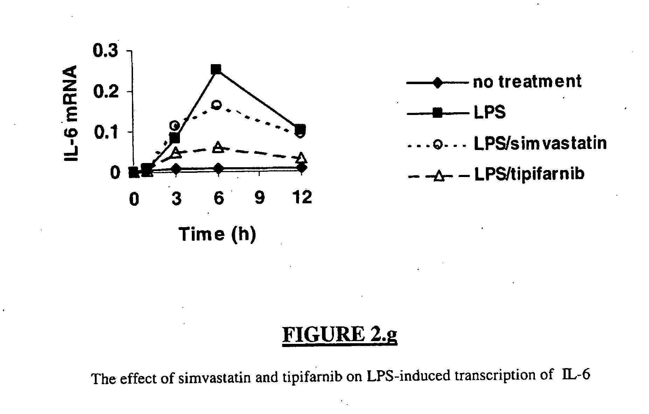

**Figures 2a-2g** show the effect of simvastatin and tipifarnib on LPS-induced transcription of IL-1β **(****Figure 2.a**), MCP-1 **(****Figure 2.b**), MMP-9 **(****Figure 2****.c**), IL-8 **(****Figure 2.d**), STAT1 **(****Figure 2.e**), MyD88 **(****Figure 2.f**) and IL-6 **(****Figure 2****.g**). THP-1 cells were cultured in the absence (no treatment) or presence of LPS (100 ng/ml), plus or minus simvastatin (10 µM), or tipifarnib (5 µM). RNA was isolated at the indicated times for cDNA preparation as described in "Materials and Methods". Relative mRNA levels compared to GAPDH were determined by real-time PCR.

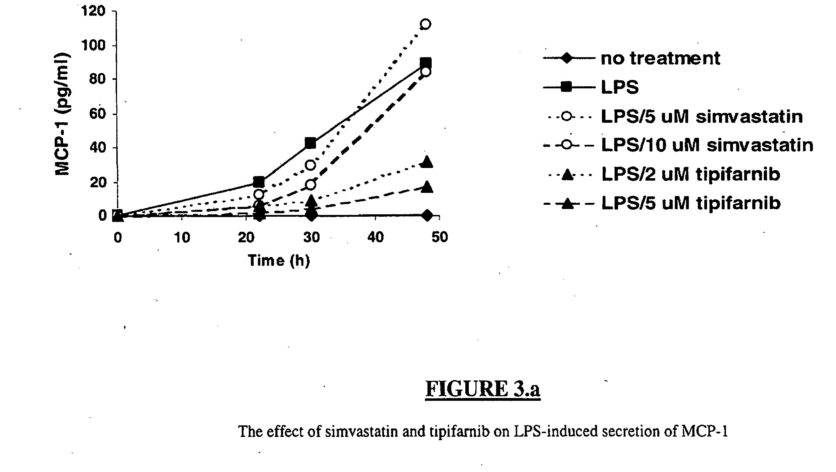

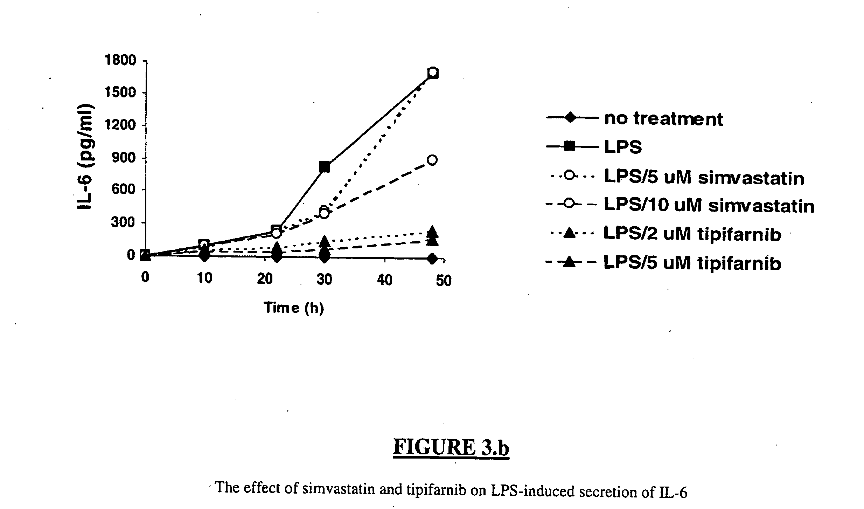

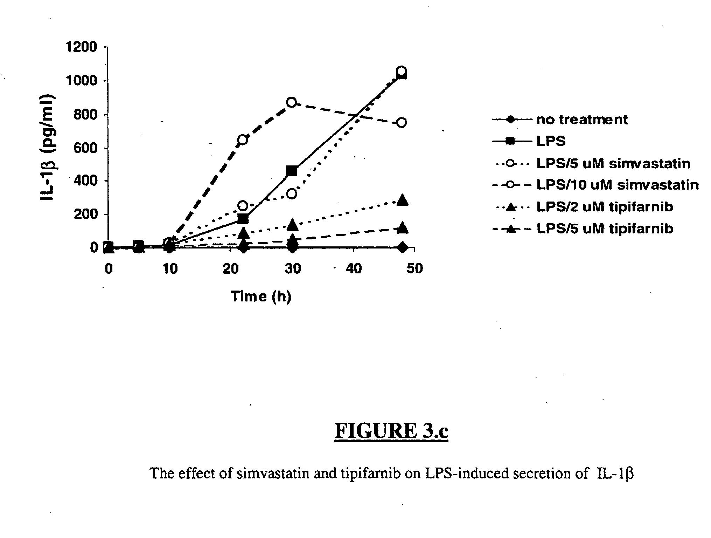

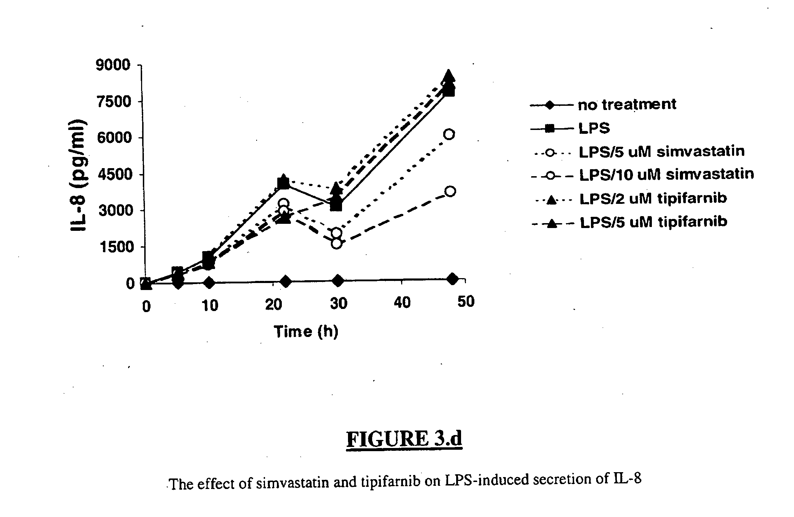

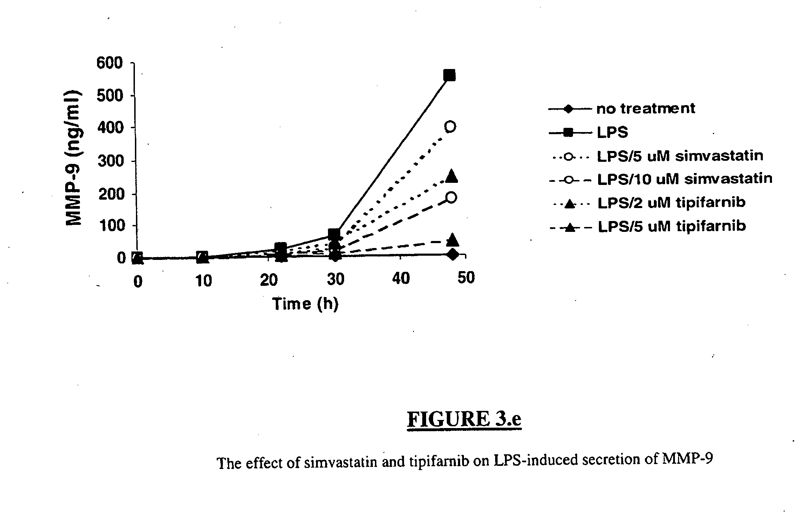

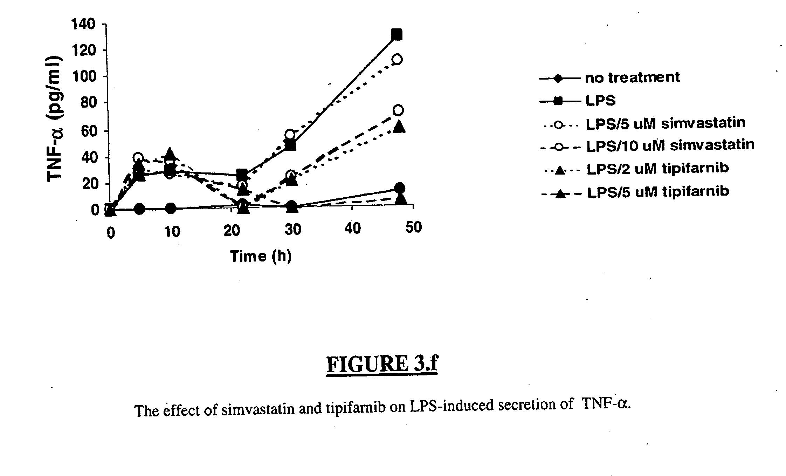

**Figures 3a-3f** show the effect of simvastatin and tipifarnib on LPS-induced secretion of MCP-1 **(****Figure 3****.a**), IL-6 **(****Figure 3****.b**), IL-1β **(****Figure 3****.c**), IL-8 **(****Figure 3****.d**), MMP-9 **(****Figure 3****.e**) and TNF-α **(****Figure 3.f**). THP-1 cells cultured in the absence (no treatment) or presence of 100ng/ml LPS, plus or minus simvastatin (5 and 10µM) or tipifarnib (2 and 5 µM), respectively. At the indicated time points, supernatanats were analyzed for cytokines, chemokines or MMP-9 by ELISA. Data points are means of triplicate incubations, for which standard deviations were typically 2 to 13 % of the mean value.

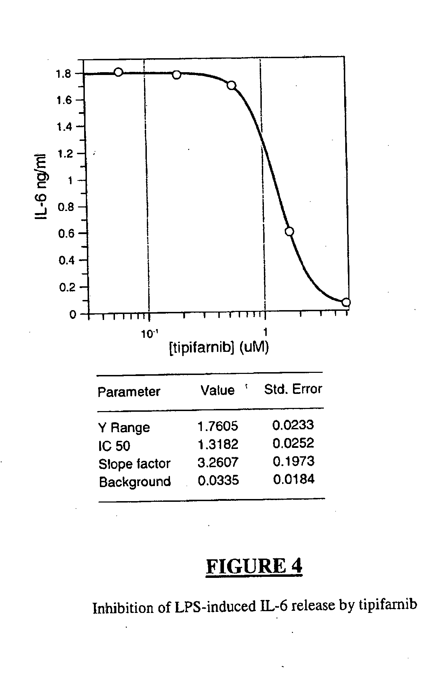

**Figure 4** shows inhibition of LPS-induced IL-6 release by tipifarnib. THP-1 cells at 3.4 x 10⁵/ml were cultured in 96-well plates in 0.5%FBS/RPMI 1640, in the absence or presence of 100ng/ml LPS, plus or minus tipifarnib (starting from 5 µM, followed by 3-fold series dilution). The concentrations of tipifarnib were: 5 µM, 1.67 µM, 0.56 µM, 0.19 µM and 0.06 µM. Supernatants were collected at 48hr. IL6 was quantified using ELISA kit from R&D Systems.

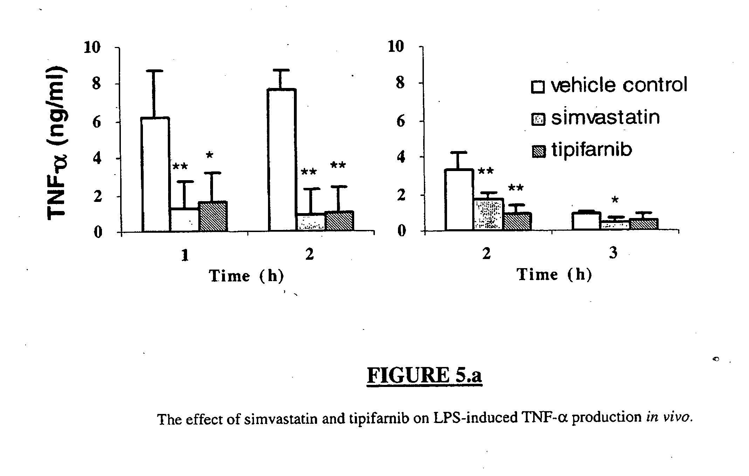

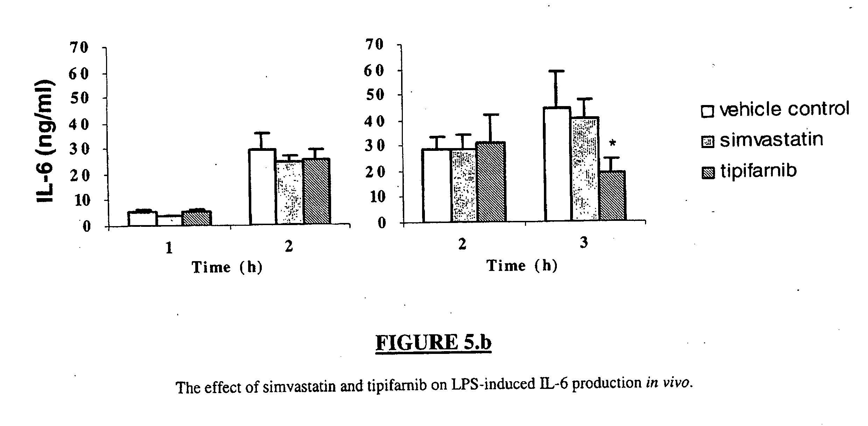

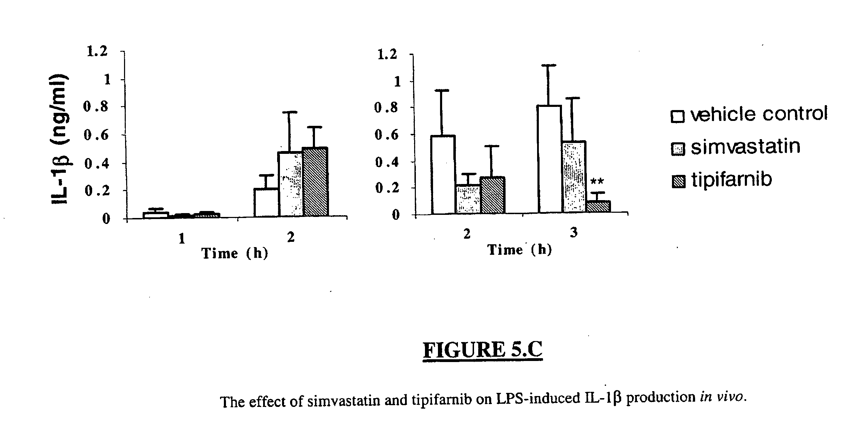

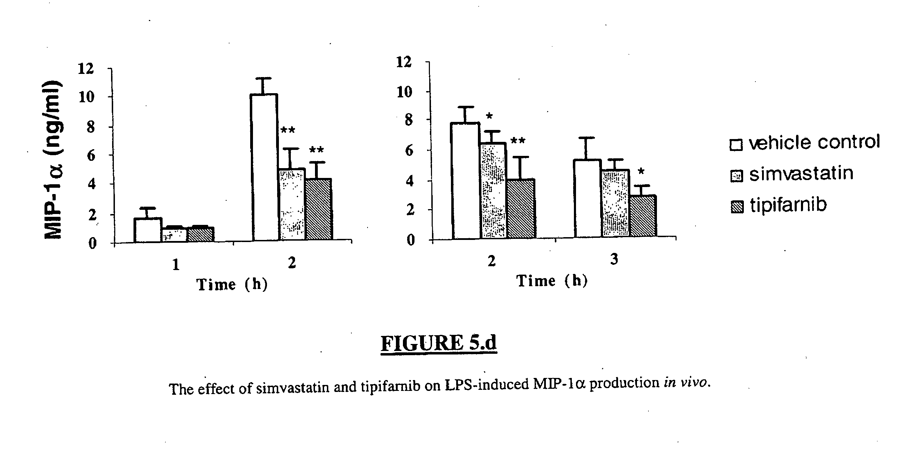

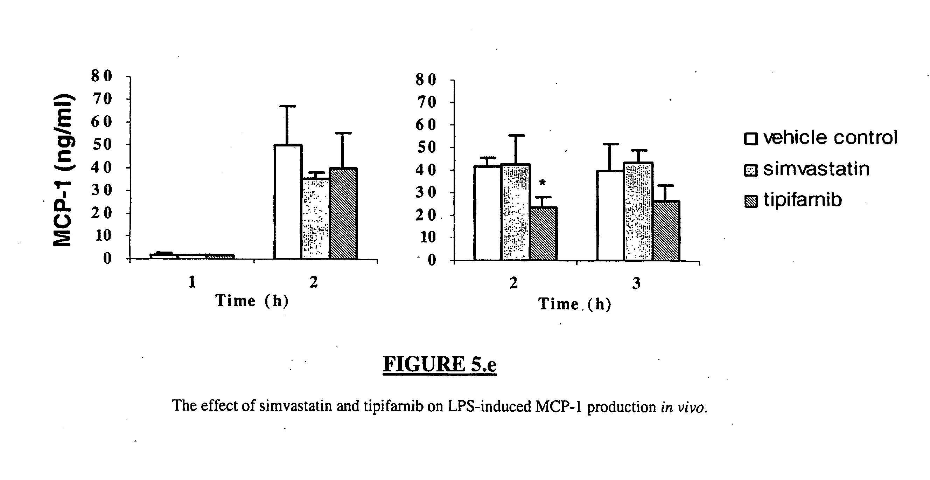

**Figures 5a-5e** show the effect of simvastatin and tipifarnib on LPS-induced cytokine production *in vivo.* BALB/c mice (n = 4 or 5 per group) were treated orally with simvastatin or tipifarnib in 20% cyclodextran, at a dose of 50mg/kg, 24 hours, 17 hours and 1 hour before LPS injection. LPS (20 µg) was administered intraperitoneally. Plasma samples were collected at 1 hour and 2 hours in the first experiment, and 2 hours and 3 hours after LPS injection in the second experiment, and tested for TNF-α (**Figure 5****.a**), IL-6 (**Figure 5****.b**), IL-1β (**Figure 5****.c**), MIP-1α (**Figure 5****.d**) and MCP-1 **(****Figure 5****.e**), using ELISA or Luminex as described in "Materials and Methods" herein. Data are presented as the mean values for 4 or 5 mice in each group, and the standard deviations from the means are indicated by error bars. Statistical significance for differences in production of inflammatory mediators in simvastatin- or tipifarnibversus vehicle-treated animals was assessed using the Student's t test. The statistically significant differences are indicated by asterisks in Figures 5a through 5e as follows: * means P value less than 0.05; ** means P value less than 0.01. The lower the P value, the more significant the difference.

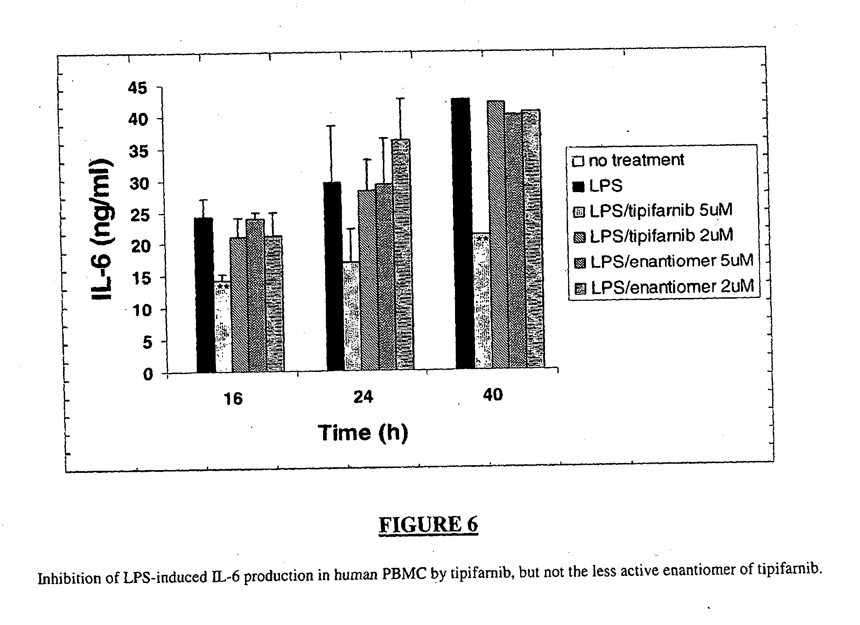

**Figure 6** shows inhibition of LPS-induced IL-6 production in human PBMC by tipfarnib, but not by the less active enantiomer of tipifarnib. Human PBMC were incubated at 37°C for 1 hour in the absence or presence of 5 µM and 2 µM tipifarnib (R115777) or its less active enantiomer (100-fold less active for inhibition of farnesyltransferase) after which LPS (or vehicle only for no treatment) was added to a final concentration of 10 ng/ml. Supernatants were collected at 16 hours, 24 hours and 40 hours of LPS treatment, and IL-6 secretion was quantified by ELISA. Statistical significance for differences in production of IL-6 in tipifarnib- versus vehicle-treated PBMC was assessed using the Student's *t* test. The statistically significant differences are indicated by asterisks ** means P value less than 0.01. The lower the P value, the more significant the difference.

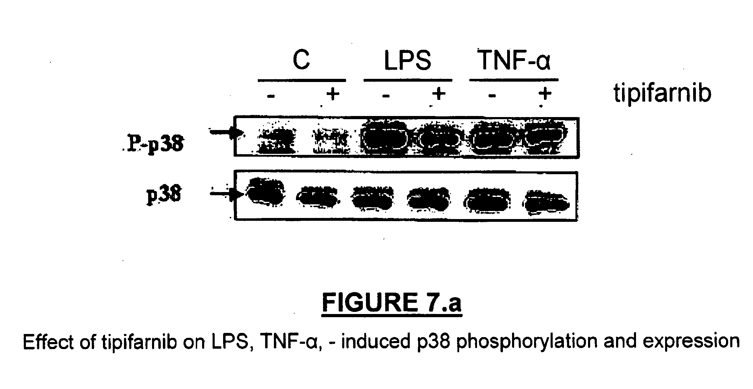

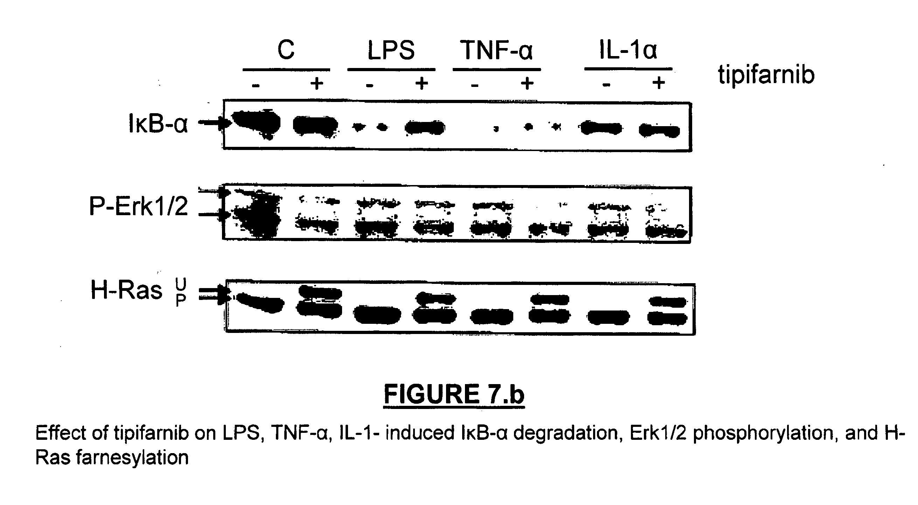

**Figure 7a-7b** shows the effect of tipifarnib on LPS, TNF-α, and IL-1-induced signaling. THP-1 cells were cultured in the absence or presence of 2 µM tipifarnib for 18 hours, then incubated with LPS and TNF-α (**Figure 7****.a**), or LPS, TNF-α and IL-1α (**Figure 7****.b**) for 30 minutes. Cell lysates were analyzed by Western blotting, using antibodies against p38, phosphorylated p38, phospho-ERK1/2, IκB, and H-Ras.

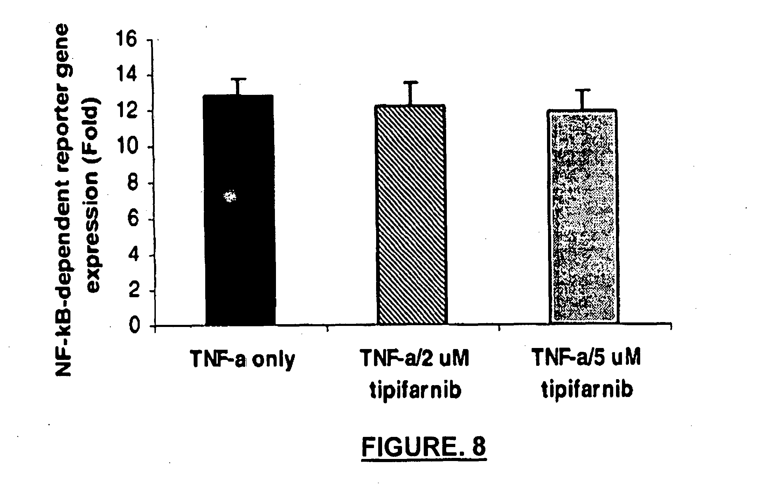

**Figure 8** shows that tipifarnib does not inhibit NF-κB-dependent reporter gene expression induced by TNF-α. NF-κB-LH transfected HEK293 cells were incubated in the absence or presence of 2 and 5 µM tipifarnib for 18 hours, then stimulated with TNF-α for 4 hours. Luciferase activity was measured and expressed as fold-change in the presence of TNF-α versus no TNF-α, or TNF-α/tipifarnib versus tipifarnib. Values are presented as fold increase ± S.D.

### DETAILED DESCRIPTION OF THE INVENTI0N AND PREFERRED EMBODIMENTS

The terms "comprising", "including", and "containing" are used herein in their open, non-limited sense.

The biological effects of FTIs have now been further elucidated, as discussed in the Experimentals section below. Inhibition of farnesyltransferase (FPTase) has been found to downregulate the genes and proteins identified in Tables 2, 3, and 4. Thus, the levels of such genes and protein may be monitored as biological markers to determine a patient's response to treatment with an FTI. For example, various subsets of LPS-induced genes and/or proteins shown in the following tables may be downregulated by administration of a farnesyltransferase inhibitor and therefore used as markers in clinical studies.

**Table 2: Genes for which LPS-induced transcription was downregulated by inhibition of farnesyltransferase, as measured by cDNA microarray analysis**

| Accession number | GeneName | GeneDescription |
|---|---|---|
| NM_002982 | CCL2 | monocyte chemotactic protein 1 (MCP-1) |
| NM_005623 | CCL8 | monocyte chemotactic protein 2 (MCP-2) |
| NM_002983 | CCL3 | small inducible cytokine A3 (homologous to mouse Mip-1a) (SCYA3) |
| NM_002984 | CCL4 | small inducible cytokine A4 (homologous to mouse Mip-1b) (SCYA4) |
| NM_005409 | CXCL11 | small inducible cytokine subfamily B, member 11 (SCYB11) |
| NM_000576 | IL1B | interleukin 1, beta |
| NM_000577 | IL1RN | interleukin-1 receptor antagonist |
| NM_002852 | PTX3 | pentaxin-related gene, rapidly induced by IL-1B |
| NM_007315 | STAT1 | signal transducer and activator of transcription 1 |
| NM_002176 | IFNB1 | interferon, beta 1, fibroblast |
| NM_005531 | IFI16 | interferon, gamma-inducible protein 16 |
| NM_001548 | IFIT1 | interferon-induced protein with tetratricopeptide repeats 1 |
| NM_002462 | MX1 | myxovirus (influenza) resistance 1 |
| NM_000598 | IGFBP3 | insulin-like growth factor-binding protein-3 gene |
| NM_053056 | CCND1 | cyclin D1 (PRAD1: parathyroid adenomatosis 1) |
| NM_002965 | S100A9 | S100 calcium-binding protein A9 (calgranulin B) |
| NM_002468 | MYD88 | myleoid differentiation primary response protein |
| NM_000593 | TAP1 | transporter 1, ATP-binding cassette, sub-family B (MDR/TAP) |
| NM_000544 | TAP2 | transporter 2, ATP-binding cassette, sub-family B (MDR/TAP) |
| NM_007188 | ABCBB | ATP-binding cassette, sub-family B (MDR/TAP), member 8 |
| NM_031460 | KCNK17 | potassium channel, subfamily K, member 17 (TASK-4) |
| NM_002659 | PLAUR | plasminogen activator, urokinase receptor |
| NM_004054 | C3AR1 | C3a anaphylatoxin chemotactic receptor (C3a-R) |
| NM_004048 | B2M | beta-2-microglobulin |
| NM_015907 | LAP3 | leucine aminopeptidase |
| NM_004994 | MMP9 | type IV collagenase, matrix metalloproteinase 9 |
| NM_006074 | TRIM22 | tripartite motif-containing 22 |
| NM_144573 | NEXN | nexilin (F actin bindinq protein) |
| NM_021634 | LGR7 | leucine-rich repeat-containing G protein-coupled receptor 7 |
| NM_198066 | GNPNAT1 | glucosamine-phosphate N-acetyltransferase 1 |
| NM_002346 | LY6E | lymphocyte antigen 6 complex, locus E |

**Table 3: Genes for which LPS-induced transcription was downregulated by inhibition of farnesyltransferase, as measured by reverse transcriptase and real-time PCR analysis**

| Accession number | GeneName | GeneDescription |
|---|---|---|
| NM_000576 | IL1B | interleukin 1, beta |
| NM_002982 | CCL2 | monocyte chemotactic protein 1 (MCP-1) |
| NM_004994 | MMP9 | type IV collagenase, matrix metalloproteinase 9 |
| NM_007315 | STAT1 | signal transducer and activator of transcription 1 |
| NM_002468 | MYD88 | myleoid differentiation primary response protein |
| NM_000600 | IL6 | interleukin 6 |

**Table 4 Proteins for which LPS-induced production was downregulated by inhibition of farnesyltransferase, as measured by ELISA or Luminex analysis**

| Accession number | Protein name | Protein description |
|---|---|---|
| NP_000585 | TNF-α | tumor necrosis factor (TNF superfamily, member 2) |
| NP_000591 | IL-6 | interleukin 6, interferon beta 2 |
| NP_000567 | IL-1β | interleukin 1, beta proprotein |
| NP_002973 | CCL2 | MCP-1, small inducible cytokine A2 precursor |
| NP_002974 | CCL3 | small inducible cytokine A3 (homologous to mouse Mip-1a) |
| AAG32620 | IL12p40 | interleukin 12 p40 subunit |
| NP_004985 | MMP-9 | type IV collagenase, matrix metalloproteinase 9 |

As described above, one or more of the above-identified genes and proteins may be used as a marker to study a subject's response to treatment with an FTI.

Inhibition of FPTase may decrease LPS-induced transcription of, e.g., IL-1β, MCP-1, MMP-9, MyD88, STAT1, MIP-1α, TNF-α or IL-6. Consequently, transcription of IL-1β, MCP-1, MMP-9, MyD88, STAT1, MIP-1α, TNF-α or IL-6 may be decreased by administration of a farnesyltransferase inhibitor and these may be used as a panel of markers for monitoring patients in a clinical study. Inhibition of FPTase may also decrease or inhibit transcription of IL-1β, MCP-1, MMP-9, MyD88, STAT1, MIP-1α, TNF-α or IL-6 induced by agents other than LPS. Thus, administration of an FTI to a patient may be useful to treat diseases and medical conditions mediated through transcription of IL-1β, MCP-1, MMP-9, MyD88, STAT1, MIP-1α, TNF-α or IL-6. Inhibition of FPTase may also decrease LPS-induced protein translation or secretion of IL-1β, MCP-1, MMP-9, MyD88, STAT1, MIP-1α, TNF-α or IL-6. Thus, a disease or medical condition mediated by LPS-induced protein translation or secretion of IL-1β, MCP-1, MMP-9, MyD88, STAT1, MIP-1α, TNF-α or IL-6 may be treated by administering to a subject in need of such treatment a farnesyltransferase inhibitor, and the progress of treatment may be monitored by measuring levels of such markers. Furthermore, since inhibition of FPTase may serve to inhibit protein translation or secretion of IL-1β, MCP-1, MMP-9, MyD88, STAT1, MIP-1α, TNF-α or IL-6 induced by agents other than LPS, administration of an FTI may be useful to treat diseases and medical conditions mediated through protein translation or secretion of IL-1β, MCP-1, MMP-9, MyD88, STAT1, MIP-1α, TNF-α and/or IL-6. Inhibition of FPTase may also serve to inhibit transcription or translation of MyD88 or STAT1. Inhibition of transcription or protein translation or secretion of TNF-α, IL-1β, MCP-1, IL-6, MMP-9, or MIP-1α may also be attained by administration of a farnesyltransferase inhibitor, and the progress of treatment may be monitored by measuring levels of this set of markers.

Accordingly, FTIs may be used to treat inflammation and inflammatory conditions. Diseases and medical conditions that may benefit from treatment with an FTI include: atherosclerosis and other vascular diseases such as thrombosis, restenosis after angioplasty and/or stenting, and vein-graft disease after bypass surgery, as well as ulcerative colitis, Crohn's disease, allergic rhinitis, graft-versus-host disease, conjunctivitis, asthma, rheumatoid arthritis, osteoarthritis, ARDS, Behcet's disease, transplant rejection, urticaria, allergic dermatitis, alopecia areata, scleroderma, exanthema, eczema, dermatomyositis, acne, diabetes, systemic lupus erythematosis, Kawasaki's disease, multiple sclerosis, emphysema, cystic fibrosis, chronic bronchitis and psoriasis.

FTIs may also be used to treat LPS-induced inflammation in particular. Thus, a therapeutically effective amount of an FTI may be administered to treat a patient in need of treatment for one of the following diseases or medical conditions: sepsis, endotoxic shock, multiple organ dysfunction syndrome, periodontitis, peritonitis, anorexia, microvascular leukocytosis, pulmonary inflammation, airway hyperreactivity, acute lung injury, acute respiratory distress syndrome, chronic airway disease, goblet cell hyperplasia, pancreatitis, nephrotic syndrome, pyrexia, liver injury, premature delivery, Crohn's disease, ulcerative colitis, cerebral edema, atherosclerosis, cardiac failure, obstructive jaundice, neuroinflammation, cerebral ischemia, hyperalgesia, rectal allodynia, disseminated intravascular coagulation syndrome, hyperglycemia, insulin resistance, obstructive sleep apnea.

FTIs may also be used to treat atherosclerosis. Thus, a therapeutically effective amount of an FTI may be administered to a subject diagnosed with atherosclerosis to treat the subject in need of such treatment.

Furthermore, FTIs may be used to treat asthma. A therapeutically effective amount of an FTI may therefore be administered to a subject in need of treatment for asthma.

Additionally, a therapeutically effective amount of an FTI may be administered to a subject diagnosed with multiple sclerosis (MS) to treat the subject in need of such treatment.

Moreover, a therapeutically effective amount of an FTI may be administered to a subject diagnosed with or experiencing sepsis or septic shock to treat the subject in need of such treatment.

Accordingly, the monitoring methods of the invention are therefore useful in connection with determining FTI efficacy in treating the above-described indications.

Exemplary farnesyltransferase-inhibiting compounds useful in such treatments are described below. An especially preferred FTI is tipifamib.

The present invention provides various methods for determining a response of a subject suffering from sepsis ou septic shock to treatment with a farnesyltransferase inhibitor (FTI). In such methods, an untreated blood sample and an FTI-treated blood sample are taken. The following may be used as types of "blood samples": whole blood; plasma (e.g., separated by centrifugation); serum from plasma (e.g., obtained by clotting or serum separator tubes); or total cells or isolated subsets (e.g., PBMC).

As a control measurement useful with any of the above types of blood samples, IL-8 protein levels may optionally be determined before and after treatment with an FTI (e.g., tipifarnib), e.g., by using immunofluorescence staining, flow cytometry, immunocytochemistry, immunohistochemistry, immunoblotting, ELISA, or multiplex cytokine array analysis. As one skilled in the art will realize, other measurements may be used as a control, or the method of the present invention may be practiced without the use of a control. For example, in plasma or serum, levels of albumin protein can be used as a control. Additionally, the invention may be practiced without an internal control by comparing measurements in an untreated blood sample to an FTI- treated blood sample.

As the marker(s) useful for monitoring progress of treatment with any of the blood samples, protein levels for one or more of the genes or proteins in Tables 2, 3, and 4 may be measured before and after FTI treatment, e.g., by using any of the methods described above, where a decrease is indicative of a response to treatment.

Alternatively, with whole blood or total cells or particular subsets of cells (e.g., PBMC), IL-8 RNA levels may optionally be measured as a control before and after FTI treatment (under conditions where IL-8 levels do not change with treatment, this gene serves as a control), e.g., by performing RNase protection, reverse transcriptase (RT) polymerase chain reaction (PCR), RT and real-time quantitative PCR, Northern blotting, and cDNA- or oligonucleotide- microarray analysis. As one skilled in the art will realize, other measurements may be used as a control, or the method of the present invention may be practiced without the use of a control. Other commonly employed control measurements included within the scope of the present invention include mRNA levels of glyceraldehyde-3-phosphate dehydrogenase (GAPDH) or beta-actin. Additionally, the present invention may be practiced without an internal control by comparing measurements in an untreated blood sample to an FTI- treated blood sample.

For this general embodiment used with the specified types of blood samples, the RNA levels of one or more of the genes or proteins listed in Tables 2, 3, and 4 are measured before and after treatment with an FTI (where a decrease with treatment reflects response).

In another alternative general embodiment, whole blood, total cells, or PBMC or other subsets of cells are treated with LPS. As a control, levels of IL-8 are optionally measured in protein or RNA, or as otherwise known in the art, as described above. The levels of one or more markers of Tables 2, 3, and 4 are measured in LPS-treated samples of protein or RNA as in the other embodiments, with a decrease in protein/RNA levels with treatment with an FTI over time indicated response to the treatment. Likewise, an agent other than LPS that also induces RNA or protein for the marker(s) may be used in place of LPS to treat the samples, and then protein or RNA levels of optionally IL-8 (or other controls) and one or more markers may be measured as before to monitor response to FTI treatment.

In one preferred embodiment, the response of a subject undergoing such treatment comprises the following steps in a blood sample taken from said subject before FTI treatment and after FTI treatment, and optionally at different times during FTI treatment; (1) optionally separating cells and plasma (preferably by centrifugation); (2) optionally measuring a level of IL-8 or other control (preferably by immunoblotting , ELISA or multiplex cytokine array analysis) in the plasma before and after FTI treatment as a control; (3) measuring (preferably by immunoblotting or ELISA or multiplex cytokine array analysis) a level of one or more of the markers identified in Tables 2, 3, and 4, preferably one or more of IL-6, MCP-1, IL-1β, MMP-9, TNF-α in the plasma before and after FTI treatment; and (4) comparing the levels of such marker(s) measured before and after FTI treatment. In one particular embodiment, decreased levels of IL-6, MCP-1, IL-1β, MMP-9, MIP-1α and/or TNF-α after, and optionally during FTI treatment, relative to before FTI treatment, would indicate a response to FTI treatment.

In a further embodiment, the present invention provides a method for determining response to treatment with a farnesyltransferase inhibitor (FTI) in a blood sample taken from the subject before FTI treatment and after FTI treatment, and optionally at different times during FTI treatment; comprising (1) optionally isolating peripheral blood mononuclear cells (PBMC) from blood (preferably by Ficoll-Paque gradient separation); (3) stimulating the isolated PBMC with LPS (preferably 10 ng/ml LPS); (4) optionally measuring a level of IL-8 or other control (preferably by immunoblotting or ELISA or multiplex cytokine array analysis) in the plasma before and after FTI treatment as a control; (4) measuring (preferably by immunoblotting or ELISA or multiplex cytokine array analysis) a level of one or more of the markers of Tables 2, 3, and 4 (e.g., IL-6, MCP-1, DL-1β, MMP-9, MIP-1α, and/orTNF-α) in the PBMC before and after FTI treatment; and (5) comparing the levels of such marker(s) measured before and after FTI treatment, wherein decreased levels of marker(s) after, and optionally during FTI treatment, relative to before FTI treatment, would indicate a response to FTI treatment.

In another embodiment, the present invention provides a method for determining response to treatment with a farnesyltransferase inhibitor (FTI), in a subject in need of treatment therewith, comprising the following steps in a blood sample taken from said subject before FTI treatment, after FTI treatment, and optionally at different times during FTI treatment; (1) isolating peripheral blood mononuclear cells (PBMC) from blood (preferably by Ficoll-Paque gradient separation), (2) stimulating the isolated PBMC with LPS (preferably 10 ng/ml LPS); (3) isolating RNA from the blood or PBMC samples; (4) measuring RNA expression (preferably by Northern blotting, cDNA- or oligonucleotide- microarray analysis, or reverse transcriptase and real-time PCR) of one or more markers (preferably IL-1β, MCP-1, MMP-9, MyD88, STAT1 and/or IL-6) in the RNA samples; (5) optionally measuring RNA expression of IL-8 (or others) as a control; and (6) comparing the levels of the marker(s) in RNA measured before and after FTI treatment, wherein decreased LPS-induced expression of the marker(s) after, and optionally during FTI treatment, relative to before FTI treatment, would indicate a response to FTI treatment.

Examples of farnesyltransferase inhibitors which may be employed in the methods or treatments in accordance with the present disclosure include compounds of formula (I), (II), (III), (IV), (V), (VI), (VII), (VIII) or (IX) above. Preferred FTIs include compounds of formula (I), (II) or (III): 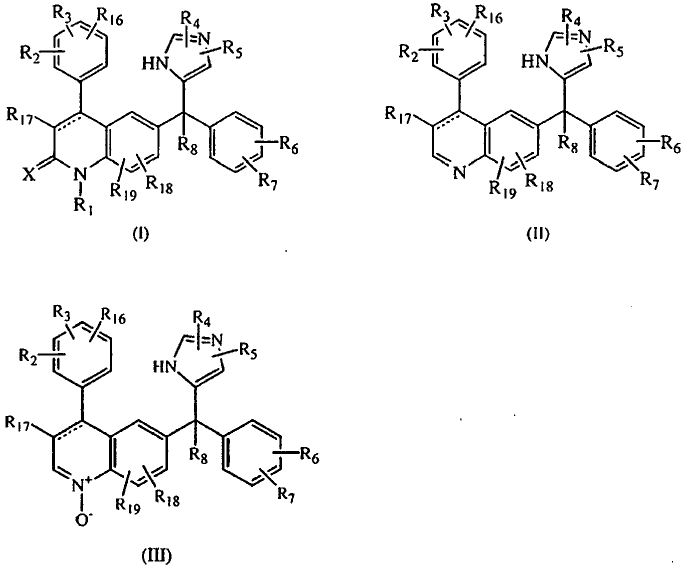 the pharmaceutically acceptable acid or base addition salts and the stereochemically isomeric forms thereof, wherein
the dotted line represents an optional bond;
- X: is oxygen or sulfur;
- R¹: is hydrogen, C₁₋₁₂alkyl, Ar¹, Ar²C₁₋₆alkyl, quinolinylC₁₋₆alkyl, pyridylC₁₋₆alkyl, hydroxyC₁₋₆alkyl, C₁₋₆alkyloxyC₁₋₆alkyl, mono- or di(C₁₋₆alkyl)aminoC₁₋₆alkyl, aminoC₁₋₆alkyl or a radical of formula -Alk¹-C(=O)-R⁹, -Alk¹-S(O)-R⁹ or -Alk¹-S(O)₂-R⁹, wherein Alk¹ is C₁₋₆alkanediyl,
- R⁹: is hydroxy, C₁₋₆alkyl, C₁₋₆alkyloxy, amino, C₁₋₈alkylamino or C₁₋₈alkylamino substituted with C₁₋₆alkyloxycarbonyl;
- R², R³ and R¹⁶: each independently are hydrogen, hydroxy, halo, cyano, C₁₋₆alkyl, C₁₋₆alkyloxy, hydroxyC₁₋₆alkyloxy, C₁₋₆alkyloxyC₁₋₆alkyloxy, aminoC₁₋₆alkyloxy, mono- or di(C₁₋₆alkyl)aminoC₁₋₆alkyloxy, Ar¹, Ar²C₁₋₆alkyl, Ar²oxy, Ar²C₁₋₆alkyloxy, hydroxycarbonyl, C₁₋₆alkyloxycarbonyl, trihalomethyl, trihalomethoxy, C₂₋₆alkenyl, 4,4-dimethyloxazolyl; or
when on adjacent positions R² and R³ taken together may form a bivalent radical of formula

-O-CH₂-O- (a-1),

-O-CH₂-CH₂-O- (a-2),

-O-CH=CH- (a-3),

-O-CH₂-CH₂- (a-4),

-O-CH₂-CH₂-CH₂- (a-5),

or

-CH=CH-CH=CH- (a-6);
- R⁴ and R⁵: each independently are hydrogen, halo, Ar¹, C₁₋₆alkyl, hydroxyC₁₋₅alkyl, C₁₋₆alkyloxyC₁₋₆alkyl, C₁₋₆alkyloxy, C₁₋₆alkylthio, amino, hydroxycarbonyl, C₁₋₆alkyloxycarbonyl, C₁₋₆alkylS(O)C₁₋₆alkyl or C₁₋₆alkylS(O)₂C₁₋₆alkyl;
- R⁶ and R⁷: each independently are hydrogen, halo, cyano, C₁₋₆alkyl, C₁₋₆alkyloxy, Ar²oxy, trihalomethyl, C₁₋₆alkylthio, di(C₁₋₆alkyl)amino, or when on adjacent positions R⁶ and R⁷ taken together may form a bivalent radical of formula

-O-CH₂-O- (c-1),

or

-CH=CH-CH=CH- (c-2);
- R⁸: is hydrogen, C₁₋₆alkyl, cyano, hydroxycarbonyl, C₁₋₆alkyloxycarbonyl, C₁₋₆alkylcarbonylC₁₋₆alkyl, cyanoC₁₋₆alkyl, C₁₋₆alkyloxycarbonylC₁₋₆alkyl, carboxyC₁₋₆alkyl, hydroxyC₁₋₆alkyl, aminoC₁₋₆alkyl, mono- or di(C₁₋₆alkyl)aminoC₁₋₆alkyl, imidazolyl, haloC₁₋₆alkyl, C₁₋₆alkyloxyC₁₋₆alkyl, aminocarbonylC₁₋₆alkyl, or a radical of formula

-O-R¹⁰ (b-1),

-S-R¹⁰ (b-2),

-N-R¹¹R¹² (b-3),

wherein R¹⁰ is hydrogen, C₁₋₆alkyl, C₁₋₆alkylcarbonyl, Ar¹, Ar²C₁₋₆alkyl, C₁₋₆alkyloxycarbonylC₁₋₆alkyl, or a radical of formula -Alk²-OR¹³ or -Alk²-NR¹⁴R¹⁵;
- R¹¹: is hydrogen, C₁₋₁₂alkyl, Ar¹ or Ar²C₁₋₆alkyl;
- R¹²: is hydrogen, C₁₋₆alkyl, C₁₋₁₆alkylcarbonyl, C₁₋₆alkyloxycarbonyl, C₁₋₆alkylaminocarbonyl, Ar¹, Ar²C₁₋₆alkyl, C₁₋₆alkylcarbonylC₁₋₆alkyl, a natural amino acid, Ar¹carbonyl, Ar²C₁₋₆alkylcarbonyl, aminocarbonylcarbonyl, C₁₋₆alkyloxyC₁₆alkylcarbonyl, hydroxy, C₁₋₆alkyloxy, aminocarbonyl, di(C₁₋₆alkyl)aminoC₁₋₆alkylcarbonyl, amino, C₁₋₆alkylamino, C₁₋₆alkylcarbonylamino, or a radical of formula -Alk²-OR1³ or -Alk²-NR¹⁴R¹⁵; wherein Alk² is C₁₋₆alkanediyl;
- R¹³: is hydrogen, C₁₋₆alkyl, C₁₋₆alkylcarbonyl, hydroxyC₁₋₆alkyl, Ar¹ or Ar²C₁₋₆alkyl;
- R¹⁴: is hydrogen, C₁₋₆alkyl, Ar¹ or Ar²C₁₋₆alkyl;
- R¹⁵: is hydrogen, C₁₋₆alkyl, C₁₋₆alkylcarbonyl, Ar¹ or Ar²C₁₋₆alkyl;
- R¹⁷: is hydrogen, halo, cyano, C₁₋₆alkyl, C₁₋₆alkyloxycarbonyl, Ar¹;
- R¹⁸: is hydrogen, C₁₋₆alkyl, C₁₋₆alkyloxy or halo;
- R¹⁹: is hydrogen or C₁₋₆alkyl;
- Ar¹: is phenyl or phenyl substituted with C₁₋₆alkyl, hydroxy, amino, C₁₋₆alkyloxy or halo; and
- Ar²: is phenyl or phenyl substituted with C₁₋₆alkyl, hydroxy, amino, C₁₋₆alkyloxy or halo.

In Formulas (I), (II) and (III), R⁴ or R⁵ may also be bound to one of the nitrogen atoms in the imidazole ring. In that case the hydrogen on the nitrogen is replaced by R⁴ or R⁵ and the meaning of R⁴ and R⁵ when bound to the nitrogen is limited to hydrogen, Ar¹, C₁₋₆alkyl, hydroxyC₁₋₆alkyl, C₁₋₆alkyloxyC₁₋₆alkyl, C₁₆alkyloxycarbonyl, C₁₋₆alkylS(O)C₁₋₆alkyl, C₁₋₆alkylS(O)₂C₁₋₆alkyl.

Preferably the substituent R¹⁸ is situated on the 5 or 7 position of the quinolinone moiety and substituent R¹⁹ is situated on the 8 position when R¹⁸ is on the 7-position.

Preferred examples of FTIs are those compounds of formula (I) wherein X is oxygen.

Also, examples of useful FPTase-inhibiting compounds are these compounds of formula (I) wherein the dotted line represents a bond, so as to form a double bond.

Another group of illustrative compounds are those compounds of formula (I) wherein R¹ is hydrogen, C₁₋₆alkyl, C₁₋₆alkyloxyC₁₋₆alkyl, di(C₁₋₆alkyl)aminoC₁₋₆alkyl, or a radical of formula -Alk¹-C(=O)-R⁹, wherein Alk¹ is methylene and R⁹ is C₁₋₈alkylamino substituted with C₁₋₆alkyloxycarbonyl.

Still another group of exemplary compounds are those compounds of formula (I) wherein R³ is hydrogen or halo; and R² is halo, C₁₋₆alkyl, C₂₋₆alkenyl, C₁₋₆alkyloxy, trihalomethoxy or hydroxyC₁₋₆alkyloxy.

A further group of illustrative compounds are those compounds of formula (I) wherein R² and R³ are on adjacent positions and taken together to form a bivalent radical of formula (a-1), (a-2) or (a-3).

A still further group of exemplary compounds are those compounds of formula (I) wherein R⁵ is hydrogen and R⁴ is hydrogen or C₁₋₆alkyl.

Yet another group of illustrative compounds are those compounds of formula (I) wherein R⁷ is hydrogen; and R⁶ is C₁₋₆alkyl or halo, preferably chloro, especially 4-chloro.

Another exemplary group of compounds are those compounds of formula (I) wherein R⁸ is hydrogen, hydroxy, haloC₁₋₆alkyl, hydroxyC₁₋₆alkyl, cyanoC₁₋₆alkyl, C₁₋₆(alkyloxycarbonylC₁₋₆alkyl, imidazolyl, or a radical of formula -NR¹¹R¹² wherein R¹¹ is hydrogen or C₁₋₁₂alkyl and R¹² is hydrogen, C₁₋₆alkyl, C₁₋₆alkyloxy, hydroxy, C₁₋₆alkyloxyC₁₋₆alkylcarbonyl, or a radical of formula -Alk²-OR¹³ wherein R¹³ is hydrogen or C₁₋₆alkyl.

Preferred compounds are those compounds wherein R¹ is hydrogen, C₁₋₆alkyl, C₁₋₆alkyloxyC₁₋₆alkyl, di(C₁₋₆alkyl)aminoC₁₋₆alkyl, or a radical of formula -Alk¹-C(=O)-R⁹, wherein Alk¹ is methylene and R⁹ is C₁₋₈alkylamino substituted with C₁₋₆alkyloxycarbonyl; R² is halo, C₁₋₆alkyl, C₂₋₆alkenyl, C₁₋₆alkyloxy, trihalomethoxy, hydroxyC₁₋₆alkyloxy or Ar¹; R³ is hydrogen; R⁴ is methyl bound to the nitrogen in 3-position of the imidazole; R⁵ is hydrogen; R⁶ is chloro; R⁷ is hydrogen; R⁸ is hydrogen, hydroxy, haloC₁₋₆alkyl, hydroxyC₁₋₆alkyl, cyanoC₁₋₆alkyl, C₁₋₆alkyloxycarbonylC₁₋₆alkyl, imidazolyl, or a radical of formula - NR¹¹R¹² wherein R¹¹ is hydrogen or C₁₋₁₂alkyl and R¹² is hydrogen, C₁₋₆alkyl, C₁₋₆alkyloxy, C₁₋₆alkyloxyC₁₋₆alkylcarbonyl, or a radical of formula -Alk²-OR¹³ wherein R¹³ is C₁₋₆alkyl; R¹⁷ is hydrogen and R¹⁸ is hydrogen.

Especially preferred compounds are:
4-((3-chlorophenyl)-6-[(4-chlorophenyl)hydroxy(1-methyl-1*H*-imidazol-5-yl)methyl]-1-methyl-2(1*H*)-quinolinone;
6-[amino(4-chlorophenyl)-1-methyl-1*H*-imidazol-5-ylmethyl]-4-(3-chlorophenyl)-1-methyl-2(1*H*)-quinolinone;
6-[((4-chlorophenyl)hydroxy(1-methyl-1*H*-imidazol-5-yl)methyl]-4-(3-ethoxyphenyl)-1-methyl-2(1H)-quinolinone;
6-[((4-chlorophenyl)(1-methyl-1*H*-imidazol-5-yl)methyl]-4-(3-ethoxyphenyl)-1-methyl-2(1*H*)-quinolinone monohydrochloride.monohydrate;
6-[amino(4-chlorophenyl)(1-methyl-1*H*-imidazol-5-yl)methyl]-4-(3-ethoxyphenyl)-1-methyl-2(1*H*)-quinolinone;
6-amino(4-chlorophenyl)(1-methyl-1*H*-imidazol-5-yl)methyl]-1-methyl-4-(3-propylphenyl)-2(1*H*)-quinolinone; a stereoisomeric form thereof or a pharmaceutically acceptable acid or base addition salt; and
(+)-6-[amino(4-chlorophenyl)(1-methyl-1*H*-imidazol-5-yl)methyl]-4-(3-chlorophenyl)-1-methyl-2(1*H*)-quinolinone (tipifarnib; Compound 75 in Table 1 of WO 97/21701); or a pharmaceutically acceptable acid addition salt thereof.

Tipifarnib or ZARNESTRA^{®} is an especially preferred FTI.

Further preferred embodiments of the present invention include compounds of formula (IX) wherein one or more of the following apply:
- =X¹-X²-X³ is a trivalent radical of formula (x-1), (x-2), (x-3), (x-4) or (x-9) wherein each R⁶ independently is hydrogen, C₁₋₄alkyl, C₁₋₄alkyloxycarbonyl, amino or aryl and R⁷ is hydrogen;
- >Y¹-Y²- is a trivalent radical of formula (y-1), (y-2), (y-3), or (y-4) wherein each R⁹ independently is hydrogen, halo, carboxyl, C₁₋₄alkyl or C₁₋₄alkyloxycarbonyl;
- r is 0, 1 or 2;
- s is 0 or 1;
- t is 0;
- R¹ is halo, C₁₋₆alkyl or two R¹ substituents ortho to one another on the phenyl ring may independently form together a bivalent radical of formula (a-1);
- R² is halo;
- R³ is halo or a radical of formula (b-1) or (b-3) wherein
   R¹⁰ is hydrogen or a radical of formula -Alk-OR¹³.
   R¹¹ is hydrogen;
- R¹² is hydrogen, C₁₋₆alkyl, C₁₋₆alkylcarbonyl, hydroxy, C₁₋₆alkyloxy or mono- or di(C₁₋₆alkyl)aminoC₁₋₆alkylcarbonyl; 36
   Alk is C₁₋₆alkanediyl and R¹³ is hydrogen;
- R⁴ is a radical of formula (c-1) or (c-2) wherein
   R¹⁶ is hydrogen, halo or mono- or di(C₁₋₄alkyl)amino;
   R¹⁷ is hydrogen or C₁₋₆alkyl;
- aryl is phenyl.

An exemplary group of compounds consists of those compounds of formula (IX) wherein =X¹-X²-X³ is a trivalent radical of formula (x-1), (x-2), (x-3), (x-4) or (x-9), >Yl-Y2 is a trivalent radical of formula (y-2), (y-3) or (y-4), r is 0 or 1, s is 1, t is 0, R¹ is halo, C₍₁₋₄₎alkyl or forms a bivalent radical of formula (a-1), R² is halo or C₁₋₄alkyl, R³ is hydrogen or a radical of formula (b-1) or (b-3), R⁴ is a radical of formula (c-1) or (c-2), R⁶ is hydrogen, C₁₋₄alkyl or phenyl, R⁷ is hydrogen, R⁹ is hydrogen or C₁₄alkyl, R¹⁰ is hydrogen or -Alk-OR¹³, R¹¹ is hydrogen and R¹² is hydrogen or C₁₆alkylcarbonyl and R¹³ is hydrogen;

Preferred compounds are those compounds of formula (IX) wherein =X¹-X²-X³ is a trivalent radical of formula (x-1) or (x-4), >Y1-Y2 is a trivalent radical of formula (y-4), r is 0 or 1, s is 1, t is 0, R₁ is halo, preferably chloro and most preferably 3-chloro, R² is halo, preferably 4-chloro or 4-fluoro, R³ is hydrogen or a radical of formula (b-1) or (b-3), R⁴ is a radical of formula (c-1) or (c-2), R⁶ is hydrogen, R⁷ is hydrogen, R⁹ is hydrogen, R¹⁰ is hydrogen, R¹¹ is hydrogen and R¹² is hydrogen.

Other preferred compounds are those compounds of formula (IX) wherein =X¹-X²-X³ is a trivalent radical of formula (x-2), (x-3) or (x-4), >Y1-Y2 is a trivalent radical of formula (y-2), (y-3) or (y-4), r and s are 1, t is 0, R¹ is halo, preferably chloro, and most preferably 3-chloro or R¹ is C₁₋₄alkyl, preferably 3-methyl, R² is halo, preferably chloro, and most preferably 4-chloro, R³ is a radical of formula (b-1) or (b-3), R⁴ is a radical of formula (c-2), R⁶ is C₁₋₄alkyl, R⁹ is hydrogen, R¹⁰ and R¹¹ are hydrogen and R¹² is hydrogen or hydroxy.

Especially preferred compounds of formula (IX) are:
7-[((4-fluorophenyl)(1*H*-imidazol-1-yl)methyl]-5-phenylimidazo[1,2-a]quinoline;
α-(4-chlorophenyl)-α-(1-methyl-1*H*-imidazol-5-yl)-5-phenylimidazo[1,2-a]quinoline-7-methanol;
5-((3-chlorophenyl)-α-(4-chlorophenyl)-α-(1-methyl-1*H*-imidazol-5-yl)-imidazo[1,2-a]quinoline-7-methanol;
5-((3-chlorophenyl)-α-(4-chlorophenyl)-α-(1-methyl-1*H*-imidazol-5-yl)imidazo[1,2-a]quinoline-7-methanamine;
5-((3-chlorophenyl)-α-(4-chlorophenyl)-α-(1-methyl-1*H*-imidazol-5-yl)tetrazolo[1,5-a]quinoline-7-methanamine;
5-((3-chlorophenyl)-α-(4-chlorophenyl)-1-methyl-α-(1-methyl-1H-imidazol-5-yl)-1,2,4-triazolo[4,3-a]quinoline-7-methanol;
5-((3-chlorophenyl)-α-(4-chlorophenyl)-α-(1-methyl-1H-imidazol-5-yl)tetrazolo[1,5-a]quinoline-7-methanamine;
5-((3-chlorophenyl)-α-(4-chlorophenyl)-α-(-methyl-1*H*-imidazol-5-yl)tetrazolo[1,5-a]quinazoline-7-methanol;
5-((3-chlorophenyl)-α-(4-chlorophenyl)-4,5-dihydro-α-(1-methyl-1*H*-imidazol-5-yl)tetrazolo[1,5-a]quinazoline-7-methanol;
5-((3-chlorophenyl)-α-(4-chlorophenyl)-α-(1-methyl-1*H*-imidazol-5-yl)tetrazolo[1,5-a]quinazoline-7-methanamine;
5-((3-chlorophenyl)-α-(4-chlorophenyl)-N-hydroxy-α-(1-methyl-1*H*-imidazol-5-yl)tetrahydro[1,5-a]quinoline-7-methanamine;
α-(4-chlorophenyl)-α-(1-methyl-1*H*-imidazol-5-yl)-5-(3-methylphenyl)tetrazolo[1,5-a]quinoline-7-methanamine; the pharmaceutically acceptable acid addition salts and the stereochemically isomeric forms thereof.

5-(3-chlorophenyl)-α-(4-chlorophenyl)-α-(1-methyl-1*H*-imidazol-5-yl)tetrazolo[1,5-a]quinazoline-7-methanamine, especially the (-) enantiomer, and its pharmaceutically acceptable acid addition salts are especially preferred.

As used in the foregoing definitions and hereinafter "halo" encompasses fluoro, chloro, bromo and iodo; "C₁₋₆alkyl" encompasses straight and branched chained saturated hydrocarbon radicals having from 1 to 6 carbon atoms such as, for example, methyl, ethyl, propyl, butyl, pentyl, hexyl and the like; "C₁₋₈alkyl" encompasses the straight and branched chained saturated hydrocarbon radicals as defined for C₁₋₆alkyl as well as the higher homologues thereof containing 7 or 8 carbon atoms, such as heptyl or octyl; "C₁₋₁₂alkyl" likewise encompasses C₁₋₈alkyl and the higher homologues thereof containing 9 to 12 carbon atoms, such as nonyl, decyl, undecyl, dodecyl; "C₁₁₆alkyl," similarly encompasses C₁₋₁₂alkyl and the higher homologues thereof containing 13 to 16 carbon atoms, such as tridecyl, tetradecyl, pentedecyl and hexadecyl;."C₂_₆alkenyl" encompasses straight and branched chain hydrocarbon radicals containing one double bond and having from 2 to 6 carbon atoms, such as ethenyl, 2-propenyl, 3-butenyl, 2-pentenyl, 3-pentenyl, 3-methyl-2-butenyl, and the like; and "C₁₋₆alkanediyl" encompasses bivalent straight and branched chained saturated hydrocarbon radicals having from 1 to 6 carbon atoms, such as methylene, 1,2-ethanediyl, 1,3-propanediyl, 1,4-butanediyl, 1,5-pentanediyl, 1,6-hexanediyl and the branched isomers thereof. The term "C(=O)" refers to a carbonyl group, "S(O)" refers to a sulfoxide, and "S(O)₂" refers to a sulfon. The term "natural amino acid" refers to a natural amino acid that is bound via a covalent amide linkage formed by loss of a molecule of water between the carboxyl group of the amino acid and the amino group of the remainder of the molecule (examples of natural amino acids are glycine, alanine, valine, leucine, isoleucine, methionine, proline, phenylanaline, tryptophan, serine, threonine, cysteine, tyrosine, asparagine, glutamine, aspartic acid, glutamic acid, lysine, arginine, and histidine).

The pharmaceutically acceptable acid or base addition salts as mentioned hereinabove are meant to comprise the therapeutically active non-toxic acid and non-toxic base addition salt forms which the compounds of formulas (I), (II), (III), (IV), (V), (VI), (VII), (VIII) or (IX) are able to form. The compounds of formulas (I), (II), (III), (IV), (V), (VI), (VII), (VIII) or (IX) which have basic properties can be converted in their pharmaceutically acceptable acid addition salts by treating the base form with an appropriate acid. Appropriate acids include, for example, inorganic acids such as hydrohalic acids, e.g. hydrochloric or hydrobromic acid; sulfuric; nitric; phosphoric and the like acids; or organic acids, such as acetic, propanoic, hydroxyacetic, lactic, pyruvic, oxalic, malonic, succinic (*i.e*. butanedioic acid), maleic, fumaric, malic, tartaric, citric, methanesulfonic, ethanesulfonic, benzenesulfonic, p-toluenesulfonic, cyclamic, salicylic, p-aminosalicylic, pamoic and the like acids.

The compounds of formulae (I), (II), (III), (IV), (V), (VI), (VII), (VIII) or (IX) which have acidic properties may be converted in their pharmaceutically acceptable base addition salts by treating the acid form with a suitable organic or inorganic base. Appropriate base salt forms comprise, for example, the ammonium salts, the alkali and earth alkaline metal salts, e.g. the lithium, sodium, potassium, magnesium, calcium salts and the like, salts with organic bases, e.g. the benzathine, N-methyl-D-glucamine, hydrabamine salts, and salts with amino acids, for example, arginine, lysine and the like.

Acid and base addition salts also comprise the hydrates and the solvent addition forms which the compounds of formulae (I), (II), (III), (IV), (V), (VI), (VII), (VIII) or (IX) are able to form. Examples of such forms are e.g. hydrates, alcoholates and the like.

The compounds of formulae (I), (II), (III), (IV), (V), (VI), (VII), (VIII) or (IX), as used hereinbefore, encompass all stereochemically isomeric forms of the depicted structural formulae (all possible compounds made up of the same atoms bonded by the same sequence of bonds but having different three-dimensional structures that are not interchangeable). Unless otherwise mentioned or indicated, the chemical designation of a compound should be understood as encompassing the mixture of all possible stereochemically isomeric forms which the compound may possess. Such mixture may contain all diastereomers and/or enantiomers of the basic molecular structure of the compound. All stereochemically isomeric forms of the compounds of formulae (I), (II), (III), (IV), (V), (VI), (VII), (VIII) or (IX) both in pure form or in admixture with each other are intended to be embraced within the scope of the depicted formulae.

Some of the compounds of formulae (I), (II), (III), (IV), (V), (VI), (VII), (VIII) or (IX) may also exist in their tautomeric forms. Such forms, although not explicitly shown in the above formulae, are intended to be included within the scope thereof.

Thus, unless indicated otherwise hereinafter, the term "compounds of formulae (I), (II), (III), (IV), (V), (VI), (VII), (VIII) or (IX)" is meant to include also the pharmaceutically acceptable acid or base addition salts and all stereoisomeric and tautomeric forms.

Other farnesyltransferase inhibitors which can be employed in accordance with the present include Arglabin, perrilyl alcohol, SCH-66336, 2(S)-[2(S)-[2(R)-amino-3-mercapto]propylamino-3(S)-methyl]-pentyloxy-3-phenylpropionyl-methionine sulfone (Merck); L778123, BMS 214662, Pfizer compounds A and B described above. Suitable dosages or therapeutically effective amounts for the compounds Arglabin (WO98/28303), perrilyl alcohol (WO 99/45712), SCH-66336 (US 5,874,442), L778123 (WO 00/01691), 2(S)-[2(S)-[2(R)-amino-3-mercapto]propylamino-3(S)-methyl]-pentyloxy-3-phenylpropionyl-methionine sulfone (WO94/10138), BMS 214662 (WO 97/30992), Pfizer compounds A and B (WO 00/12499 and WO 00/12498) are given in the published patent specifications or are known to or can be readily determined by a person skilled in the art.

A variety of farnesyltransferase inhibitors can be prepared and formulated into pharmaceutical compositions by methods described in the art, such as the publications cited herein. For example, for the compounds of formulae (I), (II) and (III) suitable examples can be found in WO-97/21701. Compounds of formulae (IV), (V), and (VI) can be prepared and formulated using methods described in WO 97/16443, compounds of formulae (VII) and (VIII) according to methods described in WO 98/40383 and WO 98/49157 and compounds of formula (IX) according to methods described in WO 00/39082 respectively. Tipifarnib (R115777) and its less active enantiomer can be synthesized by methods described in WO 97121701.

To prepare the aforementioned pharmaceutical compositions, a therapeutically effective amount of the particular compound, optionally in addition salt form, as the active ingredient is combined or admixed with a pharmaceutically acceptable carrier, vehicle, or diluent, which may take a wide variety of forms depending on the form of preparation desired for administration. These pharmaceutical compositions are desirably in unitary dosage form suitable, preferably, for systemic administration such as oral, percutaneous, or parenteral administration; or topical administration, such as via inhalation, a nose spray, eye drops or via a cream, gel, shampoo or the like. For example, in preparing the compositions in oral dosage form, any of the usual pharmaceutical media may be employed, such as, for example, water, glycols, oils, alcohols and the like in the case of oral liquid preparations such as suspensions, syrups, elixirs and solutions; or solid carriers such as starches, sugars, kaolin, lubricants, binders, disintegrating agents and the like in the case of powders, pills, capsules and tablets.

Because of their ease in administration, tablets and capsules represent a preferred oral dosage unit form, in which case solid pharmaceutical carriers are obviously employed. For parenteral compositions, the carrier will usually comprise sterile water, at least in large part, though other ingredients, for example, to aid solubility, may be included. Injectable solutions, for example, may be prepared in which the carrier comprises saline solution, glucose solution or a mixture of saline and glucose solution. Injectable solutions containing compounds of formula (I) may be formulated in an oil for prolonged action. Appropriate oils for this purpose are, for example, peanut oil, sesame oil, cottonseed oil, corn oil, soy bean oil, synthetic glycerol esters of long chain fatty acids and mixtures of these and other oils.

Injectable suspensions may also be prepared in which case appropriate liquid carriers, suspending agents and the like may be employed. In the compositions suitable for percutaneous administration, the carrier optionally comprises a penetration enhancing agent and/or a suitable wettable agent, optionally combined with suitable additives of any nature in minor proportions, which additives do not cause any significant deleterious effects on the skin. Said additives may facilitate the administration to the skin and/or may be helpful for preparing the desired compositions. These compositions may be administered in various ways, e.g., as a transdermal patch, as a spot-on or as an ointment. As appropriate, compositions for topical application may include all compositions usually employed for topically administering drugs e.g. creams, gellies, dressings, shampoos, tinctures, pastes, ointments, salves, powders and the like. Application of said compositions may be by aerosol, e.g. with a propellent such as nitrogen, carbon dioxide, a freon, or without a propellent such as a pump spray, drops, lotions, or a semisolid such as a thickened composition which can be applied by a swab. In particular, semisolid compositions such as salves, creams, gellies, ointments and the like will conveniently be used.

It is especially advantageous to formulate the aforementioned pharmaceutical compositions in dosage unit form for ease of administration and uniformity of dosage. Dosage unit or unitary form refers to physically discrete units suitable as unitary dosages, each unit containing a predetermined quantity of active ingredient calculated to produce the desired therapeutic effect in association with the required pharmaceutical carrier. Examples of such dosage unit forms are tablets (including scored or coated tablets), capsules, pills, powder packets, wafers, injectable solutions or suspensions, teaspoonfuls, tablespoonfuls and the like, and segregated multiples thereof.

A farnesyltransferase inhibiting compound or a composition containing such a compound is used in a therapeutic method of the invention to treat a subject diagnosed with or suffering from a disorder or condition mediated through farnesyltransferase inhibition. Treatments according to the invention comprise administering to a subject an effective amount of an FTI or composition containing an FTI to treat the disorder or condition. The term "treat" or "treating" as used herein is intended to refer to administration of an compound or composition to a subject in need of treatment for the purpose of effecting a desired therapeutic or prophylactic benefit through inhibition of farnesyltransferase activity. Treating includes reversing, ameliorating, alleviating, inhibiting the progress of, lessening the severity of, or preventing a disorder or condition, or one or more symptoms of such disorder or condition. The term "subject" refers to a mammalian patient, such as a human.

In a treatment method according to the invention, an effective amount of a TFI compound or composition is administered to a patient suffering from or diagnosed as having the specified disorder or condition. An "effective amount" means an amount or dose generally sufficient to bring about the desired therapeutic or prophylactic benefit in subjects undergoing treatment.

Effective amounts or doses of the agents of the present invention may be ascertained by routine methods such as modeling, dose escalation studies or clinical trials, and by taking into consideration routine factors, e.g., the mode or route of administration or drug delivery, the pharmacokinetics of the agent, the severity and course of the disorder or condition, the subject's previous or ongoing therapy, the subject's health status and response to drugs, and the judgment of the treating physician. An exemplary dose is in the range of from about 0.001 to about 200 mg per kg of subject's body weight per day, preferably about 0.05 to 100 mg/kg/day, or about 1 to 35 mg/kg/day, in single or divided dosage units (e.g., BID, TID, QID). For a 70-kg human, an illustrative dosage amount is from about 0.05 to about 7 g/day, or about 0.2 to about 2.5 g/day.

To better understand and illustrate the invention and its exemplary embodiments and advantages, reference is made to the following experimental section.

### EXPERIMENTALS

In the following studies the effects, *in vitro* and *in vivo,* of a statin (simvastatin) are compared to those of the FTI, tipifarnib (R115777, Zarnestra™), a potent, orally active inhibitor. The results confirm that some, but not all, of the anti-inflammatory effects of statins are mediated through inhibition of protein farnesylation. Tipifarnib clearly showed global inhibition of LPS-induced inflammation *in vitro* and *in vivo.*

### MATERIAL AND METHODS

### Materials

Reagent chemicals were purchased from Sigma-Aldrich (St. Louis, MO). Simvastatin (active form) was purchased from Calbiochem (La Jolla, CA). Tipifarnib (R115777) and its less active enantiomer were obtained from Johnson & Johnson Pharmaceutical Research & Development, LLC. Human THP-1 cells were obtained from the American Type Culture Collection (Manassas, VA). RPMI-1640 cell culture medium and Penicillin-Streptomycin were purchased from Sigma-Aldrich, and fetal bovine serum (FBS) from Gibco/Invitrogen (Carlsbad, CA). LPS was from Sigma-Aldrich.

### Cell Culture

THP-1 cells were cultured in RPMI1640 medium, supplemented with 10% FBS/100 units/ml Penicillin/10 µg/ml Streptomycin at 37°C, under 5% CO₂. The components in the medium were all tested for endotoxin contamination and shown to contain less than 0.3 endotoxin units/ml. For RNA isolation, THP-1 cells were harvested and washed twice with PBS, then resuspended at 4.5x10⁵/ml in 20ml RPMI 1640 medium containing 0.5%FBS/ 100ng/ml LPS and simvastatin (10 µM), or tipifarnib (5 µM). For the untreated control group (no LPS, no compound) and LPS alone group, equivalent volumes of DMSO were added to the medium. Cells were cultured at 37°C and pellets were collected at Ohr, 1hr, 3hr, 6hr, 12hr & 24hr. Cell pellets were stored at -80°C if RNA isolation was not conducted immediately. For experiments to study cytokine secretion in culture supernatants, THP-1 cells were washed twice with PBS, resuspended at 3.4 x10⁵/ml and cultured in 96-well plates at 250 µL per well at 37°C. Cells were cultured in medium containing 0.5%FBS/RPMI 1640, in the absence or presence of 100ng/ml LPS and simvastatin (5 µM and 10 µM), or tipifarnib (2 µM and 5 µM). Supernatants were collected at different time points as indicated in the figures and were kept at ≤ -20°C until analysis of cytokine production.

### Isolation of RNA

Total RNA was isolated using RNeasy mini kit from QIAGEN (Valencia, CA) according to the manufacturer's instructions. RNA samples were stored at -80°C.

### Microarray experiments and data analysis

Five µg of total RNA was linearly amplified to double-stranded cDNA using T7-based amplification. The cDNA was purified using the QIAquick 96 PCR purification kit (QIAGEN Valencia, CA), and used to generate amplified (a)RNA using AmpliScribe™ T7 High Yield Transcription Kit (Epicentre, Madison, WI). Ten µg of purified aRNA was reverse transcribed to cDNA using the SuperScript RTII kit (Invitrogen) and labeled by direct incorporation of Cy3-dCTP. The generated cDNA probe was used for hybridizing to cDNA microarrays.

The human cDNA microarrays were custom made as described previously (Peterson KS 2004), and contained approximately 8,000 human genes and ESTs. Each clone was spotted in duplicate on the array. RNA preparation, cDNA probe synthesis and hybridization were performed as described by Shaw *et al.* (Shaw KJ 2003).

Data normalization and preparation was performed as previously described (Peterson KS 2004).

Genes were selected as LPS-regulated if the ratios between LPS-treated and non-treated samples were greater than 1.5-fold at more than two time points. Genes that were further regulated by the statin or FTI were selected if their expression was at least 40% lower, at one or more time points, for treatment by LPS and statin, or LPS and FTI, versus LPS alone. After removing the redundancy, there were 22 genes selected as LPS-induced genes that were inhibited by statin or FTI treatment.

### Reverse Transciptase and Real-time quantitative PCR

Two to three micrograms of DNase-treated total RNA were reverse transcribed to cDNA using the Transcriptor First Strand cDNA Synthesis Kit (Roche Diagnostics Corporation, Indianapolis, IN) according to the instructions.

Real-time PCR was carried out with the LightCycler FastStart DNA Master^{plus} SYBR Green I kit (Roche Diagnostics) according to the manufacturer's instructions. The 20µL final volume contained: 4 mM MgCl₂, 0.5µM of each primer, 2 µL Master mix and 2 µL of cDNA.

The PCR profile was as follows: (i) denaturation at 95°C for 10 min. (ii) 45 cycles of 0 s at 95 °C, 5 s at 54-58°C (depending on Tm of the primers), 6-16 s (determined by the length of amplicon) at 72 °C. (iii) melting curves for 0 s at 95°C, 15s at 65°C and 0 s at 95 °C. (iv) cooling at 40°C for 30 s. Transition rates were: 20 °C/s for all steps except 0.1 °C/s for 95 °C segment 3 of the melting curves.

For data analysis, the baseline adjustment was carried out in the "arithmetic" mode, and the fluorescence curve analysis was carried out in the "Second Derivative Maximum" mode of the LightCycler™ software (Version 3.5).

The primers used for real-time PCR were as follows (5' to 3'):

| | |
|---|---|
| IL-1β | sense,GGATAACGAGGCTTATGTGCACG (SEQ ID NO:1); |
| | antisense, GGACATGGAGAACACCACTTGTTG (SEQ ID NO:2). |
| MCP-1 | sense, AGCCAGATGCAATCAATGCCC (SEQ ID NO:3); |
| | antisense, CCTTGGCCACAATGGTCTTGAA (SEQ ID NO:4). |
| MMP-9 | sense, ACCGCTATGGTTACACTCGG (SEQ ID NO:5); |
| | antisense, AGGGACCACAACTCGTCATC (SEQ ID NO:6). |
| IL-8 | sense, CTGATTTCTGCAGCTCTGTGTGAA (SEQ ID NO:7); |
| | antisense, TGGCATCTTCACTGATTCTTGGA (SEQ ID NO:8). |
| STAT1 | sense, TTCAGAGCTCGTTTGTGGTG (SEQ ID NO:9); |
| | antisense, AGAGGTCGTCTCGAGGTCAA (SEQ ID NO:10). |
| MyD88 | sense, TGGGACCCAGCATTGAGGAGGATT (SEQ ID NO:11); |
| | antisense, AAACTGGATGTCGCTGGGGCAA (SEQ ID NO:12). |
| IL6 | sense, TGGATTCAATGAGGAGACTTGC (SEQ ID NO:13); |
| | antisense, CAGGAACTGGATCAGGACTT (SEQ ID NO:14). |

### In vivo experiments

The experiments were performed on female BALB/c mice (6-7 week old; Charles River Laboratories, Inc. MA). Simvastatin and tipifarnib were dissolved in 20% cyclodextran at a concentration of 10 mg/ml and orally administered to mice at a dose level of 50mg/kg at 24 hours, 17 hours and 1 hour before LPS injection. The control mice received the vehicle (20% cyclodextran) only. 20 µg LPS (Sigma), diluted in PBS, was injected into each mouse intraperitoneally. Blood samples were collected either by eye bleeding or cardiac puncture at 1 and 2 hours after LPS injection in one experiment, and at 2 and 3 hours after LPS injection in a second experiment. Plasma samples were collected by centrifugation, and stored at ≤ -20°C before being tested for cytokines using individual ELISA kits or Luminex analysis (see below).

### Cytokine assay by ELISA

Human IL-6, IL8, IL1β, MCP-1, MMP-9, and mouse IL-6, IL8, TNFα, MCP-1 and IL-1β were measured with ELISA kits (R&D Systems) according to the manufacturer's instructions.

### Cytokine assay by Luminex

Samples were tested for cytokines using the Bio-plex cytokine assay kit (Bio-Rad Laboratories, Inc.) according to the manufacturer's instructions. Briefly, 50 µL anti-cytokine antibody conjugated beads were added to wells of a filter plate pre-equilibrated with assay buffer. The beads were washed, then 50 µL of undiluted cell culture supernatant or 4-fold diluted plasma samples were added and incubated for 2 hours. Biotinylated anti-cytokine antibody (25 µL) was added to each well and the plate was incubated with shaking for 1.5 hours. Streptavidin-PE (50 µL) was added to each well and the plate was incubated with shaking for 45 min. between incubations, wells were washed 3 times with 100 µL wash buffer and unbound analytes were filtered using a vacuum manifold. Following the last wash step, 125 µL of assay buffer was added to each well and the plate was placed on a shaker for 10 min. The plate was read on a Luminex¹⁰⁰ instrument (Luminex Corporation, Austin, TX) according to the manufacturer's instructions. The concentration of cytokine was determined from a standard curve assayed at the same time.

### Isolation of peripheral blood mononuclear cells (PBMC)

Heparinized human blood (25 ml) from a healthy donor was overlaid into a 50 ml conical tubes containing 20 ml Ficoll-Paque, and centrifuged for 20 min at 2000 rpm at room temperature and then allowed to stop without using the brake on the centrifuge. The upper layer was aspirated, leaving the mononuclear cell layer undisturbed at the interface. Cells were carefully transferrred the to a new 250 ml conical tube. The conical tube was filled with PBS, mixed and centrifuged at 1500rpm for 15 minutes at room temperature. The supernatant was carefully and completely removed. The cell pellet was resupended in 50 ml of PBS and centrifuged at 1500 rpm for 5 minutes. This wash step was repeated once more. The cells were counted and resuspended cells at 1.7x10⁶/ml in low-endotoxin RPMI1640/10%FBS.

### LPS/Tipifarnib treatment of human PBMC

Human PBMC (180 µl at 1.7x10⁶/ml) were pipetted into each well of a 96-well plate. 10ul of tipifarnib (R115777) or its less active enantiomer (100-fold less active for inhibition of farnesyltransferase) were added to make final concentrations of 5 µM and 2 µM. Cells were incubated at 37°C, under 5% CO₂ for 1 hour, then 10 µl LPS (diluted in medium) were added per well to reach a final concentration of 10ng/ml. Cells were cultured at 37°C, under 5% CO2. Supernatants were collected at 16 hours, 24 hours and 40 hours of LPS treatment, and IL-6 secretion was quantified.

### NF-κB reporter gene assays

HEK293 cells were transfected with the NF-κB-LH plasmid (Biomyx) using Lipofectamine 2000, and stable clones were selected by culture in hygromycin (176 µg/ml). The resulting NF-κB-LH-transfected cells were seeded in DMEM/10% FCS in 96-well plates for use in luciferase reporter assays. On the following day, cells were treated with tipifarnib for 18 hours, then stimulated with TNF-α (10ng/ml) for 4 hours, after which NF-κB activity was measured using the Steady-Glo® luciferase reporter assay system (Promega). NF-κB activation was calculated as the fold-induction by TNF-α over unstimulated control samples.

### SDS-PAGE and Western blot analysis

THP-1 cells in 0.5 % FBS/RPMI were pre-treated with or without tipifarnib for 18 hours, and subsequently stimulated with LPS (100 ng/ml), TNF-α (10 ng/ml) or IL-1α (5 ng/ml) for 30 minutes. Cells were harvested, washed three times in ice-cold PBS, and lysed in lysis buffer (20 mM Tris (pH 7.5), 150 mM NaCl, 1 mM EDTA, 1mM EGTA, 1% Triton X-100, 2.5 mM sodium pyrophosphate, 1mM β-glycerolphosphate, 1 mM Na₃VO₄, 1 µg/ml Leupeptin, 1 mM PMSF). Cell lysates from each sample were mixed with an equal amount of 2x SDS-PAGE sample buffer (Invitrogen), and proteins were separated by electrophoresis in Tris-glycine 10-20% gradient gels (Novex), and transferred to nitrocellulose membranes (PROTRAN; Schleicher & Schuell). The membranes were blocked with 3% BSA in PBS-0.05%Tween-20, and sequentially incubated with primary antibodies and HRP-conjugated secondary antibodies (anti-rabbit IgG or anti-mouse IgG and IgM; PIERCE), followed by ECL detection (Amersham Biosciences). The primary antibodies used were rabbit polyclonal anti-IκBα, and -p38 (Santa Cruz), mouse monoclonal anti-H-Ras (Chemicon), mouse monoclonal anti-phospho-p38 and rabbit polyclonal anti-phospho-Erk (Cell Signaling Technology).

### RESULTS

### Effect of simvastatin and tipifarnib on LPS-induced gene transcription

Initial analysis of global effects for simvastatin and tipifarnib on transcription of inflammatory genes was performed by cDNA microarray analysis of RNA samples from THP-1 cells, treated with LPS, in the presence or absence of simvastatin or tipifarnib, respectively. Out of 121 genes showing at least 1.5-fold induction by LPS at 2 time points, 37 showed at least 1.5-fold decrease in induction by one or both drugs, at one or more time points (see **Tables 1a, 1b, 1c and 1d** below). By these criteria, simvastatin showed downregulation of 15, and tipifarnib of 34, LPS-induced transcripts. Included in the genes down-regulated by the drugs were chemokines (MCP-1 and -2), cytokines (IL-1β), interferon pathway genes, (STAT-1, interferon β1), receptors (urokinase receptor) and proteases (MMP-9).

**Table 1.a**

| **Accession number** | **GeneName** | **GeneDescription** |
|---|---|---|
| NM_002982 | CCL2 | monocyte chemotactic protein 1 (MCP-1) |
| NM_005623 | CCL8 | monocyte chemotactic protein 2 (MCP-2) |
| NM_002983 | CCL3 | small Inducible cytokine A3 (homologous to mouse Mip-1a) (SCYA3) |
| NM_002984 | CCL4 | small inducible cytokine A4 (homologous to mouse Mip-1b) (SCYA4) |
| NM_005409 | CXCL11 | small inducible cytokine subfamily B, member 11 (SCYB11) |
| NM_000584 | IL8 | interleukin 8 |
| NM_000576 | IL1B | interleukin 1, beta |
| NM_000577 | IL1RN | interleukin-1 receptor antagonist |
| NM_002852 | PTX3 | pentaxin-related gene, rapidly induced by IL-1B |
| NM_007315 | STAT1 | signal transducer and activator of transcription 1 |
| NM_002176 | IFNB1 | interferon, beta 1, fibroblast |
| NM_005531 | IFI16 | interferon, gamma-inducible protein 16 |
| NM_001548 | IFIT1 | interferon-induced protein with tetratricopeptide repeats 1 |
| NM_002462 | MX1 | myxovirus (influenza) resistance 1 |
| NM_000598 | IGFBP3 | insulin-like growth factor-binding protein-3 gene |
| NM_053056 | CCND1 | cyclin D1 (PRAD1: parathyroid adenomatosis 1) |
| NM_002965 | S100A9 | S100 calcium-binding protein A9 (calgranulin B) |
| NM_003897 | IER3 | immediate early response 3 |
| NM_002468 | MYD88 | myleoid differentiation primary response protein |
| NM_002502 | NFKB2 | nuclear factor of kappa light polypeptide gene enhancer in B-cells 2 |
| NM_000593 | TAP1 | transporter 1, ATP-binding cassette, sub-family B (MDR/TAP) |
| NM_000544 | TAP2 | transporter 2, ATP-binding cassette, sub-family B (MDR/TAP) |
| NM_007188 | ABCB8 | ATP-binding cassette, sub-family B (MDR/TAP), member 8 |
| NM_031460 | KCNK17 | potassium channel, subfamily K, member 17 (TASK-4) |
| NM_002659 | PLAUR | plasminogen activator, urokinase receptor |
| NM_004054 | C3AR1 | C3a anaphylatoxin chemotactic receptor (C3a-R) |
| NM_004048 | B2M | beta-2-microglobulin |
| NM_015907 | LAP3 | leucine aminopeptidase |
| NM_004994 | MMP9 | type IV collagenase, matrix metalloproteinase 9 |
| NM_000362 | TIMP3 | tissue inhibitor of metalloproteinase 3 |
| NM_006074 | TRIM22 | tripartite motif-containing 22 |
| NM_004223 | UBE2L6 | ubiquitin-conjugating enzyme E2L 6 |
| NM_144573 | NEXN | nexilin (F actin binding protein) |
| NM_021634 | LGR7 | leucine-rich repeat-containing G protein-coupled receptor 7 |
| NM_198066 | GNPNAT1 | glucosamine-phosphate N-acetyltransferase 1 |
| NM_002346 | LY6E | lymphocyte antigen 6 complex, locus E |

**Table 1.b**

| **Accession number** | **Gene Name** | **Fold-change (LPS vs control** | | | | |
|---|---|---|---|---|---|---|
| | | **1hr** | **3hr** | **6hr** | **12hr** | **24hr** |
| NM_002982 | CCL2 | 1.00 | 1.41 | ***9.85*** | ***9.85*** | ***21.11*** |

| **Accession number** | **Gene Name** | **Fold-change (LPS vs control)** | | | | |
|---|---|---|---|---|---|---|
| | | **1hr** | **3hr** | **6hr** | **12hr** | **24hr** |
| NM_005623 | CCL8 | 1.00 | 1.00 | ***3.48*** | ***2.30*** | ***3.73*** |
| NM_002983 | CCL3 | 1.32 | ***4.92*** | ***6.06*** | ***4.29*** | ***7.46*** |
| NM_002984 | CCL4 | 1.32 | ***5.28*** | ***8.00*** | ***5.66*** | ***6.96*** |
| NM_005409 | CXCL11 | 1.00 | 1.15 | ***7.46*** | ***3.03*** | ***2.30*** |
| NM_000584 | IL8 | 1.41 | ***8.00*** | ***6.06*** | ***22.63*** | ***22.63*** |
| NM_000576 | IL1B | 1.41 | ***5.66*** | ***8.00*** | ***6.96*** | ***8.00*** |
| NM_000577 | IL1RN | 1.07 | 1.00 | ***3.25*** | ***1.87*** | ***4.29*** |
| NM_002852 | PTX3 | -1.07 | ***2.00*** | ***4.00*** | ***3.03*** | ***3.48*** |
| NM_007315 | STAT1 | -1.23 | 1.15 | ***4.2*** | ***3.73*** | ***4.29*** |
| NM_002176 | IFNB1 | 1.07 | 1.23 | ***3.25*** | ***6.06*** | ***1.87*** |
| NM_005531 | IFI16 | -1.07 | 1.00 | ***1.74*** | ***2.00*** | ***2.83*** |
| NM_001548 | IFIT1 | 1.00 | 1.07 | ***2.83*** | ***2.83*** | ***3.03*** |
| NM_002462 | MX1 | 1.00 | 1.07 | ***4.92*** | ***3.03*** | ***3.25*** |
| NM_000598 | IGFBP3 | 1.00 | 1.15 | ***1.62*** | ***4.00*** | ***7.46*** |
| NM_053056 | CCND1 | 1.00 | 1.07 | 1.23 | ***1.74*** | ***2.00*** |
| NM_002965 | S100A9 | -1.07 | -1.07 | 1.00 | ***1.52*** | ***1.74*** |
| NM_003897 | IER3 | 1.23 | 1.41 | ***1.87*** | ***2.00*** | ***2.14*** |
| NM_002468 | MYD88 | 1.00 | 1.07 | ***3.25*** | ***1.74*** | ***1.74*** |
| NM_002502 | NFKB2 | 1.07 | 1.41 | ***2.83*** | ***2.46*** | ***1.52*** |
| NM_000593 | TAP1 | 1.23 | 1.32 | ***4.92*** | ***2.14*** | ***1.74*** |
| NM_000544 | TAP2 | 1.07 | 1.07 | ***2.00*** | ***1.87*** | 1.41 |
| NM_007188 | ABCB8 | 1.00 | 1.00 | ***2.30*** | ***1.52*** | ***1.52*** |
| NM_031460 | KCNK17 | 1.07 | 1.23 | ***3.03*** | ***1.52*** | ***2.00*** |
| NM_002659 | PLAUR | -1.07 | 1.15 | ***1.6*** | ***1.87*** | ***3.25*** |
| NM_004054 | C3AR1 | -1.07 | 1.15 | ***20*** | ***2.30*** | ***3.48*** |
| NM_004048 | B2M | -1.07 | 1.07 | ***1.6*** | ***1.52*** | ***2.14*** |
| NM_015907 | LAP3 | -1.07 | 1.00 | ***2.46*** | ***2.30*** | ***4.00*** |
| NM_004994 | MMP9 | -1.07 | -1.07 | ***1.52*** | ***3.48*** | ***13.93*** |
| NM_000362 | TIMP3 | NA | NA | ***2.46*** | -1.41 | ***-2.00*** |
| NM_006074 | TRIM22 | 1.00 | 1.00 | ***2.83*** | ***3.03*** | ***2.83*** |
| NM_004223 | UBE2L6 | 1.00 | -1.07 | ***5.28*** | ***5.28*** | ***4.59*** |
| NM_144573 | NEXN | 1.00 | 1.00 | ***2.00*** | ***1.52*** | ***1.52*** |
| NM_021634 | LGR7 | 1.07 | -1.07 | ***1.6*** | ***1.52*** | ***1.87*** |
| NM_198066 | GNPNAT1 | 1.00 | 1.00 | ***3.25*** | ***2.14*** | ***1.74*** |
| NM_002346 | LY6E | 1.15 | 1.00 | 3.03 | 3.73 | 5.28 |

**Table 1.c**

| **Accession number** | **GeneName** | **Fold-change (simvastatin/LPS vs LPS)** | | | | |
|---|---|---|---|---|---|---|
| | | **1hr** | **3hr** | **6hr** | **12hr** | **24hr** |
| NM_002982 | CCL2 | 1.00 | -1.23 | ***-1.62*** | ***-2.14*** | ***NA*** |
| NM_005623 | CCL8 | -1.07 | -1.07 | -1.32 | ***-1.52*** | -1.23 |
| NM_002983 | CCL3 | 1.15 | 1.00 | ***-1.62*** | -1.15 | 1.41 |
| NM_002984 | CCL4 | -1.07 | ***1.74*** | -1.32 | -1.15 | 1.41 |
| NM_005409 | CXCL11 | 1.00 | 1.00 | -1.15 | 1.32 | -1.32 |
| NM_000584 | IL8 | -1.15 | -1.23 | -1.07 | -*1.74* | 1.23 |
| NM_000576 | IL1B | 1.00 | -1.15 | -*1.62* | -*2.14* | *-2.00* |
| NM_000577 | IL1RN | -1.15 | -1.07 | -1.15 | -1.07 | ***-1.62*** |
| NM_002852 | PTX3 | -1.07 | -1.07 | -1.07 | -1.23 | 1.23 |
| NM_007315 | STAT1 | 1.07 | 1.07 | -1.32 | 1.07 | ***-1.52*** |
| NM_002176 | IFNB1 | -1.15 | -1.15 | 1.23 | ***-1.87*** | -1.23 |
| NM_005531 | IFI16 | 1.00 | 1.00 | 1.00 | -1.07 | -1.32 |
| NM_001548 | IFIT1 | 1.00 | 1.00 | 1.00 | -1.07 | -1.15 |
| NM_002462 | MX1 | 1.00 | 1.07 | -1.15 | 1.23 | -1.41 |
| NM_000598 | IGFBP3 | 1.00 | 1.07 | 1.00 | 1.15 | 1.07 |
| NM_053056 | CCND1 | -1.07 | 1.00 | -1.07 | **-1.62** | *-1.52* |
| NM-002965 | S100A9 | -1.07 | -1.07 | -1.07 | -1.32 | 1.41 |
| NM_003897 | IER3 | 1.00 | 1.00 | -1.23 | ***-1.62*** | 1.23 |
| NM_002468 | MYD88 | 1.07 | 1.07 | -1.07 | 1.00 | 1.00 |
| NM_002502 | NFKB2 | -1.07 | 1.00 | ***-1.74*** | -1.07 | 1.41 |
| NM_000593 | TAP1 | -1.07 | 1.15 | -1.41 | 1.00 | 1.15 |
| NM_000544 | TAP2 | 1.00 | 1.00 | -1.23 | 1.00 | -1.15 |
| NM_007188 | ABCB8 | 1.00 | 1.07 | 1.00 | 1.00 | -1.07 |
| NM_031460 | KCNK17 | 1.00 | 1.00 | 1.07 | 1.00 | -1.32 |
| NM_002659 | PLAUR | 1.00 | -1.15 | -1.23 | ***-1.87*** | -1.41 |
| NM_004054 | C3AR1 | -1.15 | 1.00 | 1.00 | -1.32 | 1.32 |
| NM_004048 | B2M | 1.07 | 1.00 | 1.07 | -1.07 | 1.00 |
| NM_015907 | LAP3 | 1.00 | 1.00 | 1.15 | 1.07 | ***-1.52*** |
| NM_004994 | MMP9 | 1.00 | 1.00 | -1.15 | -1.32. | ***-2.14*** |
| NM_000362 | TIMP3 | NA | NA | **-*2.64*** | -1.07 | -1.07 |
| NM_006074 | TRIM22 | 1.00 | 1.07 | 1.07 | 1.07 | -1.15 |
| NM_004223 | UBE2L6 | -1.07 | 1.00 | 1.00 | 1.15 | -1.62 |
| NM_144573 | NEXN | -1.07 | 1.07 | -1.07 | 1.15 | -1.32 |
| NM_021634 | LGR7 | 1.07 | 1.15 | -1.07 | -1.41 | -1.32 |
| NM_198066 | GNPNAT1 | 1.00 | 1.00 | -1.07 | 1.15 | -1.23 |
| NM_002346 | LY6E | 1.15 | 1.00 | -1.07 | ***1.52*** | -1.15 |

**Table 1.d**

| **Accession number** | **Gene Name** | **Fold-change (tipifarnib/LPS vs LPS)** | | | | |
|---|---|---|---|---|---|---|
| | | **1hr** | **3hr** | **6hr** | **12hr** | **24hr** |
| NM_002982 | CCL2 | -1.07 | -1.32 | ***-2.83*** | ***-3.25*** | ***-4.29*** |
| NM_005623 | CCL8 | -1.07 | 1.00 | ***-2.30*** | **-2.3C** | ***-3.48*** |
| NM_002983 | CCL3 | -1.07 | 1.00 | -1.07 | 1.23 | ***-1.52*** |
| NM_002984 | CCL4 | 1.07 | ***1.87*** | 1.07 | ***-1.52*** | ***-1.52*** |
| NM_005409 | CXCL11 | 1.00 | -1.07 | ***-2.83*** | **-*1.52*** | **-*2.00*** |
| NM_000584 | IL8 | -1.07 | -1.23 | -1.15 | 1.32 | -1.07 |
| NM_000576 | IL1B | 1.07 | 1.32 | **-*1.87*** | **-*2.46*** | -1.23 |
| NM_000577 | IL1RN | -1.15 | -1.15 | **-*1.62*** | -1.15 | **-*3.73*** |
| NM_002852 | PTX3 | 1.15 | -1.15 | -1.32 | -1.32 | **-*2.00*** |
| NM_007315 | STAT1 | 1.23 | -1.32 | **-*2.14*** | 1.00 | **-*1.62*** |
| NM_002176 | IFNB1 | -1.23 | -1.15 | **-*1.62*** | **-*3.03*** | ***2.14*** |
| NM_005531 | IF116 | 1.07 | 1.00 | -1.32 | -1.23 | **-*1.74*** |
| NM_001548 | IFIT1 | 1.00 | 1.00 | -1.41 | -1.32 | **-*2.14*** |
| NM_002462 | MX1 | 1.00 | -1.07 | ***-1.74*** | 1.32 | -1.32 |
| NM_000598 | IGFBP3 | 1.00 | -1.07 | 1.00 | **-*1.52*** | **-*2.00*** |
| NM_053056 | CCND1 | 1.00 | -1.07 | -1.15 | **-*1.62*** | -1.23 |
| NM_002965 | S100A9 | 1.00 | -1.15 | -1.07 | **-*2.00*** | **-*1.62*** |
| NM_003897 | IER3 | 1.32 | ***1.52*** | 1.15 | 1.00 | 1.23 |
| NM_002468 | MYD88 | 1.07 | -1.15 | **-*2.14*** | -1.23 | -1.32 |
| NM_002502 | NFKB2 | -1.07 | 1.23 | -1.07 | -1.32 | ***1.52*** |
| NM_000593 | TAP1 | -1.23 | 1.15 | **-*1.74*** | -1.15 | -1.23 |
| NM_000544 | TAP2 | 1.07 | -1.15 | ***-1.62*** | -1.32 | -1.23 |
| NM_007188 | ABCB8 | 1.07 | 1.00 | **-*1.62*** | -1.23 | -1.23 |
| NM_031460 | KCNK17 | -1.07 | -1.15 | -1.32 | 1.00 | **-*1.74*** |
| NM_002659 | PLAUR | 1.00 | -1.23 | **-*1.62*** | ***-1.87*** | **-*1.62*** |
| NM_004054 | C3AR1 | 1.00 | 1.07 | -1.23 | **-*1.62*** | **-*1.74*** |
| NM_004048 | B2M | 1.15 | 1.00 | -1.23 | ***-1.62*** | 1.15 |
| NM_015907 | LAP3 | -1.07 | -1.07 | **-*1.62*** | -1.23 | **-*2.83*** |
| NM_004994 | MMP9 | -1.07 | -1.07 | -1.41 | -***1.74*** | -1.41 |
| NM_000362 | TIMP3 | NA | -1,41 | -1.23 | -1.15 | 1.15 |
| NM_006074 | TRIM22 | 1.00 | 1.00 | **-*1.62*** | -1.07 | **-*1.52*** |
| NM_004223 | UBE2L6 | 1.00 | -1.07 | -1.41 | -1.15 | -1.41 |
| NM_144573 | NEXN | 1.00 | 1.00 | -1.41 | -1.15 | **-*1.62*** |
| NM_021634 | LGR7 | 1.23 | -1.15 | -1.41 | **-*1.62*** | **-*1.52*** |
| NM_198066 | GNPNAT1 | 1.00 | 1.00 | **-*2.00*** | -1.41 | **-*1.74*** |
| NM_002346 | LY6E | ***1.52*** | -1.15 | **-*1.52*** | 1.15 | -1.32 |

**Tables 1a**, **1b, 1c** and **1d** show LPS-induced genes that were inhibited by simvastatin or tipifarnib. Genes were selected (see **Table 1a** for description of genes) as LPS-induced if the ratio between LPS-treated and non-treated samples was greater than 1.5-fold at more than two time points. These are shown as ***bold italicized numbers*** in the "Fold-change (LPS versus control)" column (**Table 1.b**). Gene expression during treatment with LPS and the statin (simvastatin/LPS) (**Table 1.c**), or LPS and the FTI (tipifamib/LPS) (**Table 1.d**), was compared with LPS alone at corresponding time points. Genes that showed at least 1.5-fold decrease in induction by one or both drugs, at one or more time points were selected for inclusion in the table. Genes for which LPS induction was inhibited according to these criteria are shown as ***negative, bold and italicized numbers.***

The results of the microarray analysis were confirmed for selected genes by real-time PCR analysis **(****Figure 2****).** Transcription of IL-1β was induced by LPS as early as 3 hours, and the induction was unaffected by simvastatin or tipifarnib up to this time point. However, after 6 hours, and to a greater extent, after 12 hours the LPS-induced induction significantly downregulated by simvastatin and tipifarnib **(****Figure 2.a**). MCP-1 was induced by LPS from 6 hours, and this induction was inhibited significantly by simvastatin and tipifarnib **(****Figure 2****.b**). MMP-9 induction by LPS increased up to 12 hours, at which time point a 50% decrease in induction was observed for simvastatin, and no induction in the presence of tipifarnib **(**Figure 2.c). Induction of IL-8 by LPS was evident as early as 3 hours and was unaffected by the drugs at this time point. At 6 and 12 hours, IL-8 induction was inhibited by simvastatin, while the FTI showed no significant effect **(**Figure 2.d). STAT1 induction was inhibited by tipifarnib at 6 and 12 hours, while inhibition by the statin was only observed at 12 hours **(****Figure 2.e**). Myeloid differentiation factor, MyD88, the adaptor for Toll-like receptor-mediated responses, was significantly upregulated by LPS at 6 and 12 hours, and this was marginally inhibited by simvastatin at 12 hours **(****Figure 2.f**). In contrast, tipifarnib showed almost complete inhibition of MyD88 induction, down to levels similar to that in unchallenged cells. While IL-6 transcription did not meet the criteria from the microarray analysis for inclusion in Table 1, analysis by quantitative real-time PCR showed inhibition by simvastatin, and particularly tipifarnib, of the peak of LPS-induced IL-6 transcription at 6 hours **(Figure 2g)****.** At 12 hours, only tipifarnib showed inhibition.

Thus, both simvastatin and tipifarnib were able to inhibit LPS induction of numerous inflammatory genes and tipifarnib showed stronger inhibition in most cases. Tipifarnib also showed downregulation of some transcripts not affected by the statin.

### Effect of simvastatin and tipifarnib on LPS-induced cytokine production

To further investigate the effects of simvastatin and tipifarnib at the protein level, cytokine and MMP-9 secretion by THP-1 cells in response to LPS was examined at different time points over 48 hours, in the presence or absence of simvastatin (5 and 10 µM) or tipifarnib (2 and 5 µM), respectively.

MCP-1 secretion **(****Figure 3.a**) was dose-dependently inhibited by simvastatin at 22 and 30 h after LPS addition, but the inhibitory effect was lost at 48 hours. Tipifarnib showed dose-dependent inhibition of MCP-1 secretion up to 48 hours. Even at 2 µM, the extent of inhibition by tipifarnib was much greater than that observed for 10 µM simvastatin. Both drugs showed inhibition of IL-6 secretion (**Figure 3****.b**), the extent of which was again greater in the presence of tipifarnib. Inhibition by 5 µM simvastatin was observed only at 30 hours, and at 10 µM, was observed at both 30 and 48 hours. The extent of inhibition of IL-6 release was similar for 2 and 5 µM tipifarnib and inhibition was observed at all time points.

An IC₅₀ of approximately 1 µM was obtained for tipifarnib inhibition of LPS-induced IL-6 secretion in a separate experiment (**Figure 4**). The IC₅₀ of approximately 1 µM was obtained for inhibition of LPS-induced IL-6 release by tipifarnib in the following manner: THP-1 cells were cultured in RPMI-1640 medium, containing 10% FBS, 100 units/ml Penicillin, 10 mg/ml Streptomycin, at 37°C, under 5% CO₂. Medium components were certified to contain less than 0.3 endotoxin units/ml. THP-1 cells at 3.4 x 105/ml were cultured in 96-well plates in 0.5%FBS/RPMI 1640, in the absence or presence of 100ng/ml LPS plus or minus tipifarnib (starting from 5uM, followed by 3-fold series dilution). The concentrations of tipifarnib were: 5 µM, 1.67µM, 0.56uM, 0.19 uM and 0,06uM. Supernatants were collected at 48hr. IL6 was tested using ELISA kit from R&D Systems. Results are shown in **Figure 4**.

Both simvastatin and tipifarnib showed significant inhibition of LPS-induced IL-1β transcription (**Figure 2****.a**). However, the results on IL-1β secretion were more complex **(****Figure 3.c**). While tipifarnib showed dose-dependent inhibition of IL-1β secretion, simvastatin at 5µM had little effect, and at 10 µM showed significant stimulation of IL-1β secretion at 22 and 30 hours. Simvastatin inhibited LPS-induced IL-8 production at 22, 30 and 48 hours, showing dose-dependence at 48h (**Figure 3****.d**). In contrast, tipifarnib, showed no significant inhibition of IL-8 production, except for 5 µM at 22 hours. These results are consistent with the lack of effect for tipifarnib on IL-8 transcription (**Figure 2****.d**). Both simvastatin and tipifarnib dose-dependently inhibited LPS-induced MMP-9 secretion at 30 and 48 hours **(****Figure 3****.e**).

LPS-induced TNF-α production by THP-1 cells peaked between 5 and 10 hours, and then showed a secondary induction after 22 h up to 48 hours. The initial peak of induction appeared to be unaffected by the presence of either inhibitor, but the second phase of induction showed dose-dependent inhibition by simvastatin, and, more significantly, by tipifarnib (**Figure 3****.f**). At 5 µM, tipifarnib totally inhibited the second phase of LPS-induced TNF-α production, while 2 µM tipifarnib showed a similar extent of inhibition to 10 µM simvastatin.

### Effect of tipifarnib and its less active enantiomer on LPS-induced IL-6 production in human peripheral blood mononuclear cells (PBMC)

This experiment was performed to test the effect of tipifarnib on LPS-induced IL-6 production in human peripheral blood mononuclear cells (PBMC) as an example of how response to treatment with tipifarnib could be monitored in isolated PBMC from patients treated with tipifarnib. The enantiomer of tipifarnib is about 100-fold less active than tipifarnib (R 115777) for inhibition of farnesyltransferase (Venet M., "R115777 as farnesyl protein transferase inhibitor: Synthesis and SAR." ACS Meeting 2001, 222nd:Chicago (Abs MEDI 5)). IL-6 could not be detected after incubation of human PBMC for up to 40 hours. Incubation of human PBMC with LPS strongly induced IL-6 production after 16 hours and production continued to increase up to 40 hours. No difference in production was noted when the less active enantiomer was added to the incubation at 2 or 5 µM. The presence of tipifarnib at 2 µM during LPS stimulation had no effect on IL-6 production but at 5 µM, highly significant inhibition of IL-6 production was observed after 16 and 40 hours (P < 0.01). At 24 hours, the mean value in the presence of 5 µM tipifarnib was approximately 40 % lower than in its absence, but the data did not reach statistical significance (P = 0.104). Thus, tipifarnib showed dose-dependent inhibiton of LPS-induced IL-6 production in human PBMC. The concentration required for inhibition in PBMC was higher than that required for inhibition in THP-1 cells and this difference may be related to the different concentrations of fetal bovine serum in these two experiments (10% and 0.5% FBS, respectively) because tipifarnib is known to be highly protein-bound.

### Effect of simvastatin and tipifarnib on LPS-induced cytokine production in vivo

To follow up on our *in vitro* observations, the effects of simvastatin and tipifarnib pretreatment were tested in a murine model of LPS-induced inflammation. The compounds were orally administered at 24 hours, 17 hours, and 1 hour before LPS injection.

Especially striking effects of the statin and the FTI were their highly significant inhibition of LPS-induced TNF-α production at I and 2 hours **(****Figure 5****.a**). A similar result has previously been obtained for another statin, cerivastatin (Ando, Takamura et al. 2000). This effect has now been observed for an FTI.

No significant effects were seen for simvastatin on IL-6 and IL-1β production, while approximately 50% inhibition of IL-6 production (**Figure 5****.b**)**,** and almost complete inhibition of IL-1β production (**Figure 5****.c**), was observed in tipifarnib-treated animals after 3 hours.

Approximately 50% inhibition of MIP-1α production by both the statin and the FTI was observed at 2 hours in the first experiment (**Figure 5****.d**)**.** In the second experiment, similar inhibition for tipifarnib was observed at 2 and 3 hours, and less, but significant inhibition for simvastatin at 2 hours. No significant effects were observed for simvastatin treatment on MCP-1 production, while tipifarnib showed significant inhibition at 2 hours and a decrease that did not reach statistical significance at 3 hours (**Figure 5****.e**).

In the second experiment, IL12-p40 and -p70 induction by LPS was inhibited by tipifarnib at 3 hours, while simvastatin had no significant effect (results not shown). No effects of simvastatin or tipifarnib on LPS-induced IL,-8 or RANTES were observed (results not shown).

Overall, the results show significant downregulation of numerous LPS-induced cytokines *in vivo* by tipifarnib, and of TNF-α and MIP-1α for simvastatin.

### Effect of tipifarnib on TNF-α, LPS, and IL-1-induced signaling

It was recently reported that TNF-α induced NF-κB activity was inhibited by another FTI (SCH 66336) (Takada, Y., et al. 2004. J Biol Chem 279, 26287-99). To compare the effects of tipifarnib to this finding, NF-κB activation was tested in HEK 293 cells, stably transfected with an NF-κB luciferase reporter plasmid. Cells were treated with tipifarnib overnight, followed by stimulation with TNF-α for 4 hours. In contrast to the results reported for SCH 66336, TNF-α induced NF-κB activity was not inhibited by tipifarnib at 2 or 5 µM (**Figure 8**).

The effect of tipifarnib on several signalling pathways was also examined in THP-1 cells. Cells were treated with 2 µM tipifarnib for 18 hours, then stimulated with LPS or TNF-α for 4 hours. Cell lysates were tested for p38 expression and p38 phosphorylation by western blot analysis. Phosphorylation of p38 was significantly increased by LPS and TNF-α stimulation, and only the LPS-induced increase was partially reduced by tipifarnib treatment (**Figure 7****.a**). No significant inhibition was observed for TNF-α induced p38 phosphorylation. P38 protein expression was not affected by LPS or TNF-α stimulation and/or tipifarnib treatment.

In a separate experiment, IL-1α was also included as a stimulus besides LPS and TNF-α, and these three stimuli all significantly increased IκB-α degradation. Interestingly, tipifarnib significantly inhibited LPS-induced IκB degradation, but showed no inhibition of IκB degradation induced by TNF-α and IL-1α (**Figure 7****.b**). Similar results were observed in three separate experiments. The level of ERK phosphorylation was similar in control and stimulated cells, and tipifarnib pre-treatment reduced the ERK phosphorylation to a similar extent in all samples. The effect of tipifarnib on H-Ras farnesylation was also examined. The level of farnesylated H-Ras was not significantly changed in the absence or presence of stimuli. Tipifarnib partially inhibited H-Ras farnesylation to the same degree in the different treatment groups, and this inhibition correlated with the extent of inhibition of ERK phosphorylation

### DISCUSSION

Some of the anti-inflammatory effects of statins appear to be mediated through their inhibition of protein prenylation, including famesylation. In the comparison of the statin (simvastatin) to the FTI (tipifarnib), the FTI showed similar, if not superior, anti-inflammatory activity.

Both drugs inhibited transcription and secretion of IL-6, MCP-1 and MMP-9, and the secondary phase of TNF-α secretion *in vitro.*

In the experiments of the present invention, it was found that both simvastatin and tipifarnib dose-dependently inhibited LPS-induced MMP-9 secretion.

Inhibition of STAT1 transcription induced by LPS was observed for both tipifarnib and for simvastatin. Previous studies have shown inhibition of STAT1 phosphorylation by statins (Huang, K.C., et al. 2003. Journal of Biomedical Science (Basel, Switzerland) 10, 396-405; Chung, H. K., et al. 2002. Experimental and Molecular Medicine 34, 451-461; and Horiuchi, M., et al. 2003. Circulation 107, 106-112. In contrast, the present invention provides the first evidence for inhibition of STAT1 transcription by tipifarnib and, to a lesser extent, simvastatin.

LPS-induced MyD88 transcription was significantly inhibited by tipifarnib, but not by simvastatin, suggesting that selective inhibition of farnesylation may inhibit signaling through Toll-like receptors.

Simvastatin and tipifarnib had contrasting effects on inhibition of LPS-induced IL-8 and IL-1β. Simvastatin inhibited IL-8 mRNA induction and IL-8 secretion by THP-1 cells, as previously reported (Ito, T., et al. 2002. Atherosclerosis 165, 51-5; Takata, M., et al. 2001. Br J Pharmacol 134, 753-62; Terkeltaub, R., et al. 1994. J Leukoc Biol 55, 749-55.)

In contrast, however, no inhibition of LPS-induced IL-8 transcription or secretion was observed in the studies of the present application for the FTI, tipifarnib, at concentrations that were highly effective at inhibiting IL-6, MCP-1 and MMP-9 production. These results suggest that LPS-induced IL-8 induction does not require protein farnesylation under the conditions in the studies shown in the present application. However under certain conditions, for example, pre-incubation of human PBMC with an FTI overnight, followed by LPS treatment for 24 hours, some inhibition of LPS-induced IL-8 production could be observed.

While both the statin and the FTI inhibited LPS-induced IL-1β transcription, only the FTI showed inhibition of IL-1β secretion. Simvastatin showed no effect on IL-1β secretion at 5 µM, and induction at 10 µM. Similar potentiation of inflammatory mediators by statin treatment has previously been observed in numerous studies (Terkeltaub, R., et al. 1994. J Leukoc Biol 55, 749-55; Sadeghi, M. M., et al. 2000. J Immunol 165, 2712-8; Sun, D. et al. 2003. Cell Immunol 223, 52-62; Monick, M. M., et al. 2003. J Immunol 171, 2625-30; and Matsumoto, M., et al. 2004. J Immunol 172, 7377-84.)

The difference between the effects of simvastatin on transcription and secretion of IL-1β may be related to the requirement for cleavage of the immature precursor protein by caspase-1 for secretion. A lipophilic statin, similar to simvastatin, has been shown to stimulate caspase-1 activity and IL-1β secretion in human peripheral blood mononuclear cells (Montero, Hernandez et al. 2000).

LPS has been shown to activate caspase-1 in THP-1 cells, and this activation contributes to LPS induction of IL-1β secretion (Schumann, Belka et al. 1998) Additional activation of caspase-1 by simvastatin may therefore potentiate LPS-induced secretion of mature IL-1β protein, even though transcription is inhibited.

The effects of simvastatin and tipifarnib pre-treatment on *in vivo* induction of cytokines in mice by LPS were also analyzed. The most striking effects of the statin and the FTI were their highly significant inhibition of LPS-induced TNF-α production.

Simvastatin pre-treatment gave similar inhibition of TNFα and IL-1β to that observed previously for cerivastatin (Ando, Takamura et al. 2000), although in the present study, the inhibition of IL-1β did not reach statistical significance.

The studies of the present invention additionally showed downregulation of LPS-induced MIP-1α by simvastatin. This has not previously been shown *in vivo,* although a prior study reported decreased spontaneous release of MIP-1α in cultured, cryopreserved PBMCs from patients after six months treatment with atorvastatin (Waehre T, et al. 2003. J Am Coll Cardiol 41(9), 1460-7).

In the studies of the present invention, inhibition of IL-8 production *in vivo* by either simvastatin or tipifarnib was not observed, although downregulation of this cytokine *in vitro* by simvastatin was observed. Similarly, Waehre at al observed a modest decrease in spontaneous, and no decrease in LPS-induced IL-8 from PBMC before and after atorvastatin treatment. In contrast, it has been reported that simvastatin reduced serum levels of IL-8 in hypercholesterolemic patients after 6 weeks of treatment (Rezaie-Majd, A., et al. 2002. Arterioscler Thromb Vasc Biol 22, 1194-9.) The differences in effects on IL-8 production may be related to different statins, different patient populations (CAD versus hypercholesterolemia), and/or *in vivo* versus *ex vivo* culture measurements.

The studies of the present invention provide *in vivo* evidence for global anti-inflammatory effects of FTIs, tipifarnib in particular, including downregulation of LPS-induced TNFα, IL-6, IL-1β and MIP-1α. Previous research with tipifarnib has shown inhibition of cachexia (U.S. Published Patent Application No. 2004/0157773 and WO 02/085364), and reduction of disease scores in dextran sodium sulphate-induced colitis (WO 02/43733) and collagen-induced arthritis (WO 00/01386), but the mechanism for these effects was not reported. The major roles of the TNF-α, IL-6, and IL-1β in the pathogenesis of these diseases suggest that their inhibition by the FTI may have been responsible for the observed amelioration. It is of interest to note that in Phase I studies with tipifarnib in myelodysplastic syndrome, the only correlation with positive response to treatment was a decrease in serum levels of TNF-α (Kurzrock, Kantarjian et al. 2003).

The concentrations of simvastatin used in the *in vitro* and *in vivo* studies reported of the present invention were higher than typical therapeutic doses, but similar to those previously used in other *in vitro* studies and murine efficacy models (Leung, Sattar et al. 2003; McKay A 2004). Numerous studies have now shown anti-inflammatory activity for statins at therapeutic doses in humans (Rezaie-Majd, Maca et al. 2002; Waehre T 2003; McCarey DW 2004), indicating that the results of *in vitro* and animal studies have clinical relevance.

In the case of the FTI, tipifarnib, 300 mg twice a day was the maximum tolerated dose in a number of studies (Head and Johnston 2003). At this dose, plasma levels reach approximately 2 µM (Karp, Lancet et al. 2001), at which concentration significant effects were obtained *in vitro* in the experiments of the present invention. The doses used in the LPS-induced murine inflammation model of the present invention were similar to those used in reported tumoricidal effects in mouse models (End, Smets et al. 2001).

Inhibition of inflammatory mediators by the statin and the FTI appeared to require pre-treatment time. For example, cytokines such as IL-1β and IL-8 showed significant transcriptional induction by LPS as early as 3 hours *in vitro.* When drug treatment *in vitro* was started at the same time as the LPS stimulation, the earliest signs of transcriptional inhibition were only observed after 6 hours, and of cytokine release only after 22 hours. The initial peak of LPS-induced TNF-α release was unaffected by the presence of either inhibitor, but the second phase after 22 hours, showed dose- and time-dependent inhibition by simvastatin and tipifarnib. In contrast, when animals were pre-treated with the drugs for 24 hours before LPS challenge *in vivo,* inhibition of TNF-α release could be observed as early as one hour after the challenge. The lag time in the inhibition of inflammatory responses may indicate a requirement to deplete intracellular pools of particular prenylated proteins.

The anti-inflammatory effects of statins and FTIs may be related to their inhibition of common steps in the mevalonate pathway, and/or the recent evidence that both classes of drugs inhibit activation of NFκB (Ortego M 1999; Takada, Khuri et al. 2004, Na et al. 2004).

To explore the underlying mechanism for the anti-inflammatory activity of tipifarnib, the effect of tipifarnib on different pro-inflammatory signaling pathways was examined. The FTI, SCH 66336, was previously shown to inhibit TNF-α induced IκB phosphorylation, degradation and NF-κB activity (Takada, Khuri et al. 2004). However, in the studies supporting the present invention, tipifarnib did not affect TNF-α induced NF-κB activity (as measured by a luciferase reporter assay). In addition, tipifarnib showed no inhibition of TNF-α induced IκB degradation in THP-1 cells. The reason for such contrasting results is not clear, but it can be noted that these two FTIs have also shown different efficacy in clinical trials for different tumor types. (Lancet and Karp, 2003)

The Ras/Raf/MAPK pathway has been well studied. The studies of the present invention demonstrate that tipifarnib partially inhibits ERK1/2 phosphorylation, and that the extent of inhibition correlates with that of inhibition of Ras farnesylation both in the absence or in the presence of inflammatory stimuli. This data confirms that tipifarnib suppresses ERK1/2 phosphorylation through Ras inhibition.

LPS, TNF-α and IL-1 interact with their own receptors to trigger the corresponding inflammatory signaling cascades. TNF-α signaling links to IKK through TNFR/RIP (Aggarwal, B. Nat Rev Immunol 2003). IL-1/IL-1R and LPS/TLR4 signaling both link to IKK through the shared MyD88/IRAK4/TRAF6/TAK pathway (Takeda, S. A. K. Nature Reviews Immunology 2004). IKK phosphorylates IκB, which leads to the ubiquitination and degradation of IκB, and thus enables the translocation of NF-κB to the nucleus and induction of target gene expression such as TNF-α, It-1β, IL-6, and others. In the studies of the present invention, tipifarnib did not inhibit TNF-α and IL-1 induced IκB degradation, suggesting that the target protein for tipifarnib is not involved in these two signal pathways. Besides the common MyD88 dependent pathway shared with IL-1, the LPS/TLR4 pathway also induces IκB degradation through MyD88-independent pathways, i.e. through TRIF and TRAM (Takeda, S. A. K. Nature Reviews Immunology, 2004). The specific inhibition by tipifarnib only of LPS-induced IκB degradation suggests that the protein affected by tipifarnib is either associated with TLR4 or involved in a pathway exclusive for LPS/TLR4, such as the MyD88-independent pathway. A recent study has shown that Ras is involved in CpG oligonucleotide signaling as an early event, by associating with TLR9 and promoting IRAK/TRAF6 complex formation (Xu, H. A, J Bio Chem. 2003). Another study has shown that Ras controls TRAF-6-dependent induction of NF-κB (Caunt, C. J. J Bio Chem. 2001) In the present invention, inhibition of Ras farnesylation was consistently observed in control, LPS-, IL-1- and TNF-α treated cells, but inhibition of IκB degradation was only observed in the LPS-treated cells. This lack of correlation indicates that Ras is unlikely the target protein involved in mediating the anti-inflammatory effects of tipifarnib.

Although the invention has been illustrated above by reference to a detailed description and to exemplary and preferred embodiments, the invention is intended not to be limited by the foregoing description, but to be defined by the following claims as properly construed under principles of patent law.

### References:

Aggarwal, B. (2003). Signalling pathways of the TNF superfamily: a double-edged sword. Nat Rev Immunol 3(9):745.
Aiello, R., et al. (1999). "Monocyte chemoattractant protein-1 accelerates atherosclerosis in apolipoprotein E-deficient mice." Arterioscler Thromb Vasc Biol 19:1518.
Almog, Y., et al. (2004). "Prior statin therapy is associated with a decreased rate of severe sepsis." Circulation: 110:880.
Aiello, R. J., P. A. Bourassa, et al. (1999). "Monocyte chemoattractant protein-1 accelerates atherosclerosis in apolipoprotein E-deficient mice." Arterioscler Thromb Vasc Biol 19(6): 1518-25.
Almog, Y., A. Shefer, et al. (2004). "Prior statin therapy is associated with a decreased rate of severe sepsis." Circulation 110(7): 880-5.
Ando, H., T. Takamura, et al. (2000). "Cerivastatin improves survival of mice with lipopolysaccharide-induced sepsis." J Pharmacol Exp Ther 294(3): 1043-6.
Bossink, A. W., L. Paemen, et al. (1995). "Plasma levels of the chemokines monocyte chemotactic proteins-1 and -2 are elevated in human sepsis." Blood 86(10): 3841-7.
Brown, J. L. G. M. S. (1990). "Regulation of the mevalonate pathway." Nature 343: 425-430.
Casey, PJ. (1995). "Protein lipidation in cell signaling." Science 268(5208): 221-5.
Caunt, C. J. K.-T., Endre; Carlotti, Franco; Chapman, Robert; Qwarnstrom, Eva E. 2001. Ras controls tumor necrosis factor receptor-associated factor (TRAF)6-dependent induction of nuclear factor-kB. Selective regulation through receptor signaling components. Journal of Biological Chemistry 276(9):6280
Das, U. N. (2003). "Current advances in sepsis and septic shock with particular emphasis on the role of insulin." Med Sci Monit 9(8): RA181-92.
Dawson, T. C., W. A. Kuziel, et al. (1999). "Absence of CC chemokine receptor-2 reduces atherosclerosis in apolipoprotein E-deficient mice." Atherosclerosis 143(1): 205-11.
Dichtl, W., J. Dulak, et al. (2003). "HMG-CoA reductase inhibitors regulate inflammatory transcription factors in human endothelial and vascular smooth muscle cells." Arterioscler Thromb Vasc Biol 23(1): 58-63.
Diomede L, A. D., Sottocorno M, Polentarutti N, Donati MB, Bianchi M, Fruscella P, Salmona M (2001). "The in vivo anti-inflammatory effect of statins is mediated by nonsterol mevalonate products." Arterioscler Thromb Vasc Biol 21: 1327-1332.
End, D. W., G. Smets, et al. (2001). "Characterization of the antitumor effects of the selective farnesyl protein transferase inhibitor R115777 in vivo and in vitro." Cancer Res 61(1): 131-7.
Gu, L., Y. Okada, et al. (1998). "Absence of monocyte chemoattractant protein-1 reduces atherosclerosis in low density lipoprotein receptor-deficient mice." Mol Cell 2(2): 275-81.
Guijarro C, B.-C. L., Ortego M, Alonso C, Ortiz A, Plaza JJ, Diaz C, Hernandez G, Edigo J. (1998). "3-Hydroxy-3-methylglutaryl coenzyme A reductase and isoprenylation inhibitors induce apoptosis of vascular smooth muscle cells in culture." Circ Res 83: 490-500.
Haddy, N., C. Sass, et al. (2003). "IL-6, TNF-alpha and atherosclerosis risk indicators in a healthy family population: the STANISLAS cohort." Atherosclerosis 170(2): 277-83.
Head, J. E. and S. R. Johnston (2003). "Protein farnesyltransferase inhibitors." Expert Opin Emerg Drugs 8(1): 163-78.
Ito, T., U. Ikeda, et al. (2002). "HMG-CoA reductase inhibitors reduce interleukin-6 synthesis in human vascular smooth muscle cells." Cardiovasc Drugs Ther 16(2): 121-6.
Karp, J. E., J. E. Lancet, et al. (2001). "Clinical and biologic activity of the farnesyltransferase inhibitor R115777 in adults with refractory and relapsed acute leukemias: a phase 1 clinical-laboratory correlative trial." Blood 97(11): 3361-9.
Kirii, H., T. Niwa, et al. (2003). "Lack of interleukin-1beta decreases the severity of atherosclerosis in ApoE-deficient mice." Arterioscler Thromb Vasc Biol 23(4): 656-60.
Kurzrock, R., H. M. Kantarjian, et al. (2003). "Farnesyltransferase inhibitor R115777 in myelodysplastic syndrome: clinical and biologic activities in the phase 1 setting." Blood 102(13): 4527-34.
Lalu, M. M., C. Q. Gao, et al. (2003). "Matrix metalloproteinase inhibitors attenuate endotoxemia induced cardiac dysfunction: a potential role for MMP-9." Mol Cell Biochem 251(1-2): 61-6.
Lancet, J.E., Karp, J.E. (2003). "Farnesyltransferase inhibitors in hematologic malignancies: new horizons in therapy." Blood 102(12) 3880-9)
Leung, B. P., N. Sattar, et al. (2003). "A novel anti-inflammatory role for simvastatin in inflammatory arthritis." Journal of Immunology 170(3): 1524-1530.
Liu L, M. P., Kovach NL, Bailey R, Hamilton AD, Sebti SM, Harlan JM (1999). "Integrin-dependent leukocyte adhesion involves geranylgeranylated protein(s)." J Biol Chem 274: 33334-33340.
Luo, J. L., S. Maeda, et al. (2004). "Inhibition of NF-kappaB in cancer cells converts inflammation- induced tumor growth mediated by TNFalpha to TRAIL-mediated tumor regression." Cancer Cell 6(3): 297-305.
Luttun, A., E. Lutgens, et al. (2004). "Loss of matrix metalloproteinase-9 or matrix metalloproteinase-12 protects apolipoprotein E-deficient mice against atherosclerotic media destruction but differentially affects plaque growth." Circulation 109(11): 1408-14.
Mancuso, G., A. Midiri, et al. (2004). "Dual role of TLR2 and myeloid differentiation factor 88 in a mouse model of invasive group B streptococcal disease." J Immunol 172(10): 6324-9.
McCarey DW, M. I., Madhok R, Hampson R, Scherbakov O, Ford I, Capell HA, Sattar N (2004). "Trial of Atorvastatin in Rheumatoid Arthritis (TARA): double-blind, randomised placebo-controlled trial." Lancet 363(9426): 2015-21.
McKay A, L. B., McInnes IB, Thomson NC, Liew FY. (2004). "A novel anti-inflammatory role of simvastatin in a murine model of allergic asthma." J Immunol. 172(5): 2903-8.
Michelsen, K. S., M. H. Wong, et al. (2004). "Lack of Toll-like receptor 4 or myeloid differentiation factor 88 reduces atherosclerosis and alters plaque phenotype in mice deficient in apolipoprotein E." Proceedings of the National Academy of Sciences of the United States of America 101(29): 10679-10684.
Montero, M. T., O. Hernandez, et al. (2000). "Hydroxymethylglutaryl-coenzyme A reductase inhibition stimulates caspase-1 activity and Th1-cytokine release in peripheral blood mononuclear cells." Atherosclerosis (Shannon, Ireland) 153(2): 303-313.
Na, H. J., S. J. Lee, et al. (2004). "Inhibition of farnesyltransferase prevents collagen-induced arthritis by down-regulation of inflammatory gene expression through suppression of p21(ras)-dependent NF-kappaB activation." J Immunol 173(2): 1276-83.
Ortego M, B. C., Hernandez-Presa MA, Tunon J, Diaz C, Hernandez G, Egido J (1999). "Atorvastatin reduces NF-kappaB activation and chemokine expression in vascular smooth muscle cells and mononuclear cells." Atherosclerosis 147(2): 253-61.
Peterson KS, H. J., Zhu J, D'Agati V, Liu X, Miller N, Erlander MG, Jackson MR, Winchester RJ (2004). "Characterization of heterogeneity in the molecular pathogenesis of lupus nephritis from transcriptional profiles of laser-captured glomeruli." J Clin Invest 113(12): 1722-33.
Pikarsky, E., R. M. Porat, et al. (2004). "NF-kappaB functions as a tumour promoter in inflammation-associated cancer." Nature 431(7007): 461-6.
Prendergast, G. C. and N. Rane (2001). "Farnesyltransferase inhibitors: mechanism and applications." Expert Opin Investig Drugs 10(12): 2105-16.
Rezaie-Majd, A., T. Maca, et al. (2002). "Simvastatin reduces expression of cytokines interleukin-6, interleukin-8, and monocyte chemoattractant protein-1 in circulating monocytes from hypercholesterolemic patients." Arterioscler Thromb Vasc Biol 22(7): 1194-9.
Schonbeck U, L. P. S. U., Libby P (2004). "Inflammation, immunity, and HMG-CoA reductase inhibitors: statins as antiinflammatory agents?" Circulation 109(21 Suppl 1): II18-26.
Schumann, R. R., C. Belka, et al. (1998). "Lipopolysaccharide activates caspase-1 (interleukin-1-converting enzyme) in cultured monocytic and endothelial cells." Blood 91(2): 577-84.
Shaw KJ, M. N., Liu X, Lerner D, Wan J, Bittner A, Morrow BJ (2003). "Comparison of the changes in global gene expression of Escherichia coli induced by four bactericidal agents." J Mol Microbiol Biotechnol 5(2): 105-22.
Stanislaus, R., K. Pahan, et al. (1999). "Amelioration of experimental allergic encephalomyelitis in Lewis rats by lovastatin." Neurosci Lett 269(2): 71-4.
Takada, Y., F. R. Khuri, et al. (2004). "Protein farnesyltransferase inhibitor (SCH 66336) abolishes NF-kappaB activation induced by various carcinogens and inflammatory stimuli leading to suppression of NF-kappaB-regulated gene expression and up-regulation of apoptosis." J Biol Chem 279(25): 26287-99.
Takeda, S. A. K. (2004). Toll-like receptor signalling. Nature Reviews Immunology 4:499.Waehre T, D. J., Gullestad L, Holm AM, Pedersen TR, Arnesen KE, Torsvik H, Froland SS, Semb AG, Aukrust P (2003). "Hydroxymethylglutaryl coenzyme a reductase inhibitors down-regulate chemokines and chemokine receptors in patients with coronary artery disease." J Am Coll Cardiol 41(9): 1460-7.
Weighardt, H., S. Kaiser-Moore, et al. (2002). "Cutting edge: myeloid differentiation factor 88 deficiency improves resistance against sepsis caused by polymicrobial infection." J Immunol 169(6): 2823-7.
Wong, B., W. C. Lumma, et al. (2001). "Statins suppress THP-1 cell migration and secretion of matrix metalloproteinase 9 by inhibiting geranylgeranylation." J Leukoc Biol 69(6): 959-62.
Xu, H. A., Huazhang; Yu, Yizhi; Zhang, Minghui; Qi, Runzi; Cao, Xuetao. (2003) Ras Participates in CpG Oligodeoxynucleotide Signaling through Association with Toll-like Receptor 9 and Promotion of Interleukin-1 Receptor-associated Kinase/Tumor Necrosis Factor Receptor-associated Factor 6 Complex Formation in Macrophages. Journal of Biological Chemistry 278(38).
Youssef, S., O. Stueve, et al. (2002). "The HMG-CoA reductase inhibitor, atorvastatin, promotes a Th2 bias and reverses paralysis in central nervous system autoimmune disease." Nature (London, United Kingdom) 420(6911): 78-84.
Zhang FL, C. P. (1996). "Protein prenylation: molecular mechanisms and functional consequences." Annu Rev Biochem 65: 241-269.
Zhong, W-B, Wang, C. Y.Chang, T. C. and Lee, W. S. (2003). "Lovastatin Induces Apoptosis of Anaplastic Thyroid Cancer Cells via Inhibition of Protein Geranylgeranylation and de Novo Protein Synthesis." Endocrinology 144(9): 3852-3859.

## Claims

1. A method for determining a response of a subject suffering from sepsis or septic shock to treatment with a farnesyltransferase inhibitor, comprising the steps:
(i) measuring a level of at least one marker selected from the group consisting of the proteins identified in Table 4 and of the transcripts of the genes identified in Tables 2 and 3 in an untreated blood sample obtained from the subject before treatment with the farnesyltransferase inhibitor, and
(ii) measuring a level of the marker in a treated blood sample obtained from the subject at least one time after treatment with the farnesyltransferase inhibitor has commenced, and
(iii) comparing said levels of the marker, where a decrease from the level of the marker measured in the untreated blood sample to the level of the marker measured in the treated blood sample indicates a response to the treatment.

2. The method of claim 1 wherein the method comprises the steps:
(a) (i) isolating cells or plasma from the untreated blood sample to obtain an untreated sample extract, and (ii) isolating cells or plasma from each treated blood sample to obtain a treated sample extract;
(b) (i) measuring a level of at least one of IL-6, MCP-1, IL-1β, MMP-9, and TNF-α in the untreated sample extract, and (ii) measuring a level of said at least one of IL-6, MCP-1, IL-1β, MMP-9, and TNF-α in each treated sample extract, and (iii) comparing said levels of the at least one of IL-6, MCP-1, IL-1β, MMP-9, MIP-1α and TNF-α, where a decrease from the level of the at least one of IL-6, MCP-1, IL-1β, MMP-9, MIP-1α and TNF-α measured in the untreated sample extract to the level of the at least one of IL-6, MCP-1, IL-1β, MMP-9, MIP-1α and TNF-α measured in the treated sample extract indicates a response to the treatment.

3. The method of claim 1, wherein the method comprises the steps:
(a) (i) isolating peripheral blood mononuclear cells from the untreated blood sample to obtain untreated PBMC sample, and (ii) isolating peripheral blood mononuclear cells from each treated blood sample to obtain treated PBMC sample;
(b) (i) stimulating the untreated PBMC sample with lipopolysaccharide to obtain an LPS-stimulated untreated PBMC sample, and (ii) stimulating each treated PBMC sample with lipopolysaccharide to obtain an LPS-stimulated treated PBMC sample;
(c) (i) isolating RNA of the untreated LPS-stimulated PBMC sample, and (ii) isolating RNA of each treated LPS-stimulated PBMC sample;
(d) (i) measuring expression of at least one of IL-1β, MCP-1, MMP-9, MyD88, STAT1, and IL-6 in the RNA of the untreated LPS-stimulated PBMC sample, and (ii) measuring expression of said at least one of IL-1β, MCP-1, MMP-9, MyD88, STAT1, and IL-6 in the RNA of each treated LPS-stimulated PBMC sample, and (iii) comparing said expression measurements of the at least one of IL-1β, MCP-1, MMP-9, MyD88, STAT1, and IL-6, where a decrease indicates a response to the treatment.

4. A method as defined in any one of claims 1, 2, or 3, wherein the farnesyltransferase inhibitor comprises a compound of formula (I): 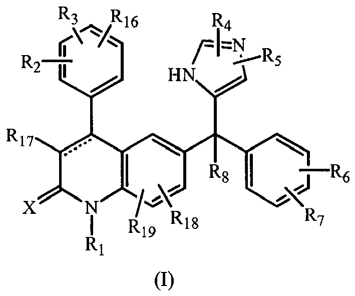 a stereoisomeric form thereof, a pharmaceutically acceptable acid or base addition salt thereof, wherein
the dotted line represents an optional bond;
X is oxygen or sulfur;
R¹ is hydrogen, C₁₋₁₂alkyl, Ar¹, Ar²C₁₋₆alkyl, quinolinylC₁₋₆alkyl, pyridylC₁₋₆alkyl, hydroxyC₁₋₆alkyl, C₁₋₆alkyloxyC₁₋₆alkyl, mono- or di(C₁₋₆alkyl)aminoC₁₋₆alkyl, aminoC₁₋₆alkyl, or a radical of formula -Alk¹-C(=O)-R⁹, -Alk¹-S(O)-R⁹ or -Alk¹-S(O)₂-R⁹, wherein Alk¹ is C₁₋₆alkanediyl, R⁹ is hydroxy, C₁₋₆alkyl, C₁₋₆alkyloxy, amino, C₁₋₈alkylamino or C₁₋₈alkylamino substituted with C₁₋₆alkyloxycarbonyl;
R², R³ and R¹⁶ each independently are hydrogen, hydroxy, halo, cyano, C₁₋₆alkyl, C₁₋₆alkyloxy, hydroxyC₁₋₆alkyloxy, C₁₋₆alkyloxyC₁₋₆alkyloxy, amino-C₁₋₆alkyloxy, mono- or di(C₁₋₆alkyl)aminoC₁₋₆alkyloxy, Ar¹, Ar²C₁₋₆alkyl, Ar²oxy, Ar²C₁₋₆alkyloxy, hydroxycarbonyl, C₁₋₆alkyloxycarbonyl, trihalomethyl, trihalomethoxy, C₂₋₆alkenyl, 4,4-dimethyloxazolyl; or
when on adjacent positions R² and R³ taken together may form a bivalent radical of formula
-O-CH₂-O- (a-1),
-O-CH₂-CH₂-O- (a-2),
-O-CH=CH- (a-3),
-O-CH₂-CH₂- (a-4),
-O-CH₂-CH₂-CH₂- (a-5),
or
-CH=CH-CH=CH- (a-6);
R⁴ and R⁵ each independently are hydrogen, halo, Ar¹, C₁₋₆alkyl, hydroxy-C₁₋₆alkyl, C₁₋₆alkyloxyC₁₋₆alkyl, C₁₋₆alkyloxy, C₁₋₆alkylthio, amino, hydroxycarbonyl, C₁₋₆alkyloxycarbonyl, C₁₋₆alkylS(O)C₁₋₆alkyl or C₁₋₆alkylS(O)₂C₁₋₆alkyl;
R⁶ and R⁷ each independently are hydrogen, halo, cyano, C₁₋₆alkyl, C₁₋₆alkyloxy, Ar²oxy, trihalomethyl, C₁₋₆alkylthio, di(C₁₋₆alkyl)amino, or when on adjacent positions R⁶ and R⁷ taken together may form a bivalent radical of formula
-O-CH₂-O- (c-1),
or
-CH=CH-CH=CH- (c-2);
R⁸ is hydrogen, C₁₋₆alkyl, cyano, hydroxycarbonyl, C₁₋₆alkyloxycarbonyl, C₁₋₆alkylcarbonylC₁₋₆alkyl, cyanoC₁₋₆alkyl, C₁₋₆alkyloxycarbonylC₁₋₆alkyl, carboxyC₁₋₆alkyl, hydroxyC₁₋₆alkyl, aminoC₁₋₆alkyl, mono- or di(C₁₋₆alkyl)-aminoC₁₋₆alkyl, imidazolyl, haloC₁₋₆alkyl, C₁₋₆alkyloxyC₁₋₆alkyl, aminocarbonylC₁₋₆alkyl, or a radical of formula
-O-R¹⁰ (b-1),
-S-R¹⁰ (b-2),
-N-R¹¹R¹² (b-3),
wherein R¹⁰ is hydrogen, C₁₋₆alkyl, C₁₋₆alkylcarbonyl, Ar¹, Ar²C₁₋₆alkyl, C₁₋₆alkyloxycarbonylC₁₋₆alkyl, a radical or formula -Alk²-OR¹³ or -Alk²-NR¹⁴R¹⁵;
R¹¹ is hydrogen, C₁₋₁₂alkyl, Ar¹ or Ar²C₁₋₆alkyl;
R¹² is hydrogen, C₁₋₆alkyl, C₁₋₁₆alkylcarbonyl, C₁₋₆alkyloxycarbonyl, C₁₋₆alkylaminocarbonyl, Ar¹, Ar²C₁₋₆alkyl, C₁₋₆alkylcarbonylC₁₋₆alkyl, a natural amino acid, Ar¹carbonyl, Ar²C₁₋₆alkylcarbonyl, aminocarbonylcarbonyl, C₁₋₆alkyloxyC₁₋₆alkylcarbonyl, hydroxy, C₁₋₆alkyloxy, aminocarbonyl, di(C₁₋₆alkyl)aminoC₁₋₆ alkylcarbonyl, amino, C₁₋₆alkylamino, C₁₋₆alkylcarbonylamino, or a radical of formula -Alk²-OR¹³ or -Alk₂-NR¹⁴R¹⁵; wherein Alk² is C₁₋₆alkanediyl; R¹³ is hydrogen, C₁₋₆alkyl, C₁₋₆alkylcarbonyl, hydroxyC₁₋₆alkyl, Ar¹ or Ar²C₁₋₆alkyl; R¹⁴ is hydrogen, C₁₋₆alkyl, Ar¹ or Ar²C₁₋₆alkyl; R¹⁵ is hydrogen, C₁₋₆alkyl, C₁₋₆alkylcarbonyl, Ar¹ or Ar²C₁₋₆alkyl;
R¹⁷ is hydrogen, halo, cyano, C₁₋₆alkyl, C₁₋₆alkyloxycarbonyl, Ar¹;
R¹⁸ is hydrogen, C₁₋₆alkyl, C₁₋₆alkyloxy or halo;
R¹⁹ is hydrogen or C₁₋₆alkyl;
Ar¹ is phenyl or phenyl substituted with C₁₋₆alkyl, hydroxy, amino, C₁₋₆alkyloxy or halo; and
Ar² is phenyl or phenyl substituted with C₁₋₆alkyl, hydroxy, amino, C₁₋₆alkyloxy or halo.

5. The method of claim 4, wherein said farnesyltransferase inhibitor is:
(a) a compound of formula (I) wherein X is oxygen and the dotted line represents a bond; or
(b) a compound of formula (I) wherein R¹ is hydrogen, C₁₋₆alkyl, C₁₋₆alkyloxy-C₁₋₆alkyl or, mono- or di(C₁₋₆alkyl)aminoC₁₋₆alkyl; R² is halo, C₁₋₆alkyl, C₂₋₆alkenyl, C₁₋₆alkyloxy, trihalomethoxy, or hydroxyC₁₋₆alkyloxy; and R³ is hydrogen; or
(c) (+)-6-[amino(4-chlorophenyl)(1-methyl-1*H*-imidazol-5-yl)methyl]-4-(3-chlorophenyl)-1-methyl-2(1*H*)-quinolinone; or a pharmaceutically acceptable acid addition salt thereof.

6. The method of claim 4, wherein R⁸ is hydrogen, hydroxy, haloC₁₋₆alkyl, hydroxyC₁₋₆alkyl, cyanoC₁₋₆alkyl, C₁₋₆alkyloxycarbonylC₁₋₆alkyl, imidazolyl, or a radical of formula NR¹¹R¹² wherein R¹¹ is hydrogen or C₁₋₁₂alkyl and R¹² is hydrogen, C₁₋₆alkyl, C₁₋₆alkyloxy, C₁₋₆alkyloxyC₁₋₆alkylcarbonyl, hydroxy, or a radical of formula -Alk²-OR¹³ wherein R¹³ is hydrogen or C₁₋₆alkyl.

7. A method as defined in claim 1, said at least one marker is selected from the group consisting ofIL-6, MCP-1, IL-1β, MMP-9, and TNF-α.

8. A method as defined in any one of claims 1 or 2, wherein in substep (ii), plural treated blood samples from the subject are used which were taken over periodic intervals of time after treatment with the farnesyltransferase inhibitor has commenced.

9. A method as defined in claim 1, wherein the blood sample is whole blood, plasma, serum, total cells, or peripheral blood mononuclear cells.

10. A method as defined in claim 2, wherein substeps (b)(i) and (b)(ii) comprise: (i) isolating plasma from the untreated blood sample by centrifugation to obtain the untreated sample extract, and (ii) isolating plasma from each treated blood sample by centrifugation to obtain the treated sample extract.

11. A method as defined in claim 2, wherein substeps (b)(i) and (b)(ii) comprise: (i) isolating peripheral blood mononuclear cells from the untreated blood sample by Ficoll-Paque gradient separation to obtain the untreated sample extract, and (ii) isolating peripheral blood mononuclear cells by Ficoll-Paque gradient separation from each treated blood sample to obtain the treated sample extract.

12. A method as defined in claim 2, further comprising: (i) stimulating the untreated sample extract with lipopolysaccharide, and (ii) stimulating the treated sample extract with lipopolysaccharide.

13. A method as defined in claim 3, wherein substeps (b)(i) and (b)(ii) each comprises isolating the peripheral blood mononuclear cells using Ficoll-Paque gradient separation.

14. A method as defined in claim 13, wherein the subject is a patient in a clinical trial.

15. A method as defined in claim 1 or 14, wherein the farnesyltransferase inhibitor used to treat the subject is tipifarnib.

## Patentansprüche

1. Verfahren zur Bestimmung des Ansprechens eines an Sepsis oder septischem Schock leidenden Patienten auf eine Behandlung mit einem Farnesyltransferaseinhibitor, welches die folgenden Schritte umfasst:
(i) Messen einer Konzentration mindestens eines aus der in Tabelle 4 aufgeführten Proteine bestehenden Gruppe ausgewählten Markers und der Transskripte der in den Tabellen 2 und 3 aufgeführten Gene in einer von dem Patienten vor der Behandlung mit dem Farnesyltrans-feraseinhibitor entnommenen unbehandelten Blutprobe und
(ii) Messen einer Konzentration des Markers in einer behandelten Blutprobe, die von dem Patienten zumindest zu einem Zeitpunkt nach Beginn der Behandlung mit dem Farnesyltransferaseinhibitor entnommen wurde, und
(iii) Vergleich der Konzentrationen des Markers, wobei eine Abnahme der in der unbehandelten Blutprobe gemessenen Konzentration des Markers zur in der behandelten Blutprobe gemessenen Konzentration des Markers ein Ansprechen auf die Behandlung anzeigt.

2. Verfahren nach Anspruch 1, wobei das Verfahren die folgenden Schritte umfasst:
(a) (i) Isolieren von Zellen oder Plasma aus der unbehandelten Blutprobe zur Gewinnung eines unbehandelten Probenextrakts und (ii) Isolieren von Zellen oder Plasma aus den einzelnen behandelten Blutproben zur Gewinnung eines behandelten Probenextrakts;
(b) (i) Messen einer Konzentration von mindestens einem von IL-6, MCP-1, IL-1β, MMP-9 und TNF-α in dem unbehandelten Probenextrakt und (ii) Messen einer Konzentration von mindestens einem von IL-6, MCP-1, IL-1β, MMP-9 und TNF-α in den einzelnen behandelten Probenextrakten und (iii) Vergleichen der Konzentrationen von mindestens einem von IL-6, MCP-1, IL-1β, MMP-9, MIP-1α und TNF-α, wobei eine Abnahme der Konzentration von mindestens einem von IL-6, MCP-1, IL-1β, MMP-9, MIP-1α und TNF-α, gemessen im unbehandelten Probenextrakt, zur Konzentration von mindestens einem von IL-6, MCP-1, IL-1β, MMP-9, MIP-1α und TNF-α, gemessen in dem behandelten Probenextrakt, ein Ansprechen auf die Behandlung anzeigt.

3. Verfahren nach Anspruch 1, wobei das Verfahren die folgenden Schritte umfasst:
(a) (i) Isolieren von mononukleären Zellen des peripheren Blutes (Peripheral Blood Mononuclear Cells) aus der unbehandelten Blutprobe zur Gewinnung einer unbehandelten PBMC-Probe und (ii) Isolieren von mononukleären Zellen des peripheren Blutes aus den einzelnen behandelten Blutproben zur Gewinnung einer behandelten PBMC-Probe;
(b) (i) Stimulieren der unbehandelten PBMC-Probe mit Lipopolysaccharid zur Gewinnung einer LPS-stimulierten unbehandelten PBMC-Probe und (ii) Stimulieren der einzelnen behandelten PBMC-Proben mit Lipopolysaccharid zur Gewinnung einer LPS-stimulierten behandelten PBMC-Probe;
(c) (i) Isolieren von RNA der unbehandelten LPS-stimulierten PBMC-Probe und (ii) Isolieren von RNA von den einzelnen behandelten LPS-stimulierten PBMC-Proben;
(d) (i) Messen der Expression von mindestens einem von IL-1β, MCP-1, MMP-9, MyD88, STAT1 und IL-6 in der RNA der unbehandelten LPS-stimulierten PBMC-Probe und (ii) Messen der Expression von mindestens einem von IL-1β, MCP-1, MMP-9, MyD88, STAT1 und IL-6 in der RNA der einzelnen behandelten LPS-stimulierten PBMC-Proben und (iii) Vergleich der Expressionsmessungen von mindestens einem von IL-1β, MCP-1, MMP-9, MyD88, STAT1 und IL-6, wobei eine Abnahme ein Ansprechen auf die Behandlung anzeigt.

4. Verfahren nach einem der Ansprüche 1, 2 oder 3, wobei der Farnesyltransferaseinhibitor eine Verbindung der Formel (I): 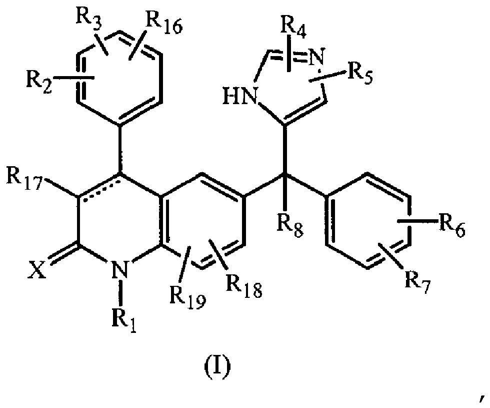 eine stereoisomere Form davon oder ein pharmazeutisch unbedenkliches Säure- oder Basen-additionssalz davon umfasst, wobei
die gestrichelte Linie für eine gegebenenfalls vorhandene Bindung steht;
X für Sauerstoff oder Schwefel steht;
R¹ für Wasserstoff, C₁₋₁₂-Alkyl, Ar¹, Ar²-C₁₋₆-Alkyl, Chinolinyl-C₁₋₆-alkl, Pyridyl-C₁₋₆-alkyl, Hydroxy-C₁₋₆-alkyl, C₁₋₆-Alkyloxy-C₁₋₆-alkyl, Mono- oder Di-(C₁₋₆-alkyl)amino-C₁₋₆-alkyl, Amino-C₁₋₆-alkyl oder einen Rest der Formel -Alk¹-C(=O)-R⁹, -Alk¹-S(O)-R⁹ oder -Alk¹-S(O)₂-R⁹ steht, wobei Alk¹ für C₁₋₆-Alkandiyl steht und R⁹ für Hydroxy, C₁₋₆-Alkyl, C₁₋₆-Alkyloxy, Amino, C₁₋₈-Alkylamino oder durch C₁₋₆-Alkyloxycarbonyl substituiertes C₁₋₈-Alkylamino steht;
R², R³ und R¹⁶ jeweils unabhängig voneinander für Wasserstoff, Hydroxy, Halogen, Cyano, C₁₋₆-Alkyl, C₁₋₆-Alkyloxy, Hydroxy-C₁₋₆-alkyloxy, C₁₋₆-Alkyloxy-C₁₋₆-alkyloxy, Amino-C₁₋₆-alkyloxy, Mono- oder Di-(C₁₋₆-alkyl)amino-C₁₋₆-alkyloxy, Ar¹, Ar²-C₁₋₆-Alkyl, Ar²-Oxy, Ar²-C₁₋₆-Alkyloxy, Hydroxycarbonyl, C₁₋₆-Alkyloxycarbonyl, Trihalogenmethyl, Trihalogenmethoxy, C₂₋₆-Alkenyl, 4,4-Dimethyloxazolyl stehen; oder,
wenn R² und R³ an benachbarten Positionen stehen, sie zusammen einen zweiwertigen Rest der Formel
-O-CH₂-O- (a-1),
-O-CH₂-CH₂-O- (a-2),
-O-CH=CH- (a-3),
-O-CH₂-CH₂- (a-4),
-O-CH₂-CH₂-CH₂ (a-5)
oder
-CH=CH-CH=CH- (a-6)
bilden können;
R⁴ und R⁵ jeweils unabhängig voneinander für Wasserstoff, Halogen, Ar¹, C₁₋₆-Alkyl, Hydroxy-C₁₋₆-alkyl, C₁₋₆-Alkyloxy-C₁₋₆-alkyl, C₁₋₆-Alkyloxy, C₁₋₆-Alkylthio, Amino, Hydroxycarbonyl, C₁₋₆-Alkyloxycarbonyl, C₁₋₆-Alkyl-S(O)-C₁₋₆-alkyl oder C₁₋₆-Alkyl-S(O)₂-C₁₋₆-alkyl stehen;
R⁶ und R⁷ jeweils unabhängig voneinander für Wasserstoff, Halogen, Cyano, C₁₋₆-Alkyl, C₁₋₆-Alkyloxy, Ar²-Oxy, Trihalogenmethyl, C₁₋₆-Alkylthio, Di-(C₁₋₆-alkyl) amino stehen oder, wenn R⁶ und R⁷ an benachbarten Positionen stehen, sie zusammen einen zweiwertigen Rest der Formel
-O-CH₂-O- (c-1)
oder
-CH=CH-CH=CH- (c-2)
bilden können;
R⁸ für Wasserstoff, C₁₋₆-Alkyl, Cyano, Hydroxycarbonyl, C₁₋₆-Alkyloxycarbonyl, C₁₋₆-Alkylcarbonyl-C₁₋₆-alkyl, Cyano-C₁₋₆-alkyl, C₁₋₆-Alkyloxycarbonyl-C₁₋₆-alkyl, Carboxy-C₁₋₆-alkyl, Hydroxy-C₁₋₆-alkyl, Amino-C₁₋₆-alkyl, Mono- oder Di-(C₁₋₆-alkyl)amino-C₁₋₆-alkyl, Imidazolyl, Halogen-C₁₋₆-alkyl, C₁₋₆-Alkyloxy-C₁₋₆-alkyl, Aminocarbonyl-C₁₋₆-alkyl oder einen Rest der Formel
-O-R¹⁰ (b-1),
-S-R¹⁰ (b-2),
-N-R¹¹R¹² (b-3)
steht,
wobei R¹⁰ für Wasserstoff, C₁₋₆-Alkyl, C₁₋₆-Alkylcarbonyl, Ar¹, Ar²-C₁₋₆-Alkyl, C₁₋₆-Alkyloxycarbonyl-C₁₋₆-alkyl, einen Rest oder Formel -Alk²-OR¹³ oder -Alk²-NR¹⁴R¹⁵ steht;
R¹₁ für Wasserstoff, C₁₋₁₂-Alkyl, Ar¹ oder Ar²-C₁₋₆-Alkyl steht;
R¹² für Wasserstoff, C₁₋₆-Alkyl, C₁₋₁₆-Alkylcarbonyl, C₁₋₆-Alkyloxycarbonyl, C₁₋₆-Alkylaminocarbonyl, Ar¹, Ar²-C₁₋₆-Alkyl, C₁₋₆-Alkylcarbonyl-C₁₋₆-alkyl, eine natürliche Aminosäure, Ar¹-Carbonyl, Ar²-C₁₋₆-Alkylcarbonyl, Aminocarbonylcarbonyl, C₁₋₆-Alkyloxy-C₁₋₆-alkylcarbonyl, Hydroxy, C₁₋₆-Alkyloxy, Aminocarbonyl, Di-(C₁₋₆-alkyl)amino-C₁₋₆-alkylcarbonyl, Amino, C₁₋₆-Alkylamino, C₁₋₆-Alkylcarbonylamino oder einen Rest der Formel -Alk²-OR¹³ oder -Alk²NR¹⁴R¹⁵ steht;
wobei Alk² für C₁₋₆-Alkandiyl steht; R¹³ für Wasserstoff, C₁₋₆-Alkyl, C₁₋₆-Alkylcarbonyl, Hydroxy-C₁₋₆-alkyl, Ar¹ oder Ar²-C₁₋₆-Alkyl steht; R¹⁴ für Wasserstoff, C₁₋₆-Alkyl, Ar¹ oder Ar²-C₁₋₆-Alkyl steht; R¹⁵ für Wasserstoff, C₁₋₆-Alkyl, C₁₋₆-Alkylcarbonyl, Ar¹ oder Ar²-C₁₋₆-Alkyl steht;
R¹⁷ für Wasserstoff, Halogen, Cyano, C₁₋₆-Alkyl, C₁₋₆-Alkyloxycarbonyl oder Ar¹ steht;
R¹⁸ für Wasserstoff, C₁₋₆-Alkyl, C₁₋₆-Alkyloxy oder Halogen steht;
R¹⁹ für Wasserstoff oder C₁₋₆-Alkyl steht;
Ar¹ für Phenyl oder durch C₁₋₆-Alkyl, Hydroxy, Amino, C₁₋₆-Alkyloxy oder Halogen substituiertes Phenyl steht und
Ar² für Phenyl oder durch C₁₋₆-Alkyl, Hydroxy, Amino, C₁₋₆-Alkyloxy oder Halogen substituiertes Phenyl steht.

5. Verfahren nach Anspruch 4, wobei es sich bei dem Farnesyltransferaseinhibitor um:
(a) eine Verbindung der Formel (I), in welcher X für Sauerstoff steht und die gestrichelte Linie für eine Bindung steht; oder
(b) eine Verbindung der Formel (I), in welcher R¹ für Wasserstoff, C₁₋₆-Alkyl, C₁₋₆-Alkyloxy-C₁₋₆-alkyl oder Mono- oder Di-(C₁₋₆-alkyl)amino-C₁₋₆-alkyl steht; R² für Halogen, C₁₋₆-Alkyl, C₂₋₆-Alkenyl, C₁₋₆-Alkyloxy, Trihalogenmethoxy oder Hydroxy-C₁₋₆-alkyloxy steht und R³ für Wasserstoff steht; oder
(c) (+) -6- [Amino (4-chlorphenyl) (1-methyl-1H-imidazol-5-yl)methyl]-4-(3-chlorphenyl)-1-methyl-2(1H)-chinolinon oder ein pharmazeutisch unbedenkliches Säureadditionssalz davon
handelt.

6. Verfahren nach Anspruch 4, wobei R⁸ für Wassersoff, Hydroxy, Halogen-C₁₋₆-alkyl, Hydroxy-C₁₋₆-alkyl, Cyano-C₁-₆-alkyl, C₁₋₆-Alkyloxycarbonyl-C₁₋₆-alkyl, Imidazolyl oder einen Rest der Formel -NR¹¹R¹² steht, wobei R¹¹ für Wasserstoff oder C₁₋₁₂-Alkyl steht und R¹² für Wasserstoff, C₁₋₆-Alkyl, C₁₋₆-Alkyloxy, C₁₋₆-Alkyloxy-C₁₋₆-alkylcarbonyl, Hydroxy oder einen Rest der Formel -Alk²-OR¹³ steht, wobei R¹³ für Wasserstoff oder C₁₋₆-Alkyl steht.

7. Verfahren nach Anspruch 1, wobei der mindestens eine Marker ausgewählt ist aus der Gruppe bestehend aus IL-6, MCP-1, IL-1β, MMP-9 und TNF-α.

8. Verfahren nach einem der Ansprüche 1 oder 2, wobei im Unterschritt (ii) eine Mehrzahl von behandelten Blutproben des Patienten verwendet werden, die in periodischen Zeitabständen nach Beginn der Behandlung mit dem Farnesyltransferaseinhibitor entnommen wurden.

9. Verfahren nach Anspruch 1, wobei es sich bei der Blutprobe um Vollblut, Plasma, Serum, Gesamtzellen oder mononukleäre Zellen des peripheren Blutes handelt.

10. Verfahren nach Anspruch 2, wobei die Unterschritte (b)(i) und (b) (ii) Folgendes umfassen: (i) Isolieren von Plasma aus der unbehandelten Blutprobe durch Zentrifugieren zur Gewinnung des unbehandelten Probenextrakts und (ii) Isolieren von Plasma aus den einzelnen behandelten Blutproben durch Zentrifugieren zur Gewinnung des behandelten Probenextrakts.

11. Verfahren nach Anspruch 2, wobei die Unterschritte (b)(i) und (b) (ii) Folgendes umfassen: (i) Isolieren von mononukleären Zellen des peripheren Blutes aus der unbehandelten Blutprobe durch Ficoll-Paque-Gradiententrennung zur Gewinnung des unbehandelten Probenextrakts und (ii) Isolieren von mononukleären Zellen des peripheren Blutes durch Ficoll-Paque-Gradiententrennung aus den einzelnen behandelten Blutproben zur Gewinnung des behandelten Probenextrakts.

12. Verfahren nach Anspruch 2, welches weiterhin Folgendes umfasst: (i) Stimulieren des unbehandelten Probenextrakts mit Lipopolysaccharid und (ii) Stimulieren des behandelten Probenextrakts mit Lipopolysaccharid.

13. Verfahren nach Anspruch 3, wobei die Unterschritte (b)(i) und (b)(ii) jeweils das Isolieren von mononukleären Zellen des peripheren Blutes unter Anwendung einer Ficoll-Paque-Gradiententrennung umfassen.

14. Verfahren nach Anspruch 13, wobei es sich bei dem Patienten um einen Patienten in einem klinischen Versuch handelt.

15. Verfahren nach Anspruch 1 oder 14, wobei es sich bei dem zur Behandlung des Patienten verwendeten Farnesyltransferaseinhibitor um Tipifarnib handelt.

## Revendications

1. Procédé pour déterminer une réponse d'un sujet souffrant d'un état septique ou un choc septique à un traitement avec un inhibiteur de farnésyltransférase, comprenant les étapes de :
(i) mesure de taux d'au moins un marqueur choisi dans le groupe constitué des protéines identifiées dans le tableau 4 et des produits de transcription des gènes identifiés dans les tableaux 2 et 3 dans un échantillon de sang non traité obtenu à partir du sujet avant traitement avec l'inhibiteur de farnésyltransférase, et
(ii) mesure d'un taux du marqueur dans un échantillon de sang traité obtenu à partir du sujet au moins une fois une fois que le traitement avec l'inhibiteur de farnésyltransférase a commencé, et
(iii) comparaison desdits taux du marqueur, où une diminution du taux du marqueur mesuré dans l'échantillon de sang non traité au taux du marqueur mesuré dans l'échantillon de sang traité indique une réponse au traitement.

2. Procédé de la revendication 1 dans lequel le procédé comprend les étapes de :
(a) (i) isolement de cellules ou de plasma à partir de l'échantillon de sang non traité pour obtenir un extrait d'échantillon non traité, et (ii) isolement de cellules ou de plasma à partir de chaque échantillon de sang traité pour obtenir un extrait d'échantillon traité ;
(b) (i) mesure d'un taux d'au moins l'un de IL-6, MCP-1, IL-1β, MMP-9, et TNF-α dans l'extrait d'échantillon non traité, et (ii) mesure d'un taux dudit au moins l'un de IL-6, MCP-1, IL-1β, MMP-9, et TNF-α dans chaque extrait d'échantillon traité, et (iii) comparaison desdits taux de l'au moins un de IL-6, MCP-1, IL-1β, MMP-9, MIP-1α et TNF-α, où une diminution du taux de l'au moins un de IL-6, MCP-1, IL-1β, MMP-9, MIP-1α et TNF-α mesuré dans l'extrait d'échantillon non traité par rapport au taux de l'au moins un de IL-6, MCP-1, IL-1β, MMP-9, MIP-1α et TNF-α mesuré dans l'extrait d'échantillon traité indique une réponse au traitement.

3. Procédé de la revendication 1, le procédé comprenant les étapes de :
(a) (i) isolement de cellules mononucléaires périphériques (PBMC) à partir de l'échantillon de sang non traité pour obtenir un échantillon de PBMC non traité, et (ii) isolement de cellules mononucléaires périphériques à partir de chaque échantillon de sang traité pour obtenir un échantillon de PBMC traité ;
(b) (i) stimulation de l'échantillon de PBMC non traité avec un lipopolysaccharide pour obtenir un échantillon de PBMC non traité stimulé par LPS, et (ii) stimulation de chaque échantillon de PBMC traité avec un lipopolysaccharide pour obtenir un échantillon de PBMC traité stimulé par LPS ;
(c) (i) isolement d'ARN de l'échantillon de PBMC stimulé par LPS non traité, et (ii) isolement d'ARN de chaque échantillon de PBMC stimulé par LPS traité ;
(d) (i) mesure de l'expression d'au moins un de IL-1β, MCP-1, MMP-9, MyD88, STAT1, et IL-6 dans l'ARN de l'échantillon de PBMC stimulé par LPS non traité, et (ii) mesure de l'expression dudit au moins un de IL-1β, MCP-1, MMP-9, MyD88, STAT1, et IL-6 dans l'ARN de chaque échantillon de PBMC stimulé par LPS traité, et (iii) comparaison desdites mesures d'expression de l' au moins un de IL-1β, MCP-1, MMP-9, MyD88, STAT1, et IL-6, où une diminution indique une réponse au traitement.

4. Procédé tel que défini dans l'une quelconque des revendications 1, 2, ou 3, dans lequel l'inhibiteur de farnésyltransférase comprend un composé de formule (I) : 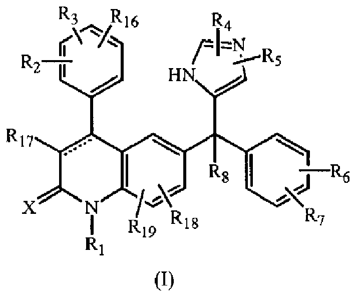 une forme de stéréo-isomère de celui-ci, un sel d'addition d'acide ou de base pharmaceutiquement acceptable de celui-ci, dans lequel
le trait pointillé représente une liaison facultative ; X est l'oxygène ou le soufre ;
R¹ est hydrogène, alkyle en C₁₋₁₂, Ar¹, Ar²- (alkyle en C₁₋₆), quinoléinyl (alkyle en C₁₋₆), pyridyl (alkyle en C₁₋₆), hydroxy- (alkyle en C₁₋₆), (alkyloxy en C₁₋₆) - (alkyle en C₁₋₆), mono- ou di (alkyle en C₁₋₆) amino-(alkyle en C₁₋₆), amino (alkyle en C₁₋₆), ou un radical de formule -Alk¹-C(=O)-R⁹, -Alk¹-S(O)-R⁹ ou -Alk¹-S(O)₂-R⁹, où Alk¹ est alcanediyle en C₁₋₆, R⁹ est hydroxy, alkyle en C₁₋₆, alkyloxy en C₁₋₆, amino, (alkyle en C₁₋₈) amino ou (alkyle en C₁₋₈) amino substitué par (alkyloxy en C₁₋₆) carbonyle;
R², R³ et R¹⁶ chacun indépendamment sont hydrogène, hydroxy, halogéno, cyano, alkyle en C₁₋₆, alkyloxy en C₁₋₆, hydroxy (alkyloxy en C₁₋₆), (alkyloxy en C₁₋₆)-(alkyloxy en C₁₋₆), amino(alkyloxy en C₁₋₆), mono- ou di (alkyle en C₁₋₆)-amino-(alkyloxy en C₁₋₆), Ar¹, Ar²- (alkyle en C₁₋₆), Ar²oxy, Ar²- (alkyloxy en C₁₋₆), hydroxycarbonyle, (alkyloxy en C₁₋₆)carbonyle, trihalogénométhyle, trihalogénométhoxy, alcényle en C₂₋₆, 4, 4-diméthyloxazolyle ; ou
lorsqu'ils sont sur des positions adjacentes, R² et R³ peuvent former un radical bivalent de formule
-O-CH₂-O- (a-1),
-O-CH₂-CH₂-O- (a-2),
-O-CH=CH- (a-3),
-O-CH₂-CH₂- (a-4),
-O-CH₂-CH₂-CH₂- (a-5),
ou
-CH=CH-CH=CH- (a-6) ;
R⁴ et R⁵ sont chacun indépendamment hydrogène, halogéno, Ar¹, alkyle, hydroxy-(alkyle en C₁₋₆), (alkyloxy en C₁₋₆)- (alkyle en C₁₋₆), alkyloxy en C₁₋₆, (alkyle en C₁₋₆)thio, amino, hydroxycarbonyle, (alkyloxy en C₁₋₆)carbonyle, (alkyle en C₁₋₆)-S(O)-(alkyle en C₁₋₆) ou (alkyle en C₁₋₆) -S (O)₂- (alkyle en C₁₋₆);
R⁶ et R⁷ chacun indépendamment sont hydrogène, halogéno, cyano, alkyle en C₁₋₆, alkyloxy en C₁₋₆, Ar² oxy, trihalogénométhyle, (alkyle en C₁₋₆)thio, di(alkyle en C₁₋₆) amino, ou lorsqu' ils sont sur des positions adjacentes, R⁶ et R⁷ peuvent former un radical bivalent de formule
-O-CH2-O- (C-1),
ou
-CH=CH-CH=CH- (C-2) ;
R⁸ est hydrogène, alkyle en C₁₋₆, cyano, hydroxycarbonyle, (alkyloxy en C₁₋₆)carbonyle, (alkyle en C₁₋₆)-carbonyl-(alkyle en C₁₋₆), cyano (alkyle en C₁₋₆), (alkyloxy en C₁₋₆)-carbonyl-(alkyle en C₁₋₆), carboxy-(alkyle en C₁₋₆), hydroxy- (alkyle en C₁₋₆), amino- (alkyle en C₁₋₆), mono- ou di (alkyle en C₁₋₆)-amino-(alkyle en C₁₋₆), imidazolyle, halogéno (alkyle en C₁₋₆), (alkyloxy en C₁₋₆)-(alkyle en C₁₋₆), aminocarbonyl- (alkyle en C₁₋₆), ou un radical de formule
-O-R¹⁰ (b-1),
-S-R¹⁰ (b-2),
-N-R¹¹R¹² (b-3),
où R¹⁰ est hydrogène, alkyle en C₁₋₆, (alkyle en C₁₋₆)carbonyle, Ar¹, Ar²- (alkyle en C₁₋₆), (alkyloxy en C₁₋₆)-carbonyl-(alkyle en C₁₋₆), un radical ou formule - Alk²-OR¹³ ou -Alk²-NR¹⁴R¹⁵ ;
R¹¹ est hydrogène, alkyle en C₁₋₁₂, Ar¹ ou Ar²- (alkyle en C₁₋₆) ;
R¹² est hydrogène, alkyle en C₁₋₆, (alkyle en C₁₋₁₆)carbonyle, (alkyloxy en C₁₋₆)carbonyle, (alkyle en C₁₋₆)aminocarbonyle, Ar¹, Ar²- (alkyle en C₁₋₆), (alkyle en C₁₋₆)-carbonyl-(alkyle en C₁₋₆), un acide aminé naturel, Ar¹-carbonyle, Ar²- (alkyle en C₁₋₆)carbonyle, aminocarbonylcarbonyle, (alkyloxy en C₁₋₆)-(alkyle en C₁₋₆)carbonyle, hydroxy, alkyloxy en C₁₋₆, aminocarbonyle, di (alkyle en C₁₋₆)-amino-(alkyle en C₁₋₆)carbonyle, amino, (alkyle en C₁₋₆)amino, (alkyle en C₁₋₆)carbonylamino, ou un radical de formule -Alk²-OR¹³ ou -Alk²-NR¹⁴R¹⁵ ; où Alk² est alcanediyle en C₁₋₆ ; R¹³ est hydrogène, alkyle en C₁₋₆, (alkyle en C₁₋₆)carbonyle, hydroxy (alkyle en C₁₋₆), Ar¹ ou Ar²- (alkyle en C₁₋₆) ; R¹⁴ est hydrogène, alkyle en C₁₋₆, Ar¹ ou Ar²- (alkyle en C₁₋₆) ; R¹⁵ est hydrogène, alkyle en C₁₋₆, (alkyle en C₁₋₆)carbonyle, Ar¹ ou Ar²- (alkyle en C₁₋₆) ;
R¹⁷ est hydrogène, halogéno, cyano, alkyle en C₁₋₆, (alkyloxy en C₁₋₆)carbonyle, Ar¹ ;
R¹⁸ est hydrogène, alkyle en C₁₋₆, alkyloxy en C₁₋₆ ou halogéno ;
R¹⁹ est hydrogène ou alkyle en C₁₋₆ ;
Ar¹ est phényle ou phényle substitué par alkyle en C₁₋₆, hydroxy, amino, alkyloxy en C₁₋₆ ou halogéno ; et
Ar² est phényle ou phényle substitué par alkyle en C₁₋₆, hydroxy, amino, alkyloxy en C₁₋₆ ou halogéno.

5. Procédé de la revendication 4, dans lequel ledit inhibiteur de farnésyltransférase est :
(a) un composé de formule (I) dans lequel X est oxygène et le trait pointillé représente une liaison ; ou
(b) un composé de formule (I) dans lequel R¹ est hydrogène, alkyle en C₁₋₆, (alkyloxy en C₁₋₆)-(alkyle en C_{1-F}) ou, mono- ou di (alkyle en C₁₋₆)-amino-(alkyle en C₁₋₆) ; R² est halogéno, alkyle en C₁₋₆, alcényle en C₂₋₆, alkyloxy en C₁₋₆, trihalogénométhoxy, ou hydroxy (alkyloxy en C₁₋₆) ; et R³ est hydrogène ; ou
(c) (+)-6-[amino(4-chlorophényl)(1-méthyl-1H-imidazol-5-yl) méthyl] -4- (3-chlorophényl) -1-méthyl-2 (1H)-quinoléinone ; ou un sel d'addition d'acide pharmaceutiquement acceptable de celui-ci.

6. Procédé de la revendication 4, dans lequel R⁸ est hydrogène, hydroxy, halogéno(alkyle en C₁₋₆), hydroxy(alkyle en C₁₋₆), cyano (alkyle en C₁₋₆), (alkyloxy en C₁₋₆)-carbonyl-(alkyle en C₁₋₆), imidazolyle, ou un radical de formule -NR¹¹R¹² où R11 est hydrogène ou alkyle en C₁₋₁₂ et R¹² est hydrogène, alkyle en C₁₋₆, alkyloxy en C₁₋₆, (alkyloxy en C₁₋₆)-(alkyle en C₁₋₆)carbonyle, hydroxy, ou un radical de formule -Alk²-OR¹³ où R¹³ est hydrogène ou alkyle en C₁₋₆.

7. Procédé tel que défini dans la revendication 1, dans lequel ledit au moins un marqueur est choisi dans le groupe constitué de IL-6, MCP-1, IL-1β, MMP-9, et TNF-α.

8. Procédé tel que défini dans l'une quelconque des revendications 1 ou 2, dans lequel, dans la sous-étape (ii), une pluralité d'échantillons de sang traités du sujet sont utilisés qui sont prélevés à des intervalles périodiques une fois que le traitement avec l'inhibiteur de farnésyltransférase a commencé.

9. Procédé tel que défini dans la revendication 1, dans lequel l'échantillon de sang est du sang total, du plasma, du sérum, des cellules totales, ou des cellules mononucléaires de sang périphérique.

10. Procédé tel que défini dans la revendication 2, dans lequel les sous-étapes (b)(i) et (b)(ii) comprennent : (i) l'isolement de plasma à partir de l'échantillon de sang non traité par centrifugation pour obtenir l'extrait d'échantillon non traité, et (ii) l'isolement de plasma à partir de chaque échantillon de sang traité par centrifugation pour obtenir l'extrait d'échantillon traité.

11. Procédé tel que défini dans la revendication 2, dans lequel les sous-étapes (b)(i) et (b)(ii) comprennent : (i) l'isolement de cellules mononucléaires de sang périphérique à partir de l'échantillon de sang non traité par séparation sur gradient Ficoll-Paque pour obtenir l'extrait d'échantillon non traité, et (ii) l'isolement de cellules mononucléaires de sang périphérique par séparation sur gradient Ficoll-Paque à partir de chaque échantillon de sang traité pour obtenir l'extrait d'échantillon traité.

12. Procédé tel que défini dans la revendication 2, comprenant en outre : (i) la stimulation de l'extrait d'échantillon non traité avec un lipopolysaccharide, et (ii) la stimulation de l'extrait d'échantillon traité avec un lipopolysaccharide.

13. Procédé tel que défini dans la revendication 3, dans lequel les sous-étapes (b)(i) et (b)(ii) comprennent chacune l'isolement des cellules mononucléaires de sang périphérique en utilisant une séparation sur gradient Ficoll-Paque.

14. Procédé tel que défini dans la revendication 13, dans lequel le sujet est un patient dans un essai clinique.

15. Procédé tel que défini dans la revendication 1 ou 14, dans lequel l'inhibiteur de farnésyltransférase utilisé pour traiter le sujet est le tipifarnib.
